# EUROPEAN PATENT APPLICATION

(11) **EP 1 097 991 A2**
(43) Date of publication of application: **09.05.2001**
(21) Application number: 00308130.4
(22) Date of filing: 18.09.2000
(51) Int. Cl.: C12N 9/88, C12N 15/60, C12N 15/85, A61K 38/51, A61K 39/395, A61K 45/06, G01N 33/573, A61K 31/445, A61K 38/13

(54) **Human adenylate cyclase IX and use therefor**

(30) Priority: 17.09.1999 US 398193
(71) Applicant: MEDICAL RESEARCH COUNCIL, London W1N 4AL (GB)
(72) Inventor: Antoni, Ferenc, Edinburgh EH10 5RW (GB); Paterson, Janice M., Edinburgh EH11 1NZ (GB)
(74) Representative: Murgitroyd, Ian G.

(57) **Abstract**

There is provided a novel adenylate cyclase enzyme, the nucleotide sequence of which is set out in SEQ ID No: 98. The activity of the adenylate cyclase is uniquely regulated by calcineurin, a protein phosphatase. The calcineurin binding site has been identified at amino acids 503 to 610 of the novel adenylate cyclase.

Regulation of the novel adenylate cycalse may be useful in treating certain disorders. Suitable regulators include agents which bind to the 503-610 amino acid site and include calcineurin, its activators, inhibitors and competitors and antibodies specific to that site. Specific disorders include neurological disorders such as Parkinsons' disease, cardiovascular disorders such as angina pectoris and tumours, especially ovarian tumours.

## Description

The present invention relates to the control of cellular metabolic process by human adenylate cyclase.

Cells of multi-cellular organisms may be metabolically affected by external factors, which are usually chemical. Hormones are a well-known example of such chemical factors. Generally the external chemical factors interact with a specific receptor located on the membrane of the targeted cell. The binding event of the factor to its receptor may induce alterations in cellular metabolism via a "secondary messenger" mediator.

One of the key "secondary messengers" is cyclic AMP (cAMP) (see Sutherland, Science 177:401-407 (1972)). Cyclic AMP is produced from ATP through the action of an enzyme, adenylate cyclase. It is now known that adenylate cyclase activity may be affected by a factor/receptor binding event transmitted through an associated G protein.

Alteration of the intracellular concentration of cAMP affects many cellular reactions. For example, an increase in cAMP intracellular concentration stimulates the activity of protein kinases (enzymes that transfer terminal phosphate groups from ATP to specific sites on targeted proteins). The action of the protein kinases changes the activity or function of its substrate.

For a general review of cAMP and secondary messenger systems reference is made to "Molecular Cell Biology", Darnell et al, 1986, Chapter 16, incorporated herein by reference.

Further investigations of cAMP as a secondary messenger revealed that an alteration in cAMP intracellular concentration was caused by the interaction of several different external factors with their distinct receptors. Further, it was found that different receptors were associated with their own particular G-protein intermediary which was itself associated with adenylate cyclase. More recent investigations have shown that there are in fact different types (isoenzymes) of adenylate cyclase, which display considerable regulatory diversity.

To date eight distinct isoenzymes of adenylate cyclase have been identified and described in the literature. The complete cDNA sequences are known for isoenzymes types 1 to 8. A review of the current understanding and knowledge of the known adenylate cyclase isoenzymes is set out in Pieroni et al, Current Opinion in Neurobiology 3:345-351 (1993); Kerwin Jr in Annual Reports in Medicinal Chemistry, Section VI, Chapter 29, Pages 287-295 (ed Venuti), (1994) and Premont, Methods in Enzymology 238:116-127 (1994).

A summary of the regulation of the known isoenzymes of adenylate cyclase is set out below in Table 1.

**Table 1**

| Isoenzyme | Regulated by | cAMP concentration |
|---|---|---|
| 1 | Ca²⁺/CaM | ↑ |
| | | |
| | β γ dimer | ↓ |
| 2 | Gₓᵢ + PKC | ↑ |
| | | |
| | β γ dimer | ↑ |
| | | |
| | PKC | ↑ |
| 3 | Ca²⁺/CaM | ↑ |
| 4 | β γ dimer | ↑ |
| 5 | Ca²⁺ | ↓ |
| 6 | Ca²⁺ | ↓ |
| CaM = calmodulin | | |
| PCK = protein Kinase C | | |

It has now been found, for the first time, that the protein phosphatase calcineurin regulates an adenylate cyclase isoenzyme.

It has further now been found that the isoenzyme regulated by calcineurin is a novel previously uncharacterised adenylate cyclase isoenzyme. The novel isoenzyme of the present invention was originally referred to as adenylate cyclase 10 (AC10), but a review of nomenclature has now caused the novel adenylate cyclase to be referred to as adenylate cyclase 9 (AC9). To avoid confusion with the different isoenzyme known before the nomenclature revision as "adenylate cyclase 9", the novel adenylate cyclase of the present invention will herein simply be referred to as "AC".

The nucleotide sequence encoding for mouse AC has been identified, cloned and sequenced (see Example 2). The nucleotide sequence encoding for mouse AC is given in SEQ ID No 1. The sequence is also accessible in the Genbank™ database under accession No. MMU30602 and in the EMBL database under accession No. Z50190.

A nucleotide sequence purporting to be that for human AC is described in WO-A-99/01540. The nucleotide sequence presented in WO-A-99/01540 indicates that the C-terminal portion of the protein is truncated relative to that of the mouse. We have now conducted additional work in this area and, surprisingly, have cloned and sequenced a human AC construct which does not have the truncated C-terminal portion reported in WO-A-99/01540. The sequence obtained by us for human AC is set out in SEQ ID No 98.

The present invention therefore provides a polypeptide encoded by the nucleotide sequence of SEQ ID No 98 or as set out in SEQ ID No 99 (or functional equivalents or parts of those sequences).

The term "functional equivalents" is used herein to refer to any modified version of a nucleotide or polypeptide which retains the basic function of its unmodified form. As an example, it is well-known that certain alterations in amino acid or nucleic acid sequences may not affect the polypeptide encoded by that molecule or the function of the polypeptide. It is also possible for deleted versions of a molecule to perform a particular function as well as the original molecule. Even where an alteration does affect whether and to what degree a particular function is performed, such altered molecules are included within the term "functional equivalent" provided that the function of the molecule is not so deleteriously affected as to render the molecule useless for its intended purpose.

Whilst we do not wish to be bound to theoretical considerations, it is believed that calcineurin regulates AC by removal of phosphate group(s) required for the active form of the enzyme. Thus, the adenylate cyclase activity of AC is believed to decrease in the presence of calcineurin.

In a further aspect, therefore, the present invention provides the use of calcineurin in the regulation of adenylate cyclase activity, in particular in the regulation of AC.

The activity of calcineurin is itself enhanced by the presence of Ca²⁺ ions and further enhanced by the additional presence of calmodulin.

In a further aspect, the present invention provides an adenylate cyclase isoenzyme, the activity of which can be regulated by calcineurin.

Desirably the calcineurin regulatable adenylate cyclase isoenzyme is encoded by the nucleotide sequence of SEQ ID No 98, functional equivalents or parts thereof.

In a yet further aspect, the present invention provides a polynucleotide comprising a sequence derived from the sequence set out in SEQ ID No 98 or a part thereof.

The phrase "derived from" includes identical and complementary copies of the sequence of SEQ ID No 1, whether of RNA or DNA and whether in single or doublestranded form. The phrase "derived from" further includes sequences with alterations which (due to the degeneracy of the genetic code) do not affect the amino acid sequence of the polypeptide expressed, as well as sequences modified by deletions, additions or replacements of nucleotide(s) which cause no substantial deleterious affection to function (including the function of the polypeptide expressed).

The polynucleotide of the present invention includes all recombinant constructs comprising a nucleotide sequence of the invention as defined above. Such recombinant constructs may be designed to express only part of AC. The constructs may include expression control sequence(s) which differ to the control sequence(s) naturally adjoining the AC gene. Optionally, the construct may include a non-AC protein encoding region. Thus the recombinant construct includes constructs encoding for chimeric proteins, which comprise at least part of AC or a functional equivalent thereof.

In a particular embodiment, the present invention provides a vector (such as a cloning or expression vector) which comprises a recombinant construct as defined above. Vectors include conventional cloning and expression plasmids for bacterial and yeast host cells as well as virus vectors such as vaccinia, which may be useful for expression in eukaryotic cell lines. Such a vector may be used to transform a suitable host cell (either for cloning or expression purposes) and the transformed host cell also forms a further aspect of the present invention. If the vector produced is comprised only in part of a nucleotide sequence derived from SEQ ID No 1 it may be appropriate to select a host cell type which is compatible with the vector. Mention may be made of prokaryotic host cells such as E coli as well as eukaryotic host cells, including yeasts, algae and fish, insect or mammalian cells in culture. Insect cells may be especially useful where a baculovirus expression system is used. Suitable host cells will be known to those skilled in the art.

As a general reference to genetic engineering techniques, mention may be made of Sambrook, Fritsch, Maniatis, in "Molecular Cloning, a Laboratory Manual", Cold Spring Harbor Laboratory, Cold Spring Harbor, New York, 1989; and also to Old and Primrose "Principles of Genetic Manipulation", 5th edition, 1994.

In particular cell lines derived from the human embryonic kidney cell line 293 (HEK293) have been generated and stably express AC. Briefly, the cells were transfected with pcDNA3 containing the full length AC DNA (clone JP 173 - see Example 3) and cells were selected on the basis of resistance to G418 antibiotic (0.8mg/ml) for 4 weeks. Individual clones of resistant cells were expanded and tested for cAMP production in response to CRF and the effects of immunosuppressants were also examined. At least three cell lines exhibited cAMP production in response to CRF which was enhanced by both cyclosporin A and FK506. In the case of FK506, this effect is blocked by L-685,818. Furthermore, the accumulation of cAMP in the presence of inhibitors of phosphodiesterase is about 10-fold higher than in wild type HEK293 cells. This latter finding suggests that the transfected cyclase is constitutively active and thus may be a mutant of the wild type enzyme.

In a yet further aspect, the present invention provides regulators of AC. Such regulators may act directly on AC itself, and may include (but are not limited to) calcineurin and antibodies specific to AC, or may affect production of AC, including (for example) antisense oligonucleotides (which prevent expression of the AC gene by binding to a portion of DNA preventing transcription and/or by binding to the mRNA preventing translation), and agents which bind to receptors and thus affect the activity of AC, for example β-adrenergic agonists and β-adrenergic antagonists. As β-adrenergic agonists mention may be made of salbutamol, clenbuterol, fenoterol and the like, whereas suitable β-adrenergic antagonists include propranolol.

Antibodies (including monoclonal antibodies) specific to AC may be produced using conventional immunological techniques.

In a yet further aspect, the present invention provides a method of regulating cellular metabolism, wherein said method comprises altering the activity or amount of AC. For example, the activity or amount of AC may be influenced by the regulators described above. Alternatively, for example, the amount of AC could be controlled through genetic manipulation of the genome, by providing agents that affect transcription and/or translation of the AC gene.

The distribution of AC varies between different tissues or cell types and it is postulated that the intracellular concentration of AC may fluctuate as a result of expression control of the AC gene in response to particular stimuli.

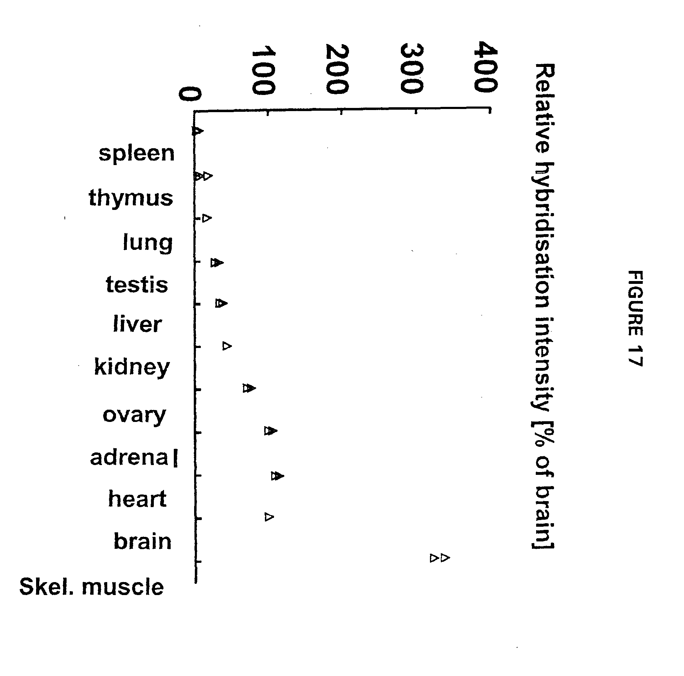

Studies of AC distribution in tissues suggest that it is prominently present in skeletal muscle and heart (Figure 17). Its likely function in these tissues is the coordination between contraction and metabolic demand. Intracellular calcium ions promote contraction, and cAMP promotes glycogenolysis.
Feedback of calcium ions on cAMP synthesis would delimit the accumulation of calcium and ensure that the contractions are no longer than what can be metabolically tolerated by the cells. In this respect it is plausible that the levels of cyclase will be regulated by the trophic state of the muscle such as that seen in the training of sportsmen and animals such as racing horses. Thus the measurement of AC levels by means of specific probes would constitute a means of predicting optimal regulation of contraction and metabolism of nutrients.

Thus for example AC may be present at abnormal levels in certain disease states or conditions. Mention may be made in this regard of neurological-based disorders (for example Parkinson's disease, epilepsy), psychiatrically-based disorders (for example, anxiety, major depression disorder, mania, schizophrenia, obsessive-compulsive disorder, Tourette's Syndrome and related tics) endocrine-based disorders (for example Cushing's Syndrome and disease, Nelson's Syndrome, Cohn's Syndrome, glucocorticoid resistance, Graves' disease (thyrotoxicosis) with or without exophthalmia, hyper and hypothyroidism; hyperprolactinaemia and its effects); hypertrophy of the prostate; cardiovascular-based disorders (for example, angina pectoris, cardiac infarction, hypertension (benign and malignant)), pregnancy-based disorders (for example, recurrent spontaneous abortion, pre-eclampsia and eclampsia) respiratory-based disorders (for example, asthma, bronchitis, chronic bronchitis emphysema and cor pulmonale), bone-based disorders (for example, hyperparathyroidism, osteomalacia, Paget's disease, osteoporosis), promotion of bone healing, kidney-based disorders (for example, acute and chronic glomerular nephritis, Albright's Syndrome - or the McCune Albright's Syndrome), gut-based disorders (for example, ulcerative colitis, irritable bowel, Crohn's disease, Hirschssprung's disease), tumours (benign and malignant), especially ovarian tumours and prostate tumours, and in the control or promotion of fertility. AC has been found to be of greater prevalence in the brain (in particular the cortex, striatum and hippocampus regions of the brain), in the ovaries and in the lungs.

Finally, substances developed to regulate the activity of this cyclase may be useful as improvers of metabolic balance in ischemic heart muscle and syndromes of skeletal muscle atrophy.

In a further aspect, therefore, the present invention provides a method of treating such conditions by control of AC activity. An example of such control could be the design of compounds that mimic the steric conformation of amino acids in positions 503 to 610, especially 503 to 570, of SEQ ID No 99 which may function as a calcineurin binding-site.

The amino acid sequence of human AC has been analysed (analogously to that of the mouse sequence in Example 3) and a domain corresponding to the immunophilin protein FKBP12 (which potently inhibits calcineurin) has been located in residues 594 to 611. The sequence similarity strongly suggests that this is the calcineurin binding site.

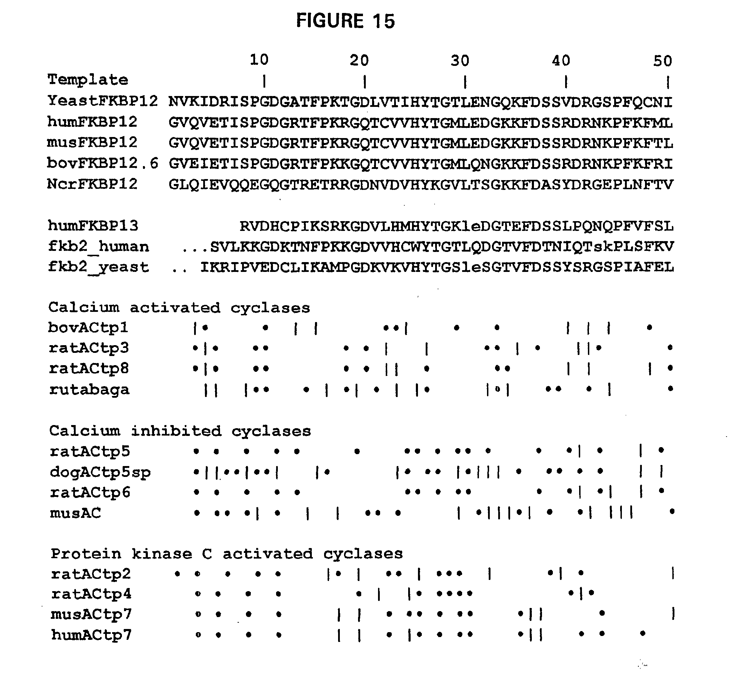

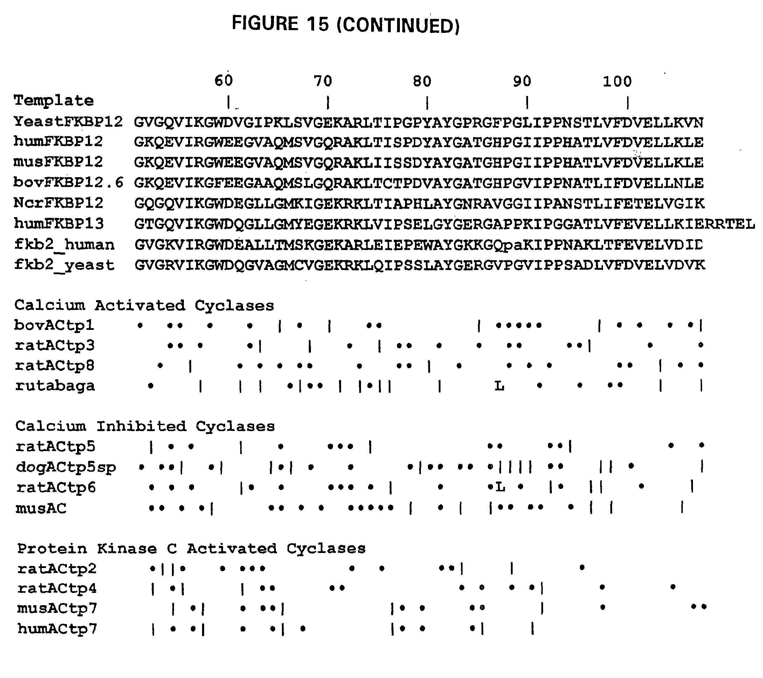

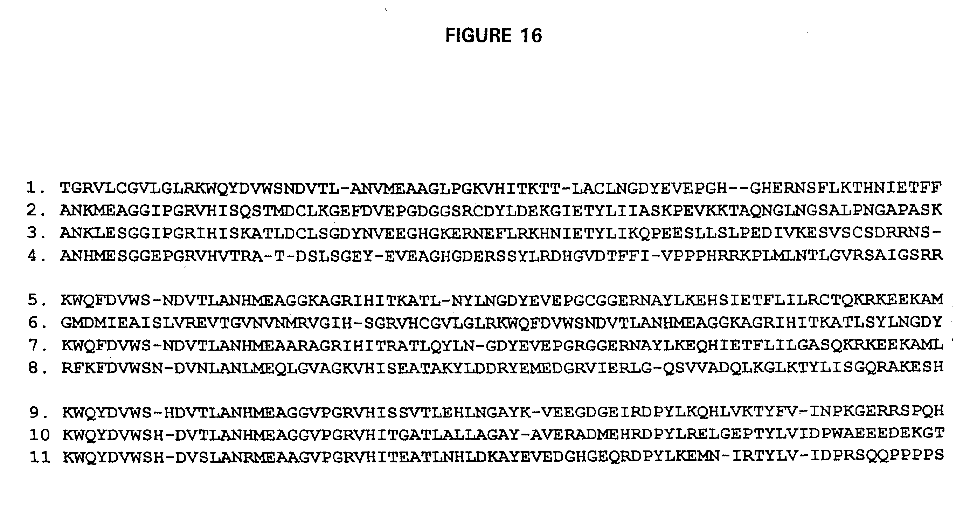

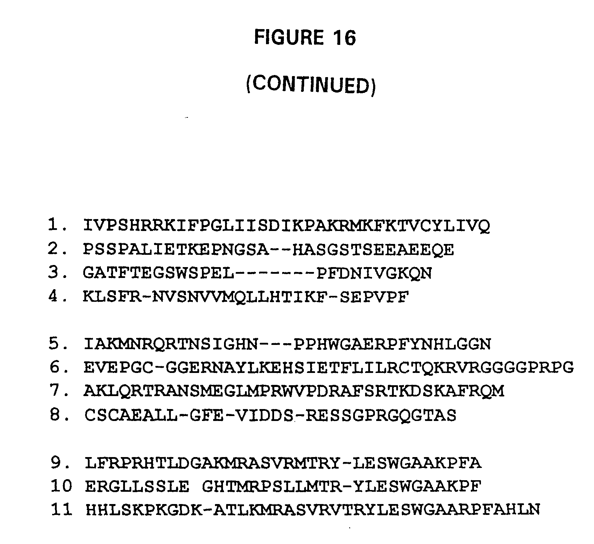

Other adenylyl cyclases were also examined for potential sequence similarities with the FKPB12 and 13 proteins as described in Example 3. A summary of the findings is shown in Figure 15. Briefly, all of the known adenylyl cyclase sequences appear to show significant similarity with FKBPs in the area that corresponds to the junction of the C1α and C1β domains (see Example 3 for nomenclature). As shown in Figure 16 the alignments of individual cyclases with FKBP12 are distinct i.e. different portions of the C1α high homology region are part of the FKBP like sequence. These alignments are possible because of modular repeats in the cyclase amino acid sequence in this region. The guiding motif for these alignments is the requirement of the presence of an "80s" loop of FKBP12 in a homologous position in the cyclase "FKBP like" sequence (Yang et al, (1993) Journal of the American Chemical Society 115:819-820). It is of note for instance, that ACtype1, a calcium activated adenylyl cyclase contains a significant portion (sequence: FGPLI) of the 80s loop of FKBP12 which is essential for the interaction with calcineurin (Yang et al, 1993 supra).

Taken together with the current knowledge on the regulation of ACtype1 it appears that the C1α-C1β junction domain is an important regulatory site of adenylyl cyclase activity. It is proposed that all adenylyl cyclases are regulated by tightly associated calcineurin bound in this region. This association may eventually prove to be dependent on calcium ions. It is also of note that there are potential protein kinase A, CAM kinase II and casein kinase II phosphorylation sites in this area in most adenylyl cyclases, which could be substrates of calcineurin and/or may influence the avidity of association of calcineurin with the cyclases.

The FKBP12 like domain (residues 594-611) of mouse AC has been expressed as a Glutathione-S-transferase (GST) fusion protein in the expression vector pGEX-2T (Pharmacia). The cyclase portion of this fusion protein can be phosphorylated by cAMP dependent protein kinase and dephosphorylated in a calcium dependent manner by calcineurin. These data support the notion that the region of the cyclase described here is a clacineurin binding site and contains phosphorylated amino acids that are substrates for calcineurin.

Data recently published show a remarkable similarity between a phosphorylation site for myotonic dystrophy protein kinase on the β-subunit of the L type skeletal muscle calcium channel (Timchenko et al, (1995) Proceedings of the National Academy of Sciences of the United States of America 92:5366-5370) and the FKBP-like domain of AC as shown below (wherein • shows amino acid identity and | shows functionally conservative subsitutions):

IDDSRESSGPR    AC594-604 (SEQ ID NO: 4)
| |•• ••| |
LRQSRLSSS-K    calcium channel β-subunit176-185
               (SEQ ID NO: 3)

In addition, it has been reported that the calcium β-subunit is selectively dephosphorylated by calcineurin (Lai et al. (1993) Journal of Neurochemistry 61:1333-1339). It is therefore likely that Ser₆₀₀ or Ser₆₀₁ in
AC may be phosphorylated by myotonic dystrophy protein kinase and dephosphorylated by calcineurin. Abnormalities in this process may occur in myotonic dystrophy and contribute to the symptoms of this hereditary disorder, especially with respect to the pathological changes of brain, muscle, heart and anterior pituitary function.

In a yet further aspect, the present invention also provides a diagnostic assay, to determine the presence and/or the amount of AC within a sample, said method comprising contacting an agent specific to AC with said sample and determining the presence and/or amount of complex formed. The agent may be, for example, an anti-sense oligonucleotide which is complementary to the mRNA of AC. Alternatively, the agent may be an antibody specific to AC, or may be calcineurin.

In a preferred embodiment the diagnostic agent is immobilised on a support, for example a membrane. Further, it is also preferred that a labelling moiety is present in the assay, so that measurement of the agent/AC complex is simplified.

The present invention will now be further described with reference to the following examples and with references to the accompanying figures in which:

### FIGURE 1

Effect of FK506, cyclosporin A (CsA) and MeVal⁴-cyclosporin A (MeVal⁴CsA) (SDZ202-384) on cAMP accumulation induced by 10nmol/l CRF in AtT20 cells in the presence of 0.5mmol/l isobutylmethylxanthine.
Basal cAMP production was 0.6±0.08pmol/well. Data are means±SEM, expressed as percentage of the increment caused by 10nmol/l CRF which was 6.1pmol/well.

### FIGURE 2

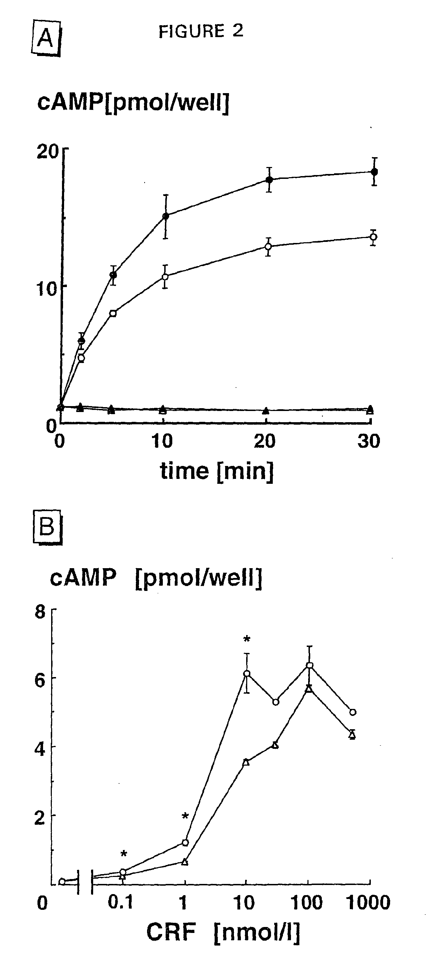

**A:** Effect of cyclosporin A on the time-course of basal and CRF-induced cAMP formation. Data are from a representative of 2 experiments. Means±SEM, n=3 for CRF treated groups (o = 10nmol/1 CRF+vehicle, ● = CRF + 1µmol/l cyclosporin A) and n=1 for basal (_ = vehicle, 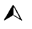 = cyclosporin A), 0.5mmol/l IBM, 0.1mM rolipram and cyclosporin A were given as pre-treatment for 30 minutes at 37°C before the application of 3nM CRF for 10 minutesat 24°C. *P<0.05 compared with respective control group (one way ANOVA followed by orthogonal contrasts).
**B:** Dependence of the effect of 1µmol/l FK506 on CRF-induced cAMP formation in the presence of 0.5mM IBMX. FK506 and IBMX are given as for Fig 2A. Data are means±SEM, n = 4-6/group. (□ = FK506 group, _ = control group) * P<0.05 compared with respective vehicle treated group (one-way ANOVA followed by orthogonal contrasts).

### FIGURE 3

**A:** The effect of L685,818 on the enhancement of CRF-induced cAMP formation by FK506. Data are means±SEM, the cAMP level in the presence of 10nmol/l CRF taken as 100% was 12pmol/well*10minutes, basal levels were 1.6pmol/well*10minutes.
**B:** The effect of L685,818 on the inhibition of calcineurin activity by 1µmol/l FK506 in AtT20 cells. Calcineurin activity was measured by the phosphocasein method, the activity measured in the absence of FK506 was taken as 100%.

### FIGURE 4

Effects of various manipulations that reduce intracellular free calcium concentration on CRF-induced cAMP accumulation in AtT20 cells. IBMX 0.5mmol/l present throughout. All data are expressed as the percentage of CRF-induced cAMP-formation, means±SEM, n=4/group.
EGTA: cells exposed to 2mmol/l EGTA in calcium free medium during the preincubation period; nimodipine: cells exposed to 1µmol/l nimodipine during preincubation period;
BAPTA-AM: cells incubated in 20µmol/l [1,2*-bis-* (*o*-Aminophenoxy)-ethane-N,N,N',N'-tetraacetic acid tetra-(acetoxymethyl)-ester] during the preincubation period.

### FIGURE 5

Effect of FK506 on the inhibition of CRF-induced cAMP accumulation by extracellular calcium ions in AtT20 cells. Cells were depleted of calcium by preincubation in 2mmol/l EGTA, calcium free medium, containing 5µmol/l A23187 and 5µmol/l nimodipine, and graded amounts of CaCl₂ were added with 10nmol/l CRF. The values on the abscissa give the nominal free extracellular calcium ion concentration. Data are means±SEM, n = 6/group.

### FIGURE 6

Effect of pertussis toxin (1µg/ml for 18 hours) on the modulation of CRF-induced cAMP formation by FK506 and somatostatin. AtT20 cells were preincubated with FK506 or somatostatin for 30 minutes. IBMX (0.5mmol/l) present throughout. Data are means±SEM, n=6/group.

### FIGURE 7

**A:** FK506 (0.5µmol/l) on basal and CRF-induced ACTH secretion in AtT20 cells.
**B:** Antagonism of the effect of FK506 (0.5µmol/l) on CRF-induced ACTH release by L685,818, which had no effect on basal ACTH secretion in this system even at 5µmol/l. n=4/group, means±SEM.

In panel A the values for basal and CRF-stimulated ACTH release taken as 100% were 15±1 and 22±1fmol/well*30 minutes, respectively.

### FIGURE 8

Comparison of the sequence (SEQ ID NO: 64) amplified from AtT20 cell RNA using primer set B, with the corresponding sequences with other adenylyl cyclases found in current databases (EMBL, GenBank, SwissProt (SEQ ID NOS: 48 to 64)). The numbers relate to the amino acid sequence of rabbit ACtype5 (ocmradcyv) (SEQ ID NO: 60). The sequence is annotated by • showing amino acid identity of the novel sequence with at least one previously reported AC, o shows funtionally conservative substitutions, - denotes non-conservative subsitutions.

### Abbreviations and sequence identification numbers

hum7 - Human ACtype7 (GenBank #D25538) (SEQ ID NO: 48);
mmu12919 - mouse ACtype7 (SEQ ID NO: 49);
cya²_rat - rat ACtype2 (SEQ ID NO: 50);
cya⁴_rat - rat ACtype4 (SEQ ID NO: 51) ;
hsadencyr8 - Human ACtype8 (SEQ ID NO: 52);
ratacviii - rat ACtype8 (SEQ ID NO: 53);
a46187 - human ACtype5 (SEQ ID NO: 54);
cya6_mouse - mouse ACtype6 (SEQ ID NO: 55);
a49201 - mouse ACtype5 (now renamed as mouse AC6) (SEQ ID NO: 56);
cya6_rat - rat ACtype6 (SEQ ID NO: 57);
cya6_canfa - dog ACtype6 (SEQ ID NO: 58);
s29717 - rat ACtype5 (SEQ ID NO: 59);
ocmradcyv - rabbit ACtype5 (SEQ ID NO: 60);
cya5_canfa - dog ACtype5 (SEQ ID NO: 61);
cya1_bovin - bovine ACtypel (SEQ ID NO: 62);
cya3_rat - rat ACtype3 (SEQ ID NO: 63);
AC - AtT20 derived new sequence, ie AC (SEQ ID NO: 64).

### FIGURE 9

Pileup analysis of adenylyl cyclase sequences found in the EMBL and GenBank data bases.

### FIGURE 10

Hydrophobicity plot of AC according to Kyte et al (1982) J Mol Biol 157:105. The numbers show predicted intramembrane domains.

### FIGURE 11

### Functional analysis of cloned AC in transfected host cells

Levels of cAMP in transiently transfected HEK293 (a-c) and COS7 (d) in the presence of phosphodiesterase inhibitors (lmmol/l isobutylmethylxanthine) and 0.1mmol/l rolipram). Data shown are means±SEM, n=4/group. Representative data from two similar series of experiments.
(a) HEK293 cells transfected with vector cDNA and pretreated with 0.2% (v/v) ethanol (empty columns) or 2µmol/l FK506 (striped columns) before being challenged with 0.5nmol/l CRF.
(b) as (a) except with cells transfected with AC (Data are means±SEM, n=4/group. *P<0.05 when compared with the respective vehicle-treated group, 1-way ANOVA, followed by Newman-Keuls test).
(c) The effect of FK506 on CRF-induced cAMP production was analysed further in (c). The enhancement of the CRF-induced cAMP response by FK506 was blocked by the
   FK506 antagonist drug L-685,818 (100µmol/l) (Dumont et al, (1992) J. Exp. Med. 176:751-760), which had no effect on CRF-induced cAMP formation when given alone. It did, however, similarly to FK506, increase the unstimulated levels of cAMP to 3-fold above basal (not shown). *P<0.05 when compared with the vehicle treated group, 1-way ANOVA, followed by Newman-Keuls test.
(d) Application of cyclosporin A, another calcineurin blocking immunosuppressant enhanced the cAMP response to 1nmol/l CRF in COS7 cells transfected with AC (*P<0.05, when compared with the vehicle-treated group).

### FIGURE 12

### An FKBP-like domain in AC

(A) Schematic drawing of the predicted structure of AC, nomenclature as suggested by Gilman and coworkers (Taussig et al, (1995) J. Biol. Chem. 270:1-4). N = N-terminal intracellular loop; M1 and M2 = membrane spanning segments; C1α and C2α (thick line) = the highly conserved, putative catalytic cyclase domains; C1β and C2β = the non-conserved, putative regulatory domains of the intracellular loops.
(B) AC₍₅₀₃₋₆₁₀₎ (SEQ ID NO: 65) shows approximately 40% overall sequence similarity with FK506 binding-protein 12 (FKBP12) (SEQ ID NOs: 66 to 69). Residue numbers in the top row correspond to AC, in the bottom row to mammalian FKBP12s. A dot indicates sequence identity between AC and at least one of the FKBPs. Vertical lines denote conserved substitutions which were defind as 1) C 2) S T P A G 3) N D E Q 4) H R K 5) M I L V 6) F Y W (see Krupinski et al, (1989) Science 244:1558-1564).
   yst = Saccharomyces cerevisiae, hum = human, mus = mouse, ncr = Neurospora crassa.
(C) Alignment of the C1α and C1β junction region of known mammalian adenylyl cyclase isotypes (SEQ ID NOs: 70 to 78). The underlined region in bovACtpl (SEQ ID NO: 70) corresponds to 24 residues of the 28 amino acid residue putative calmodulin binding site (495-522) (Vorherr et al, (1993) Biochemistry (USA) 32:6081-6088; and Wu et al, (1993) J. Biol. Chem. 286:23766-23768), bov = bovine. Abbreviations as for Figure 15.

### FIGURE 13

### Northern analysis of AtT20-cell, HEK293-cell and mouse striatal RNA

³²P-labelled cDNA probes derived from the AC sequence were used. Note approximately 9kb band in AtT20 cells that hybridizes with both probes. A similar size RNA species is also intensively hybridizing in mouse striatal RNA and a weak, barely discernible band is found in HEK293 cells. The relative hybridisation intensities (average of the two lanes) with probe jp 164 were: AtT20 2.1; HEK293 0.3; mouse striatum 1.4.

### FIGURE 14

### Localisation of AC mRNA in mouse brain

AC mRNA detected by a ³⁵S-CTP labelled antisense ribonucleic acid probe derived from plasmid JP142 in
(A) the hippocampus (pyramidal cell layer of CA1-CA4(a) and of the subiculum (b), granule cells in dentate gyrus (c) and in various parts of the cereral cortex (posterior cingulate cortex indicated by (d)).
(B) ³⁵S-CTP-sense RNA probe control.

### Magnification: 25x.

### FIGURE 15

An alignment of adenylyl cyclases with FKBPs (SEQ ID NOs: 79 to 86).
- indicates identity; | indicates conserved substitution in at least one of the FKBPs. Conserved substitutions are defined as

1) C
2) STPAG
3) NDEQ
4) HRK
5) MILV
6) FYW

| Abbreviations and sequence identifiers | |
|---|---|
| YeastFKBP12 | Yeast FKPB12 (SEQ ID NO: 79) |
| humFKBP12 | human FKBP12 (SEQ ID NO: 80) |
| musFKBP12 | mouse FKBP12 (SEQ ID NO: 81) |
| NcrFKBP12 | Neurospora crassa FKBP 12 (SEQ ID NO: 83) |
| humFKBP13 | human FKBP13 (SEQ ID NO: 84) |
| fkb2_hum | human FKBP13 precursor (SEQ ID NO: 85) |
| fkb2_yeast | yeast FKBP13 (SEQ ID NO: 86) |
| bovACtp1 | bovine adenylyl cyclase type 1 |
| ratACtp3 | rat adenylyl cyclase type 3 |
| ratACtp8 | rat adenylyl cyclase type 8 |
| rutabaga | drosophila calmodulin activated adenylyl cyclase |
| ratACtp5 | rat adenylyl cyclase type 5 |
| ACtp5sp1 | dog adenylyl cyclase type 5 splice variant |
| ratACtp6 | rat adenylyl cyclase type 6 |
| musAC | mouse adenylyl cyclase type 9 (AC) |
| ratACtp2 | rat adenylyl cyclase type 2 |
| ratACtp4 | rat adenylyl cyclase type 4 |
| musACtp7 | mouse adenylyl cyclase type 7 |
| humACtp7 | human adenylyl cyclase type 7 |

### FIGURE 16

Illustrates cyclases extracted from their alignments with FKBPs.

### Abbreviations and sequence identifiers

1. bovine adenylyl cyclase type 1 (SEQ ID NO: 87)
2. rat adenylyl cyclase type 3 (SEQ ID NO: 88)
3. rat adenylyl cyclase type 8 (SEQ ID NO: 89)
4. drosophila calmodulin activated adenylyl cyclase (SEQ ID NO: 90)
5. rat adenylyl cyclase type 5 (SEQ ID NO: 91)
6. dog adenylyl cyclase type 5 splice variant (SEQ ID NO: 92)
7. rat adenylyl cyclase type 6 (SEQ ID NO: 93)
8. mouse adenylyl cyclase type 9 ie AC (SEQ ID NO: 94)
9. rat adenylyl cyclase type 2 (SEQ ID NO: 95)
10. rat adenylyl cyclase type 4 (SEQ ID NO: 96)
11. mouse adenylyl cyclase type 7 (SEQ ID NO: 97)

### FIGURE 17

Illustrates the relative hybridisation intensity in different tissues, showing the distribution of AC mRNA in mouse, as measured by RNase protection assay.

### FIGURE 18

### Construction of complete human AC9 cDNA

Scheme showing relationship of overlapping human AC9 cDNA clones used in construction of the complete hAC9 cDNA (GenBank/EMBL Accession Number AJ133123) by joining clones HiBBC33, pJP192, pJP230 and 166657 at the Mlu I, Eco RV and Nco I sites indicated as described in materials and methods. The relationship of these clones to human AC9 cDNA clones isolated by other groups (GenBank/EMBL Accession Numbers AF036927 and AB011092) identified in subsequent searches of the GenBank/EMBL DNA sequence database is also represented.

### FIGURE 19

### Immunocytochemical detection of human AC9 protein

Cells cultured on coverslips were briefly fixed with 3% paraformaldehyde and reacted with affinity purified chicken antibodies directed against residues 1340-1353 of the mouse AC9 protein, with a tyrosine added at the N- terminus. Subsequently, rabbit anti-chicken IgG coupled to FITC was applied, and the cells were viewed under a Leica TCS-NT confocal microscope system. Note (a) preferential localisation of immunoreactivity in the vicinity of the plasma membrane and (b) dividing cell with membrane localisation of AC9. (c) Specificity control with antibody preincubated with 10µM of peptide antigen. The bar represents 10 µm.

### FIGURE 20

### Detection of multiple human AC9 polypeptides by immunoblotting

Protein extracts from membranes of HEK 293 cells, either untransfected (**a** & **b**, lane 3) or stably transfected with human AC9 cDNA (**a** & **b**, lanes 1,2,4&5) separated by SDS-PAGE and electroblotted onto PVDF membrane were incubated with antisera directed against mouse AC9 N-terminus (**a**) or C-terminus (**b**). Positions of molecular weight markers (KDa) are indicated. A major polypeptide of 175KDa was detected in two independent stable human AC9 transfectants (**a** & **b**, lanes 1 & 2). In addition, a faster migrating polypeptide was also detected. Specificity was confirmed by competition with peptide antigen (**a** & **b**, lanes 4 & 5). Increased background staining was apparent when C-terminal peptide was included (**b**, lanes 4 & 5).

### FIGURE 21

### Analysis of cAMP synthesis by HEK293 cells overexpressing human AC9

a) Time-course of cAMP accumulation. Blockers of cyclic nucleotide degrading phosphodiesterases (PDE) (isobutylmethylxanthine 1mM, and rolipram 0.1mM) and various amounts of Ca²⁺/EGTA were applied at time 0 to cells previously depleted of Ca²⁺ in HBSS/EGTA medium containing no added Ca²⁺, 1*µ*M thapsigargin and 10*µ*M ryanodine. The final extracellular levels of Ca²⁺ were (Δ) nominally 0, (o) 2mM, (n ) 5mM. Data are means±S.E.M. n=4 representative of 2 similar experiments. * P<0.05 when compared with the zero Ca²⁺ group, 1-way ANOVA followed by Dunnett's test. **b**) Effect of FK506 on the inhibitory effect of Ca²⁺ in cells pretreated with Ca²⁺ free medium containing 5*µ*M 4-Br-A23187. Blockers of PDE were added in Ca²⁺ /EGTA to achive 0.25 and 0.5 mM extracellular Ca²⁺. Data are expressed as multiples of the cAMP content measured in the absence of PDE blockers, means±S.E.M. n=4 representative of 3 similar experiments. * P<0.05 when compared with respective vehicle treated controls, + P<0.05 when compared with respective 0 Ca²⁺ group. **c**) Pharmacological analysis of the effect of various immunosuppressant compounds on Ca²⁺ inhibition of cAMP production. FK506, cyclosporin A (CsA), and rapamycin were used at 10*µ*M, L865,818 was present at 50*µ*M 20 min before the application of PDE blockers and Ca²⁺/EGTA. Data are expressed as multiples of the cAMP content measured in the absence of PDE blockers, means±S.E.M. n=4 representative of 2 similar experiments. * P<0.05 when compared with respective vehicle treated controls.

### FIGURE 22

### Differential expression of human AC9 mRNA

a) A probe derived from 5' end of human AC9 cDNA (see materials and methods) detected an 8.5kb transcript in all tissues represented on a northern blot of mRNAs from human heart (H), brain (B), placenta (Pl), lung (Lg), liver (Lv), skeletal muscle (M), kidney (K) and pancreas (Pn) as well as a 6.3kb mRNA in heart and skeletal muscle. The control probe (**b**) confirmed expression of a 2kb β-actin mRNA in all tissues as well as a 1.8kb mRNA in heart and skeletal muscle. **c**) The same northern blot re-probed with a cDNA fragment derived from the extended 3' UTR of human AC9 clone KIAA0520 (see materials and methods) shows exclusive detection of an 8.5kb mRNA. Positions of molecular weight markers (kb) are indicated.

### FIGURE 23

### Localisation of human AC9 mRNA in human forebrain

**a**) Autoradiogram showing protection of 314 bases of the single-stranded human AC9 riboprobe derived from clone pJP195 in samples of total RNA from post-mortem human cortex, hippocampus and striatum as well as in the HEK 293 cell positive control (as indicated by the arrow). No protection of probe by yeast RNA (Y) was observed. Probe present prior to single-strand specific nuclease digestion is shown in the first lane (pr). **b**) *In situ* hybridisation using ³⁵S UTP-labelled anti-sense (i) and sense (ii) riboprobes derived from clone pJP195 incubated with 15*µ*m sections from normal adult post-mortem human brain showing detection of human AC9 mRNA by the anti-sense probe in hippocampal neurones of the CA1-CA3 regions (upper arrow) and dentate gyrus (lower arrow).

### FIGURE 24

### Comparison of AC9 nucleotide and predicted amino acid sequences

The amino acid sequence deduced from the human AC9 cDNA nuleotide reported (AJ133123) is highly similar to that of the mouse AC9 protein. The polypeptide deduced from the human AC9 cDNA sequence AF036927 is divergent from mouse, predicting an alternative C-terminus due to a frame shift resulting from a 2bp deletion.

| Abbreviations: | |
|---|---|
| **legend on dendrogram** | **adenylyl cyclase isoform** |
| hum7 | Human type 7 GenBank #D25538; |
| mmu12919 | mouse type 7; |
| cya2_rat | rat type 2; |
| cya4_rat | rat type 4; |
| cya5_canfa | dog type 5; |
| cya5_rabit | rabbit type 5; |
| s29717 | rat type 5; |
| cya6_mouse | mouse type 6; |
| cyal_bovin | bovine type 1; |
| ratacviii | rat type 8; |
| cya3_rat | rat type 3; |
| Ac | mouse AC 9, ie AC. |

### EXAMPLE 1 : CALCINEURIN FEEDBACK INHIBITION OF AGONIST EVOKED cAMP FORMATION

### INTRODUCTION

The major immunosuppressant compounds cyclosporin A and FK506 are potent blockers of the Ca²⁺/calmodulin-regulated protein phosphatase calcineurin (protein phosphatase 2B) in leukocytes (Liu et al, (1991) Cell 66:807-815). This observation has led to the discovery that calcineurin is an essential element of the signal transduction pathway activated by the T-cell receptor (Schreiber (1992) Cell 70:365-368 and Sigal et al, (1992) Ann. Rev. Immunol. 10:519-560). Immunophilins, which are proteins that mediate the effects of immunosuppressants on calcineurin activity in lymphocytes (Schreiber (1992) Cell 70:365-368) have been identified in the brain (Steiner et al, (1992) Nature 358:584-586) providing the plausible molecular targets of the prominent neurological side-effects of FK506 and cyclosporin A (Frank et al, (1993) Transplantation Proceeding 25:1887-1888 and Reyes et al, (1990) Transplantation 50:10434-1081).

While calcineurin is highly abundant in the brain (0.5-1% of total protein) (Klee et al, (1988) Advances in Enzymology 61:149-200) its functions in excitable cells remain to be defined. Calcineurin has been implicated in the control of voltage regulated ion channel activity (Armstrong, (1989) Trends in Neurosciences 12:117-122), particularly with respect to L-type calcium channels (Lai et al, (1993) J. Neurochem. 61:1333-1339). More recent studies have shown that the synaptic vesicular protein dynamin, which is thought to participate in synaptic vesicle recycling in nerve endings, is a prominent substrate for calcineurin (Liu et al, (1994) Science 265:970-973). Furthermore, blockage of calcineurin by immunosuppressants enhanced glutamate release by synaptosomes prepared from rat brain and this appeared to correlate with the state of phosphorylation of dynamin (Nichols et al, (1994) J.
Biol. Chem. 269:23817-23823).

With respect to secretory function in other systems, it has been reported (Antoni et al, (1993) Biochem. Biophys. Res. Commun. 194:226-233) that immunosuppressants block calcineurin activity in pituitary corticotrope tumour (AtT20) cells and stimulate Ca²⁺-dependent hormone release in correlation with their calcineurin blocking activity. Cyclic AMP (cAMP) is a cardinal signalling molecule in pituitary corticotrophs that causes an increase of intracellular free Ca²⁺ ([Ca²⁺]ᵢ) and triggers the release of adrenocorticotrophic hormone (ACTH) (Antoni, (1986) Endocr. Rev. 7:351-378). Because [Ca²⁺]ᵢ is known to inhibit cAMP formation in a variety of systems (Cooper et al, (1993) Trends Pharm. Sci. 14:34-36) we have analyzed in AtT20 cells the effects of FK506 and cyclosporin A on cAMP production induced by the hypothalamic neuropeptide corticotropin-releasing factor (CRF) and beta-adrenergic stimulation.

### EXPERIMENTAL PROCEDURES

AtT20 D16:16 mouse anterior pituitary tumour cells were maintained in culture as previously described (Woods et al, (1992) Endocrinology 131:2873-2880). For measurements of ACTH release, cAMP production or calcineurin activity the cells were plated on 24-well tissue culture plates (5x10⁴ cells/well), used 4-6 days afterwards. ACTH (Woods et al, (1992) Endocrinology 131:2873-2880) and cAMP (Dufau et al, (1973) Endocrinology 92:6-11) were measured by specific radioimmunoassays. Calcineurin protein phosphatase activity was determined by the ³²P-labelled casein assay (Tallant et al, (1984) Arch. Biochem. Biophys. 232:269-279) or by using the ³²P-labelled RII peptide substrate (Blumenthal et al, (1986) J. Biol. Chem. 261:8140-8145) adapted to measure calcineurin phosphatase activity in AtT20 cell extracts as previously described (Antoni et al, (1993) Biochem. Biophys. Res. Commun. 194:226-233).

Experiments for cAMP were all carried out in Hanks balanced salt solution containing 2mM CaCl₂ and 1 mM MgSO₄ buffered with 25mM HEPES pH 7.4 and supplemented with 0.25% (w/v) of bovine serum albumin. The cells were preincubated in serum free medium for 1 hour, after which blockers of phosphosdiesterase (isobutylmethylxanthine 0.5mM (IBMX) and/or rolipram 0.1mM) along with various other treatments were applied for 30 minutes at 37°C. Subsequently the cells were cooled to 22°C for 5 minutes in a water bath and agonists were added for 10 minutes. The reaction was stopped by the addition of 0.2mol/l HCl to achieve a final concentration of 0.1mol/l (Brooker et al, (1979) Adv. in Cyclic Nucl. Res. Vol. 10, G. Brooker, P. Greengard and G. A. Robinson, Raven Press, New York, 2-34). In the absence of phosphodiesterase blockers agonist-induced changes of total cAMP content (cells + medium) were small (2-3 fold of basal) and no increment of intracellular cAMP could be detected (Woods et al, (1992) Endocrinology 131:2873-2880). In the presence of IBMX total cAMP content increased linearly with time up to 10 minutes after the addition of CRF and remained constant for up to 20 minutes. In contrast, cellular cAMP content peaked between 2-5 minutes and subsequently declined to basal levels even in the presence of the phosphodiesterase blockers. Hence, after establishing that immunosuppressant drugs had the same effect on peak cellular and total cAMP content under these conditions, all experiments shown here report total cAMP content.

In some experiments cells were preincubated for 30 minutes in medium containing 2mmol/l EGTA and no added
Ca²⁺, supplemented with 5µmol/1 A23187 and 2.5µmol/l nifedipine in order to deplete rapidly mobilized cellular stores of Ca²⁺. This treatment also ensured that L-type Ca²⁺ channels, the principal avenue of voltage-regulated Ca²⁺ influx in AtT20 cells (Luini et al, (1985) Proc. Natl. Acad. Sci. U.S.A. 82:8034-8038; Reisine et al, (1987) Mol. Pharmacol. 32:488-496 and Antoni et al, (1992) J. Endocrinol. 133:R13-R16) were fully blocked and Ca²⁺ subsequently added to the extracellular fluid would enter largely through the pores made by the ionophore A23187. The rationale for this pretreatment is that calcineurin reportedly influences L-channel activity (Lai et al, (1993) J. Neurochem. 61:1333-1339; and Armstrong et al, (1987) Proc. Natl. Acad. Sci. U.S.A. 84:2518-2522), whereas the treatment regimen used here would make Ca²⁺ entry independent of this regulation.

Immunosuppressant analogues (FK506, courtesy of Fujisawa Ltd, Osaka, Japan; cyclosporin A and SDZ 220-384 (MeVal⁴-cyclosporin A) (Fliri, H. (1993) Antibiotics and antiviral compounds, Chemical synthesis and modification K. Krohn, H. Kirst And H. Maasg, VCN Verlagsgesellschaft mbH, Weinheim, 229-240), courtesy of Sandoz Pharma, Basel, Switzerland, L685,818 (Dumont et al, (1992) J. Exp. Med. 176:751-760) courtesy of Merck&Co, Rahway, NJ) were also applied during the preincubation period. These compounds were made up in ethanol at 10⁻³mol/l and diluted with the incubation medium to the desired final concentrations. In some cases cells were preincubated with L685,818 the structural analogue of FK506 that binds to FKBP-12 and inhibits prolyl-isomerase activity but is devoid of immunosuppressant activity (Dumont et al, (1992) J. Exp. Med. 176:751-760) for 10 minutes before the addition of FK506 for 30 minutes at 37°C.

Incubations for ACTH secretion were carried out as previously described (Woods et al, (1992) Endocrinology 131:2873-2880) except that the test incubation with CRF was at 22°C.

### Amplification and DNA sequence analysis of adenylyl cyclases cDNAs in AtT20 cells

Total RNA was prepared from approximately 10⁷ cells using Trizol reagent (GIBCO, Paisley, UK) according to the manufacturer's instructions. RT-PCR was carried out using an RNA PCR kit (Perkin Elmer, Warrington, Cheshire, U.K.). Briefly, 0.8µg of total RNA was denatured at 95°C for 5 minutes then annealed with 2.5µM random hexanucleotide primers for first-strand cDNA synthesis which was carried out for 15 mínutes at 42°C in a 20µl reaction mixture containing 10mM Tris.HCl, pH8.3, 50mM KCl, 5mM MgCl₂, 1mM each dNTP, 20U RNase inhibitor and 50U MMLV reverse transcriptase. The reaction was terminated at 99°C for 5 minutes then cooled to 4°C and stored on ice. PCR was performed using degenerate oligonucleotides, either pair A or pair B corresponding to highly conserved regions within the first (pair A:
5'CTCATCGATGGIGAYTGYTAYTAYTG3' (SEQ ID NO: 5); 3'GGCTCGAGCCAIACRTCRTAYTGCCA5' (SEQ ID NO: 6) expected product size 220bp) and second (pair B:
5'GAAGCTTAARATIAARACIATIGGI^{T}/_{A}^{C}/_{G}IACITAYATGGC3' (SEQ ID NO: 7); 3'GGGATCCACRTTIACIGTRTTICCCCAIATRTCRTA5' (SEQ ID NO: 8) expected product size 180bp) cytoplasmic domains of previously cloned mammalian adenylyl cyclases (Yoshimura et al, (1992) Proc. Natl. Acad. Sci. U.S.A. 89:6716-6720; Krupinski et al, (1992) J. Biol. Chem. 267:24859-24862; and Gao et al, (1991) Proc. Natl. Acad. Sci. U.S.A. 88:10178-10182). For PCR the reverse transcription reaction (20µl) was expanded to 100µl and contained 10mM Tris.HCl, pH 8.3, 50mM KCl, 2mM MgCl₂, 200µM each dNTP, 35pmol of each primer and 2.5U Amplitaq DNA polymerase. PCR reactions were overlaid with mineral oil (Sigma, Poole, Dorset, U.K.) and denatured at 95°C for 3 minutes followed by 5 cycles (60 seconds denaturation at 94°C, 60 seconds annealing/extension at 45°C) then a further 35 cycles (60 seconds denaturation at 94°C, 60 seconds annealing/extension at 55°C) and finally 7 minutes annealing/extension at 55°C. An aliquot (5%) of each reaction was analysed by agarose gel electrophoresis (3% FMC, Flowgen Instruments Ltd, Sittingbourne, Kent, U.K.). Products within the expected size range for each primer pair were excised from the gel, purified using a Wizard™ PCR Prep kit (Promega, Madison, WI, U.S.A.) and ligated into the vector pGEM-T (Promega). Clones containing an insert of the expected size were identified and their DNA sequence determined by the dideoxynucleotide method (Sequenase 2.0 kit, USB, Amersham International, Aylesbury, U.K.).

### Detection of mRNA expression

Northern analysis was performed using standard procedures. Briefly, 10µg of total RNA was separated by formaldehyde gel electrophoresis and transferred by blotting onto positively charged nylon membrane (Appligene,) then fixed by baking at 80°C and prehybridised at 42°C for 2 hours in 50% deionised formamide, 5xSSPE, 0.5xDenhardt's, 0.1% w/v SDS, 0.2mg/ml denatured salmon sperm carrier DNA and 10% Dextran sulphate. Random-primed labelled DNA probe (50ng; >10⁹ cpm/µg) was then added and hybridisation continued overnight at 42°C. The membrane was washed twice for 20 minutes in 2xSSC/0.1% SDS, followed by 20 minutes in 1xSSC/0.1% SDS at 42°C and finally 20 minutes in 0.5xSSC/0.1% SDS at 50°C before wrapping in cling-film an exposing to autoradiographic film at -70°C. Ribonuclease protection assays were performed using an RPA II kit (Ambion, AMS Biotechnology, Witney, Oxon, U.K.) according to the manufacturer's instructions. Briefly, 10µg of total RNA was hybridised overnight at 45°C to 10⁵ cpm of radiolabelled anti-sense riboprobe. Following hybridisation reactions were digested with single-strand-specific RNase and protected fragments resolved on a 6% denaturing polyacrylamide gel which was fixed for 30 minutes in 15% methanol/5% acetic acid, dried and exposed to autoradiographic film at -70°C.

### RESULTS

### Enhancement of CRF-stimulated CAMP production by immunosuppressants

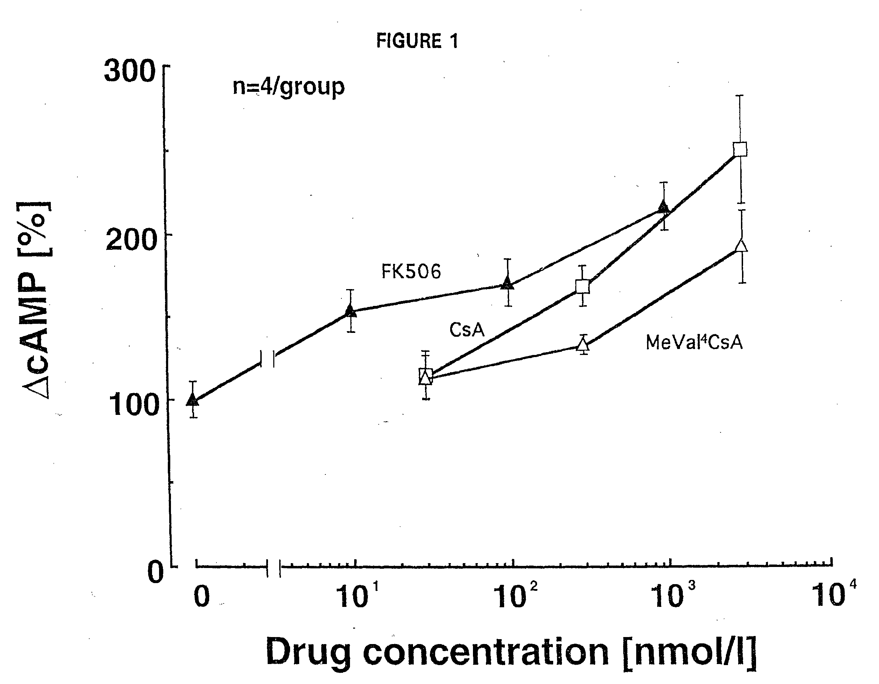

Immunosuppressant blockers of calcineurin activity, FK506, cyclosporin A and MeVal⁴-cyclosporin A enhanced CRF-induced cAMP production in a concentration dependent manner (Fig 1). The effect of cyclosporin A (Fig 2) was statistically significant (P<0.05 or less) at 2, 5 and 10 minutes after the addition of CRF, similar data were also obtained with FK506.
Enhancement of cAMP formation by FK506 was apparent at lower concentrations (0.1-10nmol/1) of CRF, while the maximal response appeared unchanged (Fig 2B).

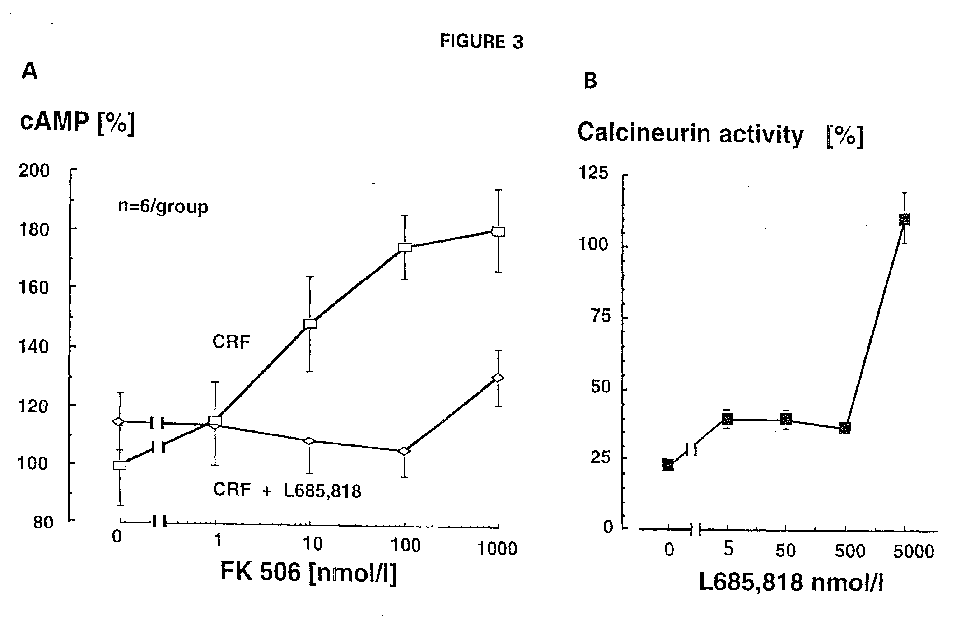

The effect of FK506 on CRF-induced cAMP production (Fig 3) could be antagonized by the FK506 analog L685,818, a potent inhibitor FKBP-12 prolyl isomerase activity which has no immunosuppressant activity and does not block calcineurin (Dumont et al, (1992) J. Exp. Med. 176:751-760) and hence, is a specific antagonist of the calcineurin inhibitory action of FK506. Importantly, this compound also blocked the inhibitory effect of FK506 on calcineurin-mediated dephosphorylation of phosphocasein (Fig 3) in AtT20 cells.

### Receptor-evoked synthesis of cAMP is under inhibitory control by intracellular Ca²⁺ and calcineurin

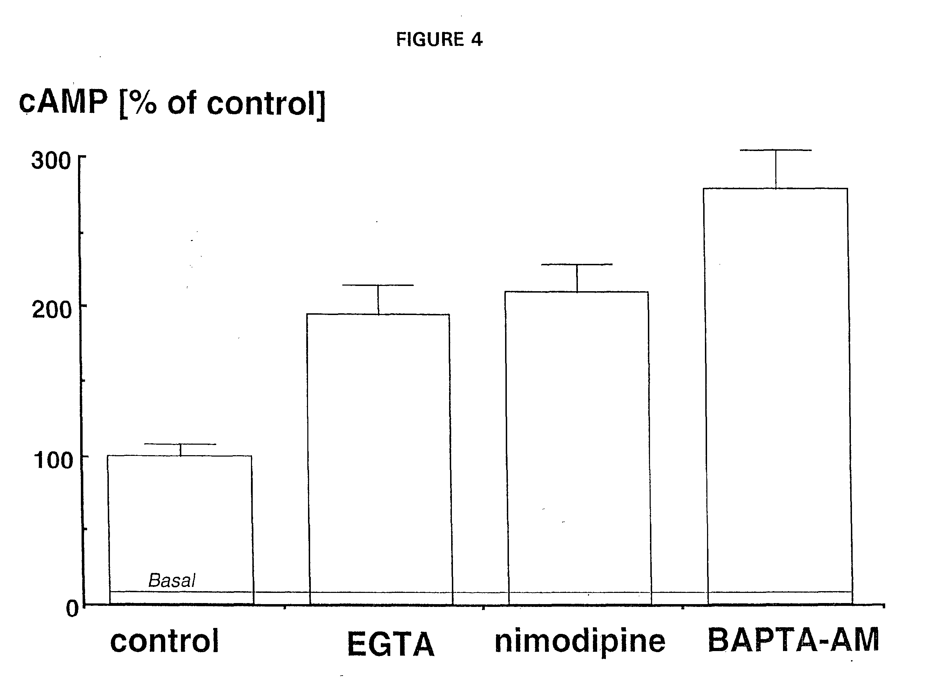

Lowering of intracellular free Ca²⁺ by a variety of methods, such as depletion by EGTA and the calcium ionophore A23187, loading of the cells with the intracellular calcium chelator BAPTA-AM and blockage of calcium channels with the dihydropyridine channel blocker nimodipine, all markedly increased the cAMP response to CRF (Fig 4). The effect of BAPTA-AM on CRF-induced cAMP formation was statistically significant (P<0.05) by 2 minutes after the addition of CRF and at all subsequent time-points studied up to 20 minutes (not shown).

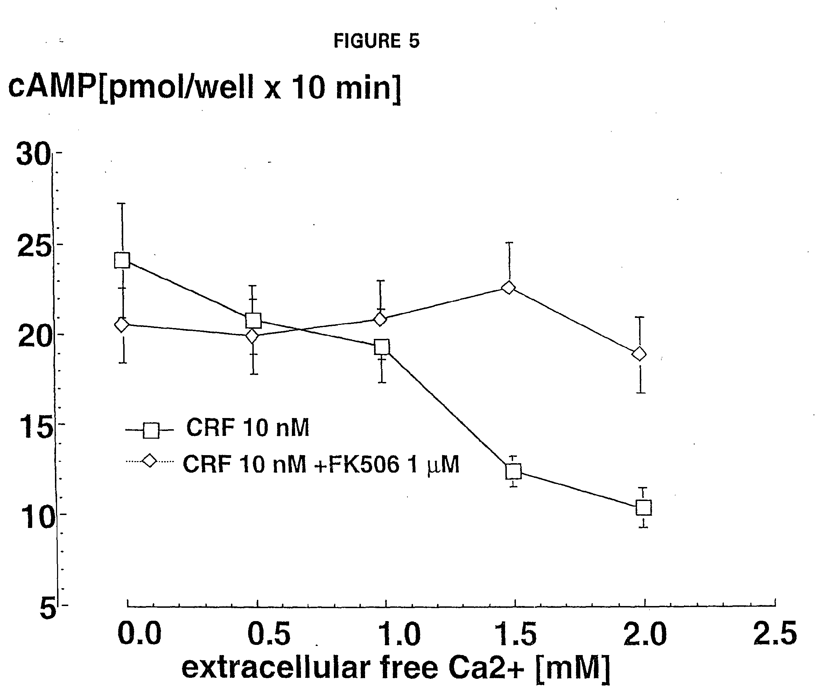

Addition of graded amounts of Ca²⁺ with CRF to cells depleted of Ca²⁺ and pretreated with the ionophore A23187 produced a concentration-dependent inhibition of CRF-induced cAMP production to levels seen in non-depleted cells incubated in medium containing 2mmol/l Ca²⁺. The effect of exogenous Ca²⁺ could be inhibited by FK506, which, in fact failed to alter cAMP accumulation in the absence of Ca²⁺ (Fig 5).

### Site and specificity of immunosuppressant action

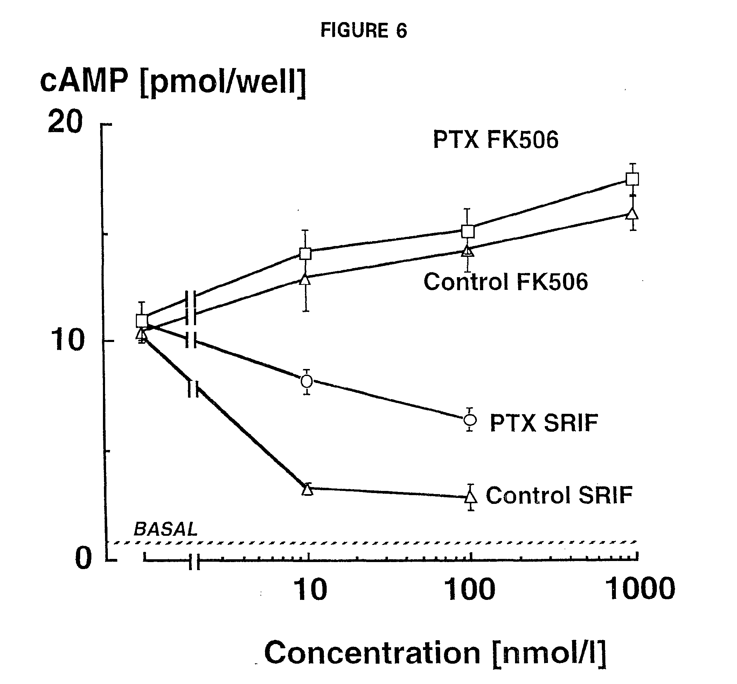

The effect of FK506 on CRF-induced cAMP formation was also evident after a 16 hours pretreatment of the cells with pertussis toxin (1µg/ml) (Fig 6) which strongly suppressed inhibitory G-protein function as assessed by the attenuation of somatostatin-mediated inhibition of cAMP formation. Pertussis toxin treatment also had no effect on the suppression of CRF-induced cAMP formation by extracelluar Ca²⁺ in Ca²⁺-depleted cells (not shown).

FK506 had no significant effect on cAMP accumulation evoked by 10 or 30µM forskolin, a drug that activates adenylyl cyclase independently of Gs. Loading of the cells with BAPTA-AM caused a small (15%), but statistically significant (P<0.05) enhancement of forskolin-evoked cAMP accumulation (not shown).

Finally, in contrast to the effects of FK506 and cyclosporin A, pretreatment with other blockers of protein phosphatases such as calyculi A (1-30nmol/l) okadaic acid (0.2-5µmol/l), caused a concentration-dependent inhibition (up to 80%) of CRF-induced cAMP accumulation (not shown and Koch et al, (1994) Cellular Signalling 6:467-473).

### Enhancement of CRF stimulated ACTH release by FK506

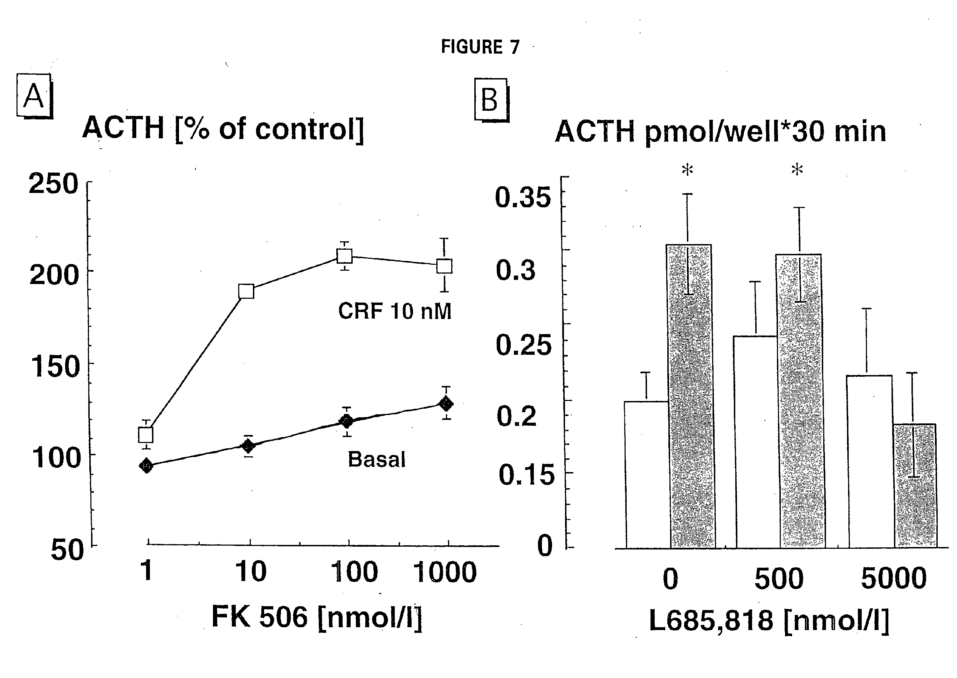

Blockage of calcineurin activity by FK506 enhanced the release of ACTH evoked by CRF (Fig 7A), and this action was prevented by L685,818 (Fig 7B). Note, that the apparent EC₅₀s of FK506 to inhibit calcineurin activity in AtT20 cells (Antoni, (1986) Endocr. Rev. 7:351-378), to stimulate ACTH release and to augment cAMP accumulation induced by CRF are all approximately lOnM.

### β-adrenergic stimulation is under similar regulation by calcineurin

Isoproterenol induces cAMP accumulation through β₂ adrenergic receptors in AtT20 cells (Heisler et al, (1983) Biochemical And Biophysical Research Communications 111:112-119). Isoproterenol induced cAMP formation was also enhanced by BAPTA-AM, FK506 and Ca²⁺-depletion in AtT20 cells (Table 1).

### Effect of immunosuppressants on cAMP accumulation in AtT20 cells correlates with the expression of a novel adenylyl cyclase mRNA

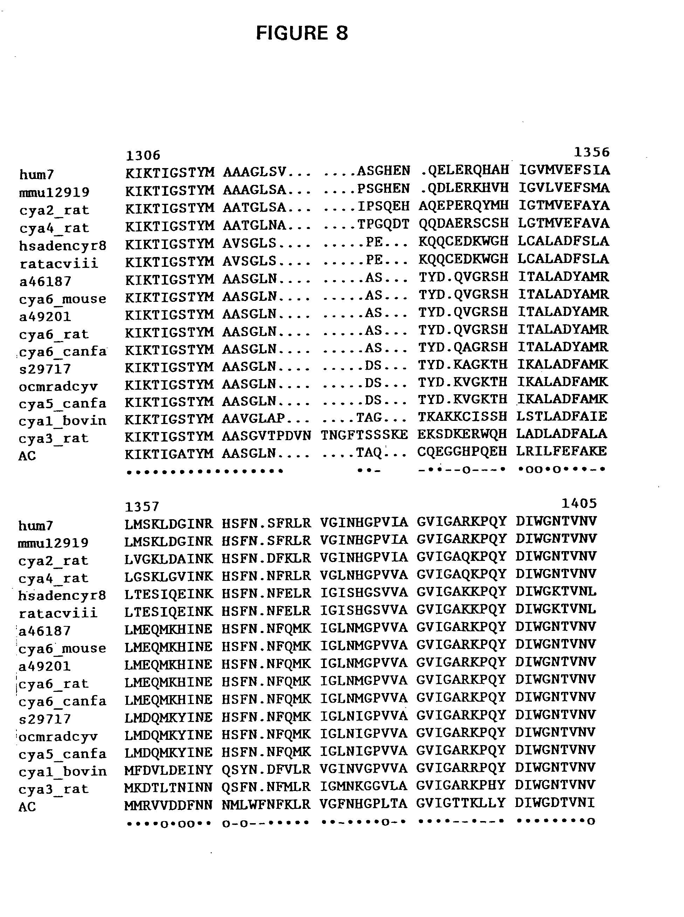

In order to determine the profile of adenylyl cyclase isoforms present in AtT20 cells two sets of degenerate oligonucleotide primers were used to analyze AtT20 cell total RNA for adenylyl cyclase related sequences by means of RT-PCR. Using primer set B a PCR product of approximately 180bp was obtained. DNA sequence analysis revealed that approximately 8% of the subcloned 180bp cDNA fragments amplified proved to be identical to Type 6 adenylyl cyclase. The majority (>90%), however, gave a novel sequence exhibiting a high level of homology with the amino acid sequences of known mammalian adenylyl cyclases present within current databases but which was not identical to any previously reported sequence (Figure 8). Type 1 adenylyl cyclase was detected in AtT20 cells using primer set A.

Northern blot analysis of total RNA using the novel adenylyl cyclase 180bp cDNA fragment as a probe indicated hybridisation to an approximately 9kb mRNA expressed in AtT20 cells. Expression of this message was not detected in NCB20 or HEK293 cell RNA.

As a more sensitive alternative mRNA expression was also assayed by ribonuclease protection of the message using a radiolabelled anti-sense riboprobe transcribed from the novel adenylyl cyclase 180bp cDNA. The presence of a ribonuclease-protected fragment migrating as a distinct, approximately 155bp band on a denaturing polyacrylamide gel indicates that the novel adenylyl cyclase mRNA is highly abundant in AtT20 cells whereas only very low levels are present in NCB20 cells and in HEK293 cells the message remains undetected.

Measurements of calcineurin activity in cell extracts from all three cell lines revealed a similar sensitivity of calcineurin protein phosphatase activity (substrate RII subunit peptide Fruman et al, (1992) Proc. Natl. Acad. Sci. U.S.A. 89:3686-3690) towards inhibition by FK506 and cyclosporin A. Furthermore, all three cell lines responded to stimulation by CRF which was enhanced by depletion of intracellular free calcium. FK506 and cyclosporin A enhanced CRF-induced CAMP formation consistently in AtT20 cells; in NCB20 cells only one out of 4 experiments gave a statistically significant effect of cyclosporin A on CRF-induced cAMP accumulation and no effects were observed in HEK293 cells.

### DISCUSSION

These data show that receptor-stimulated cAMP formation may be regulated by calcineurin, and that this regulation is associated with the expression of a novel adenylyl cyclase mRNA.

All studies of cAMP formation reported here were carried out in the presence of blocker(s) of phosphodiesterase (IBMX and/or rolipram), and hence the effects observed relate to the synthesis of cAMP rather than its degradation.

Evidence for the involvement of calcineurin in the control of cAMP accumulation is provided by the use of immunosuppressant analogs previously (Antoni et al, (1993) Biochem. Biophys. Res. Commun. 194:226-233; and Fliri (1993) Antibiotics and antiviral compounds, Chemical synthesis and modification K. Krohn, H. Kirst And H. Maasg, VCN Verlagsgesellschaft mbH, Weinheim, 229-240) shown to block calcineurin activity in AtT20 cells and T lymphocytes cells with the same order of potency that they influenced cAMP accumulation (present study). Furthermore, L685,818 an analogue of FK506 (Dumont et al, (1992) J. Exp. Med. 176:751-760) that binds to the prolyl-isomerase FKBP-12 in a manner similar to FK506, but does not give rise to a drug protein complex that inhibits the activity of calcineurin, reversed the effects of FK506 on calcineurin activity, cAMP formation as well as ACTH release. Importantly, when given alone L685,818 had no discernible effect on cAMP formation or ACTH secretion further suggesting that the changes observed upon treatment with FK506 are due to the inhibition of calcineurin. Finally, neither FK506 nor cyclosporin A were effective in cells depleted of Ca²⁺.

Taken together, these characteristics justify the conclusion that the effects of immunosuppressants described here are attributable to the inhibition of calcineurin.

The production of cAMP in AtT20 cells is under inhibitory control by [Ca²⁺]ᵢ. Stimulation with cAMP is known to elicit a rise of [Ca²⁺]ᵢ in these cells which is largely derived from the extracellular pool by influx through dihydropyridine sensitive Ca²⁺-channels (Luini et al, (1985) Proc. Natl. Acad. Sci. U.S.A. 82:8034-8038; Reisine et al, (1987) Mol. Pharmacol. 32:488-496 and Antoni et al, (1992) J. Endocrinol. 133:R13-R16), as intracellular pools of Ca²⁺ are sparse (Fiekers (1993) Abstracts of the 23rd Annual Meeting of the Society for Neuroscience 1186 Abst 488.3). Thus the [Ca²⁺]ᵢ signal is a measure of electrical activity of the cell and in addition to triggering hormone release provides feedback inhibition to the chemical messenger system that generates it. In the case of CRF-induced cAMP formation this feedback is largely mediated by calcineurin.

Several possibilities have to be considered with respect to the site of action of Ca²⁺/calcineurin in the signal transduction cascade.

An action of calcineurin at the receptor level is conceivable, however, the prevailing concept of G-protein coupled receptors (Sibley et al, (1987) Cell 48:913-922) dictates that receptor-down regulation or uncoupling is largely due to the action of protein kinases while protein phosphatases reverse this process. In contrast, the present data implicate calcineurin as an inhibitor of receptor stimulated cAMP production.

Dephosphorylation of the coupling protein Gs is also a possible site of regulation by calcineurin (Houslay (1994) GTPases in Biology B. F. Dickey and L. Birnbaumer, Springer Verlag, Berlin, Vol 108 Pt2,147-165). Once more, current evidence in the literature associates protein phosphorylation with down-regulation of G-protein function and implicates protein phosphatases in the restoration of the cellular response (Pyne et al, (1992) Biochem. Biophys. Res. Commun. 186:1081-1086; and Strassheim et al, (1994) J. Biol. Chem. 269:14307-14313).

With respect to the effector adenylyl cyclase, these proteins have lately emerged as dynamic sites of signal integration (Taussig et al, (1995) J. Biol. Chem. 270:1-4). At least two types of cyclase, Types 5 & 6 (Iyengar (1993) Advances in Second Messenger and Phosphoprotein Research B. L. Brown and P. R. M. Dobson, Raven Press Ltd, New York, 28:27-36), are inhibited by Ca²⁺ but the mechanism of this effect has not been elucidated (Yoshimura et al, (1992) Proc. Natl. Acad. Sci. U.S.A. 89:6716-6720). The inhibition of Type 5 and 6 cyclase by Ca²⁺ is most marked after stimulation by agonists such as isoproterenol in chick heart cells (Yu et al, (1993) Mol. Pharmacol. 44:689-693), or VIP in GH₄C₁ pituitary tumour cells (Boyajian et al, (1990) Cell Calcium 11:299-307), but much less prominent after activation with forskolin in GH₄C₁ cells (Boyajian et al, (1990) Cell Calcium 11:299-307). Overall this is analogous to the observations made here, which in the first instance suggest a prominent action of calcineurin at or before the level of G-protein effector coupling. However, multiple types of adenylyl cyclase coexist in all cell types analyzed to date (Yoshimura et al, (1992) Proc. Natl. Acad. Sci. U.S.A. 89:6716-6720; DeBernardini et al, (1993) Biochem. J. 293:325-328 and Hellevuo et al, (1993) Biochem. Biophys. Res. Commun. 192:311-318) and this applies to AtT20 cells as RT-PCR analysis and sequencing of the amplified cDNAs clearly showed coexpression of at least three types of adenylyl cyclase mRNA (type 1 and 6 as well as a novel isotype). Forskolin appears to activate adenylyl cyclase isotypes by different efficacies and mechanisms (Iyengar (1993) Advances in Second Messenger and Phosphoprotein Research B. L. Brown and P. R. M. Dobson, Raven Press Ltd, New York, 28:27-36), hence the relative contribution of individual cyclase isotypes to forskolin-induced cAMP may be different from receptor activated cAMP formation, and as a result forskolin-induced cAMP synthesis and receptor activated synthesis may have different pharamcological profiles.

It is unlikely that type 1 cyclase is involved in the effects of Ca²⁺ and calcineurin reported here as it is invariably stimulated by Ca²⁺, whereas the predominant effect on CRF and β-adrenergic stimulation was an inhibition by Ca²⁺. Type 6 adenylyl cyclase could be implicated because it is strongly inhibited by Ca²⁺, however, although the Type 6 isozyme is abundant in NCB20 (Yoshimura et al, (1992) Proc. Natl. Acad. Sci. U.S.A. 89:6716-6720) as well as HEK293 cells (Hellevuo et al, (1993) Biochem. Biophys. Res. Commun. 192:311-318) the stimulation of cAMP accumulation in these cells through endogenously expressed receptors for CRF was not altered upon blockage of calcineurin protein phosphatase activity. Importantly, the novel adenylyl cyclase homologue mRNA was undetectable in HEK293 cells and was found in very low amounts in NCB20 cells, whereas it appears to be the predominant adenylyl cyclase isotype mRNA in AtT20 cells. Work reported elsewhere (Paterson et al, (1995) Biochem. Biophys. Res. Commun. 214:1000-1008) reports isolation of a 4473bp cDNA from AtT20 cells which contains the complete coding sequence of this novel mouse adenylyl cyclase.

Taken together, these data suggest that the effects of calcineurin inhibitors are associated with specific adenylyl cyclase isotype previously not characterized.

The potential functional significance of calcineurin negative feedback on cAMP formation in pituitary cells is highlighted by previous findings, showing that adrenal corticosteroid inhibition of CRF-induced ACTH release involves the *de novo* synthesis of calmodulin, the calcium sensor regulatory protein of calcineurin (Shipston et al, (1992) Biochem. Biophys. Res. Commun. 189:1382-1388). Furthermore, the efficiency of corticosteroid inhibition is reduced by approximately 10-fold by immunosuppressant blockers of calcineurin (Shipston et al, (1994) Ann. N. Y. Acad. Sci. 746:453-456; and Antoni et al, (1994) J. Physiol-London 475:137-138).

In summary, calcineurin is a Ca²⁺-operated feedback inhibitor of cAMP production activated by CRF or β₂-adrenergic receptors in pituitary cortictrope tumour cells. As [Ca²⁺]ᵢ is largely derived through voltage-regulated Ca²⁺ channels in AtT20 cells, these cells exemplify a case where calcineurin functions as a link between the chemical and electrical signalling systems of the cell. These findings conform with previous reports (Armstrong (1989) Trends in Neurosciences 12:117-122; and Nichols et al, (1994) J. Biol. Chem. 269:23817-23823) suggesting that in excitable cells calcineurin is a fundamental negative feedback regulator of cellular responses as opposed to its role as a stimulatory element in non-excitable systems (Kincaid et al, (1993) Adv. Prot. Phosphatases 7:543-583). Furthermore, our findings extend the involvement of calcineurin from calcium channels and synaptic vesicle recycling to the adenylyl cyclase signalling pathway that is fundamental for intracellular signalling throughout the CNS.

### SUMMARY

The Ca²⁺- and calmodulin-activated protein phosphatase calcineurin is highly abundant in the brain. While a physiological role for calcineurin has been delineated in T lymphocyte activation, little is known about its role in excitable cells. The present study investigated the effects of immunosuppressant blockers of calcineurin on agonist-induced cAMP formation and hormone release by mouse pituitary tumour (AtT20) cells. Inhibition of calcineurin with FK506 or cyclosporin A enhanced cAMP formation and adrenocorticotropin secretion induced by corticotropin releasing-factor (CRF). Further analysis of cAMP production revealed that intracellular Ca²⁺ derived through voltage-regulated calcium channels reduces cAMP formation induced by corticotropin releasing-factor or β₂-adrenergic stimulation, and that this effect of Ca²⁺ is mediated by calcineurin. Analysis of AtT20 cell RNA indicated the co-expression of at least three species of adenylyl cyclase mRNA encoding types 1 and 6 as well as a novel isotype, which appeared to be the predominant species. In two cell lines expressing very low or undetectable levels of the novel cyclase mRNA (NCB20 and HEK293 respectively), CRF-induced cAMP formation was not altered by FK506 or cyclosporin A.
In summary, these data identify calcineurin as a Ca²⁺ sensor that mediates a negative feedback effect of intracellular Ca²⁺ on receptor-stimulated cAMP production thereby regulating the cellular response to cAMP generating agonists. Furthermore, the effect of calcineurin on cAMP synthesis appears to be associated with the expression of a novel adenylyl cyclase isoform, which is highly abundant in AtT20 cells.

### EXAMPLE 2 : DNA CLONING AND TISSUE DISTRIBUTION OF AC

### INTRODUCTION

Adenylyl cyclases convert ATP to cAMP, one of the earliest recognized intracellular messenger molecules. The family of previously known adenylyl cyclases consists of eight members (Premont (1994) Meth. Enzymol. 238:116-127). Some of these enzymes have been analyzed functionally, and appear to confer unique signal processing capacities to cells (Taussig et al, (1995) J. Biol. Chem. 270:1-4). In particular isotype specific regulation of enzymatic activity by calmodulin, calcium (through as well as independently of calmodulin), protein kinase C as well as G-protein subunits has been demonstrated (for review see Taussig et al, (1995) J. Biol. Chem. 270:1-4). In addition to functional diversity adenylyl cyclase isotypes have distinct tissue distribution profiles (Krupinski et al, (1992) J. Biol. Chem. 267:24859-24862) and particularly marked regional differences of cyclase distribution have been observed in the brain (Xia et al, (1994) Mol. Brain Res. 22:236-244; and Glatt et al, (1993) Nature 361:536-538). Collectively, these observations indicate that the particular adenylyl cyclase isotype profile of a cell is fundamentally important with respect to cellular function.

Pharmacological analysis of the cAMP response to agonist stimulation in mouse corticotroph tumor (AtT20) cells (Antoni et al, (1994) Journal Of Physiology-London 475p:137-138) has shown that calcium inhibition of cAMP accumulation in these cells is mediated by the Ca²⁺/calmodulin activated protein phosphatase calcineurin (protein phosphatase 2B). This novel feature of the cAMP response has led to the examination of the adenylyl cyclase isotype profile of AtT20 cells (Antoni et al, (1995) EMBO Journal submitted), which, amongst two known sequences (types 1 and 6), revealed the presence of a novel isotype. The present study reports the full cDNA sequence and tissue distribution of this novel adenylyl cyclase which is the predominant species of the enzyme expressed in AtT20 cells. The enzyme mRNA is also relatively abundant in the brain as well as certain peripheral endocrine organs, including the ovary and the adrenal gland.

### MATERIALS AND METHODS

### Isolation of a cDNA containing the complete coding sequence of a novel mouse adenylyl cyclase identified in AtT20 cells

AtT20 cells were grown to sub-confluency in DMEM supplemented with 10% foetal calf serum (Woods et al, (1992) Endocrinology 131:2873-2880) and total RNA was isolated from approximately 10⁷ cells using Trizol reagent (GIBCO, Paisley, U.K.) according to the manufacturer's instructions. RT-PCR was carried out using an RNA PCR kit (Perkin Elmer, Warrington, Cheshire, U.K.) as previously described (Antoni et al, (1995) EMBO Journal submitted), using degenerate oligonucleotides corresponding to a highly conserved region within the second cytoplasmic domain of previously cloned mammalian adenylyl cyclases (Krupinski et al, (1992) J. Biol. Chem. 267:24859-24862; Yoshimura et al, (1992) Proc. Natl. Acad. Sci. U.S.A. 89:6716-6720; and Gao et al, (1991) Proc. Natl. Acad. Sci. U.S.A. 88:10178-10182) A 180bp cDNA fragment of a novel adenylyl cyclase amplified from AtT20 cells (Antoni et al, (1995) EMBO Journal submitted) was used as a probe to obtain a cDNA containing the complete coding sequence by screening an oligo dT-primed cDNA library prepared from AtT20 cells and constructed in the vector ZAP II (a generous gift of Dr. M J Shipston, Edinburgh (see Shipston (1992) PhD Thesis, University of Edinburgh)) together with 5' RACE PCR. Screening of approximately 5x10⁵ clones was carried out according to standard procedures (Sambrook et al, (1989) Cold Spring Harbor Press, USA). Plaque-purified positive clones were recovered as pBluescript phagemids by excision using the helper phage ExAssist (Stratagene, Cambridge, U.K.). The insert size of these clones ranged from 1-3kb. DNA sequence was determined from several independent isolates of each clone using Exo III/Mung Bean nuclease digestion and/or clone-specific primers with the Sequenase 2.0 kit (USB, Amersham International, Aylesbury, U.K.). Analysis of the DNA sequence data generated revealed the presence of an open frame which lacked a suitable initiator codon.

Two rounds of 5' RACE-PCR were performed to obtain the remaining of the open reading frame using a 5' RACE System kit (GIBCO, Paisley, U.K.) according to the manufacturer's instructions and summarised in brief as follows. Based on the DNA sequence at the 5' end of the largest cDNA clone isolated by library screening (jp134) a set of three nested anti-sense oligonucleotides were designed. The first of these (most proximal to the 3' end of the cDNA isolated; primer 1: CGTCAATGACCTCAAAGCC (SEQ ID NO: 9)) was used to prime synthesis of first strand cDNA by reverse transcription of 0.8µg of total RNA isolated from AtT20 cells in a 25µl reaction containing 20mM Tris.HCl (pH 8.4), 50mM KCl, 3mM MgCl₂, 10mM DTT, 100nM primer 1, 400µM each dNTP and 200U Superscript II Reverse Transcriptase at 42°C for 30 minutes. First strand cDNA was purified away from this primer using the GlassMax purification system (GIBCO) and one-fifth of the purified cDNA 3'-tailed with dCTP using 10U terminal deoxynucleotide transferase (TdT) in a 25µl reaction containing 20mM Tris.HCl (pH 8.4), 50mM KCl, 1.5mM MgCl₂, 200µM dCTP at 37°C for 15 minutes. Following heat inactivation of the TdT a second strand cDNA synthesis reaction was primed by the anchor primer supplied with the 5' RACE system kit present at 200nM and carried out as for first strand synthesis. Doublestranded cDNA was subsequently purified using the GlassMax system and one-fifth of this preparation used as a template in PCR containing 20mM Tris.HCl (pH 8.4), 50mM KCl, 1.5mM MgCl₂, 200mM each dNTP, 200nM each of universal amplification primer (UAP; supplied with the kit) and jp134-specific nested anti-sense primer 2 (SEQ ID NO: 10) (GCCTCTGCACAGCTGCAGTGGGACTCC) and 0.03U/µl *Amplitaq* DNA polymerase (Perkin Elmer) (parameters for all the PCR reactions can be found in Table II). A second round of PCR using 0.05% of the primary PCR reaction (or 0.05% of size-selected primary PCR products) as a template was performed as described above except jp134-specific nested anti-sense primer 3 (SEQ ID NO: 11) (CCTGGCAGAACTGCTCGATGGCTTTTATCATGC) was substituted for primer 2. This first round of 5' RACE-PCR yielded a product of approximately lkb which was purified from an agarose gel and ligated into the plasmid vector pGEM-T (Promega, Madison, WI.,U.S.A.). Although DNA sequence analysis of this fragment (from both strands of two independent clones) extended the open reading frame observed in jp134 by 842bp no suitable initiator codon was yet apparent. Another set of three anti-sense primers was designed on the basis of sequence data from the 5' end of the lkb fragment obtained from the first round and used in a second round of 5' RACE-PCR performed as described above except that 1) the first strand cDNA was synthesized at 50°C for 30 minutes and 2) substitution of primers 1, 2 and 3 with 4 (SEQ ID NO: 12) (GGAGAAGCTTCCTACTTG), 5 (SEQ ID NO: 13) (GTGGCCGTGAGAGTATGATTGGAGCTGTC) and 6 (SEQ ID NO: 14) (GTCCAAACCTGAAACTGCGCACGCAG), respectively. This yielded a product of approximately 650bp which, when cloned into pGEM-T and several independent isolates sequenced, completed the open reading frame encoding the novel mouse adenylyl cyclase.

### Detection of mRNA expression

Northern analysis was performed using standard procedures. Briefly, 20µg of total RNA was separated by formaldehyde gel electrophoresis and transferred by blotting onto positively charged nylon membrane (Appligene, Ullkirch, France) then fixed by baking at 80°C and prehybridised at 42°C for 2 hours in 50% deionised formamide, 5xSSPE, 0.5xDenhardt's, 0.1% w/v SDS, 0.2mg/ml denatured salmon sperm carrier DNA and 10% Dextran sulphate. Random-primed labelled DNA probe (50ng; >10⁹cpm/µg) was then added and hybridisation continued overnight at 42°C. The membrane was washed twice for 20 minutes in 2xSSC/0.1% SDS, followed by 20 minutes in lxSSC/0.1% SDS at 42°C and finally 20 minutes in 0.5xSSC/0.1% SDS at 50°C before wrapping in plastic an exposing to autoradiographic film at -70°C. Ribonuclease protection assays were performed using an RPA II kit (Ambion, AMS Biotechnology, Witney, Oxon, U.K.) according to the manufacturer's instructions. Briefly, 10µg of total RNA was hybridised overnight at 45°C to 10⁵ cpm of radiolabelled anti-sense riboprobe. Following hybridisation, reactions were digested with single-strand-specific RNase and protected fragments resolved on a 6% denaturing polyacrylamide gel which was fixed for 30 minutes in 15% methanol/5% acetic acid, dried and exposed to autoradiographic film at -70°C.

### RESULTS AND DISCUSSION

### Analysis of the cDNA and amino acid sequence

The sequence of the full length cDNA isolated from AtT20 cells (sense strand on top) and the deduced primary structure of AC are shown in SEQ ID No 1. A 4473bp cDNA containing a 4062bp open reading frame which encodes a 1352 amino acid protein. A stretch of 10 amino acids near the 5' end of the cDNA strongly resembles the Kozak consensus sequence for initiation of translation and contains the presumed initiator codon of the novel adenylyl cyclase protein. The presence of a translational stop codon upstream of this methionine residue suggests that the complete open reading frame encoding this protein has been cloned. The deduced amino acid sequence between residues 1105 and 1192 appears to be identical (except for a single asparagine to aspartic acid switch at 1191) to a short sequence derived from mouse brain RNA reported in preliminary form (Premont (1994) Meth. Enzymol. 238:116-127) and designated as AC. Although this region of the enzyme contains at least two highly preserved stretches of amino acid sequence common to all cyclases, on the basis of the abundance of AC mRNA in the brain (see below) it seems plausible that the full sequence reported here corresponds to the same enzyme and will subsequently referred to as AC.

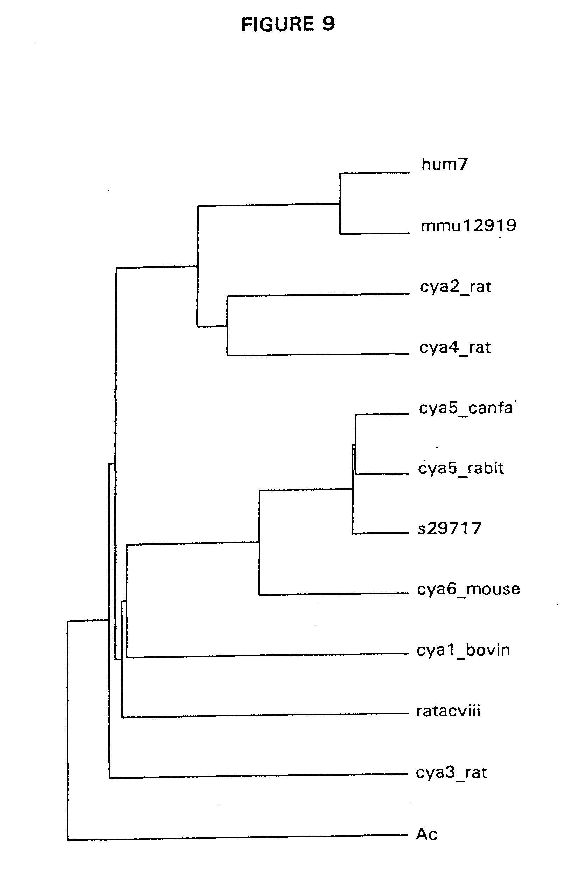

While the key adenylyl cyclase consensus sequences thought to be involved in catalytic activity (Taussig et al, (1995) J. Biol. Chem. 270:1-4) are well conserved in AC, overall amino acid sequence homology comparisons indicate that AC is not sufficiently similar to currently known cyclases to be placed within one of the previously suggested subfamilies as illustrated by Figure 9. Therefore AC represents a sixth adenylyl cyclase subfamily. It cannot be excluded at present that perhaps other members of this subfamily exist.

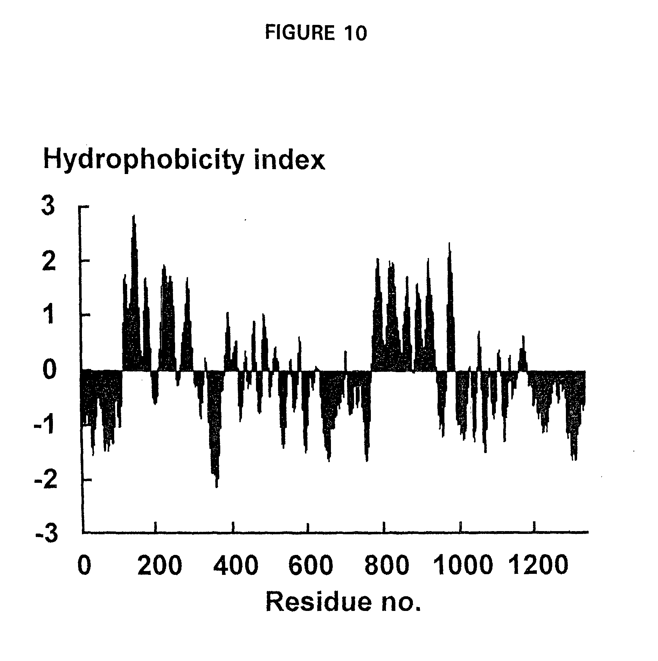

Hydrophobicity plots (Kyte et al, (1982) J. Mol. Biol. 157:105) return the previously reported adenylyl cyclase structure (Figure 10) a short N-terminal loop followed by two homologous segments each consisting of a hydrophobic chain with 6 intermembrane helices and a large hydrophilic (putative cytoplasmic) loop. The first cytoplasmic loop appears considerably longer (by about 130 amino acids) in AC than previously reported for other isotypes.

Analysis of putative phosphorylation sites (Pearson et al, (1991) Meth. Enzymol. 200:61-81) indicates several strong consensus protein kinase A and protein kinase C sites as well as two casein kinase phosphorylation sites (Table 3). Elements of the functionally relevant calmodulin binding site identified in ACtypel (Wu et al, (1993) J. Biol. Chem. 286:23766-23768 and Vorherr et al, (1993) Biochemistry (U.S.A.) 32:6081-6088) can be discerned on the basis of conserved amino acid residues at positions 504-505 in the first cytoplasmic domain. However, two important basic residues Lys500 and Lys497 which are part of the calmodulin binding site in ACtypel are substituted by Gly in AC making calmodulin binding unlikely in this region of the enzyme.

### Distribution of mRNA for AC in rat tissues and mouse brain

Ribonuclease protection analysis of rat tissues showed hybridizion of total RNA with probe JP114 gave a 125bp double stranded product that was most abundant in brain, with significant hybridizing activity in the anterior pituitary gland, the ovary, the adrenal gland, the lung and the kidney. Liver, pancreas,spleen, thymus, heart gave no detectable signal, suggesting that the distribution of the enzyme is highly tissue specific.

Gross dissection of the mouse brain suggests that AC mRNA is most abundant in the cortex, striatum and the hippocampus, with lower levels in the cerebellum and much lower but detectable concentrations in the olfactory bulb, the diencephalon, the brain stem and the pituitary gland. It is of note that the RNAse resistant hybrid in mouse tissues and AtT20 cells is larger, about 155bp, than in rat tissues indicating interspecies sequence variation(s) in this region of the enzyme.

Northern mRNA analysis showed an approximately 9kb size RNA in AtT20 cells that hybridizes with a JP114 antisense ³²P-DNA probe.

In summary, the present results show the existence of a further member of the adenylyl cyclase family of proteins, which has a restricted tissue distribution and a distinct regional pattern in the brain. The functional properties of this cyclase have yet to be explored in detail. Pharmacological analysis (Antoni et al, (1994) Journal Of Physiology-London 475p:137-138 and Antoni et al, (1995) EMBO Journal submitted) indicates that AC is inhibited by Ca²⁺ through calcineurin and that this may be relevant for corticosteroid inhibition of corticotropin secretion in anterior pituitary cells (Shipston et al, (1994) Ann. N.Y. Acad. Sci. 746:453-456). Furthermore, the abundance of AC mRNA in the striatum and hippocampus, where calcineurin is particularly abundant relative to other regions of the brain (Kuno et al, (1992) J. Neurochem. 58:1643-1651) is of interest with respect to synaptic function.

### SUMMARY

The cDNA of a novel adenylyl cyclase isotype (AC) has been cloned and sequenced from the mouse pituitary corticotrope tumour cell line AtT20. Adenylyl cyclase cDNA sequences were amplified from AtT20 cells using degenerate primers bracketing a highly conserved motif near the carboxyl terminus region of the protein. The majority of the sublcloned amplified cDNA products revealed a novel sequence which was utilized to screen an AtT20 cell cDNA library, from which a 3120bp clone was isolated and sequenced. The full length coding sequence was obtained upon two rounds of 5' RACE-PCR, yielding a 4473bp long cDNA containing an open reading frame of 4062bp which encodes a protein of 1352 amino acids. The hydrophobicity profile of this protein resembles that of other members of the adenylyl cyclase family in that two sets of six hydrophobic, putatively membrane-spanning regions are predicted as well as a large central cytoplasmic loop and long C-terminal cytoplasmic tail. Amino acid sequence comparisons suggest that this novel enzyme is quite distinct from other known mammalian adenylyl cyclases and cannot be easily assigned to any of the previously observed subfamilies. Tissue distribution of mRNA was examined by RNAse protection assay and indicated the highest abundance of this novel adenylyl cyclase to be in the brain followed by anterior pituitary gland, ovary and adrenal gland, which appeared to express approximately equal levels. Low level expression of this mRNA was detected in lung and kidney while in the heart, liver and pancreas none was apparent. Within the brain, relatively high levels of novel adenylyl cyclase mRNA were detected in cortex, hippocampus, striatum, cerebellum, with much less in diencephalon, olfactory bulb and the brain stem.

### EXAMPLE 3 : FUNCTIONAL PROPERTIES OF AC

### METHOD

The complete coding sequence of AC was subcloned into the eukaryotic expression vector pcDNA3 (Invitrogen). The cDNA clone for the CRF₁ receptor cloned into the vector pcDNAI (Chang et al, (1993) Neuron 11:1187-1195) was a gift of Drs. R. V. Pearse II and M. G. Rosenfeld (University of California, San Diego). Both expression plasmids (10µg) were transfected into SV40-transformed monkey embryonic kidney (COS-7) cells grown in DMEM and 10% fetal bovine serum to 70% confluency in 75cm² flasks following the double DEAE-dextran transfection protocol (Ishikawa et al, (1992) Nucl. Acids Res. 20:4367), while only AC cDNA was transfected into adenovirus-transformed human embryonic kidney (HEK293) cells as these express endogenous receptors for CRF (F. A. Antoni, unpublished data). In control transfections pcDNA3 vector DNA replaced the AC expression construct. Forty-eight hours after the second transfection the cells were harvested in Hank's Ca²⁺ and Mg²⁺ free balanced salt solution containing 0.1% EDTA and centrifuged at 200xg, for 10 minutes. Following resuspension the cells were centrifuged again to remove serum proteins and resuspended in lml of HEPES (25mmol/l, pH 7.4) buffered Hank's balanced salt solution and a 50µl aliquot was removed for the measurement of protein content. The cells were then diluted further with 4ml of HEPES buffered Hank's solution containing 0.25% BSA, and preincubated for 1 hour at 37°C under air. Subsequently the cells were pelleted again and distributed (final concentration 3 x 10⁵ cells/ml) into polypropylene vials and processed as AtT20 cells except that lmmol IBMX and 0.1mmol/l rolipram were used as inhibitors of cAMP-degrading phosphodiesterases (see Example 1).

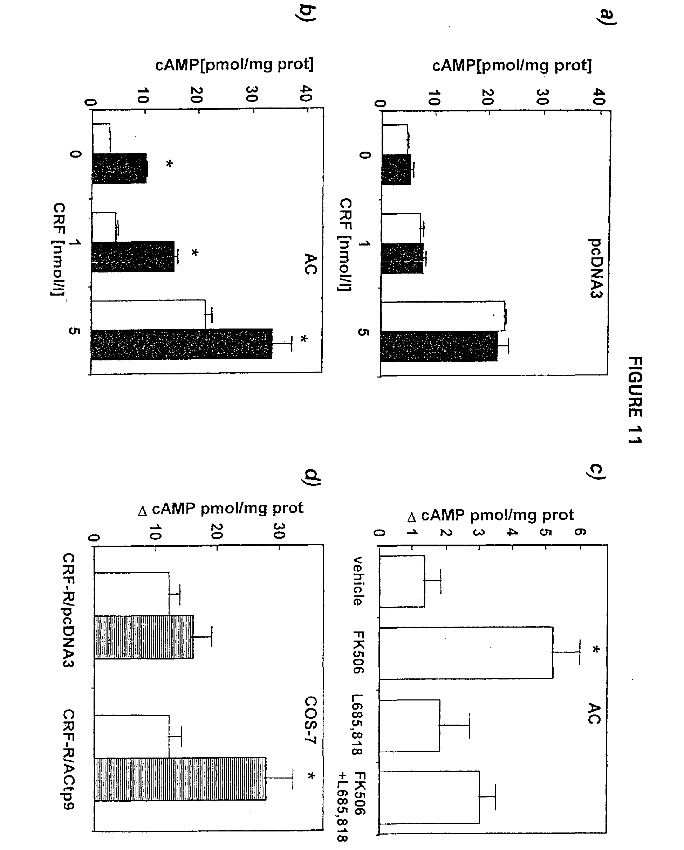

The functional properties of the cloned AC10 were investigated in transiently transfected HEK293 and COS7 cells (Figure 11). In HEK293 cells which have endogenous receptors for CRF, unstimulated levels of cAMP and the cAMP response to CRF were unchanged upon transfection with AC (Figure 11a and b). However, preincubation with FK506 increased unstimulated cAMP levels 3-fold and significantly enhanced the cAMP response to 1 but not to 5 (Figure 11b) or 25nmol/l CRF (not shown). These effects were specific to cells transfected with AC (Figure lla and b). Surprisingly, in cells transfected with AC 100µmol/l L-685,818 increased unstimulated cAMP levels to the same extent as 2µmol/l FK506, while having no effect in sham-transfected or pcDNA3-transfected cells (not shown). Importantly, L-685,818 had no significant effect on CRF-induced cAMP production but blocked the enhancement of the response to lnmol/1 CRF by FK506 in cells transfected with AC (Figure 11c). Slightly different results were obtained in COS7 cells. In this system unstimulated cAMP production in cells transfected with AC cDNA (without or in combination with the CRF receptor) was 1.9-fold higher than in controls receiving vector DNA (unstimulated cAMP [pmol/mg protein] in COS7 cells transfected with CRF receptor and pcDNA3 : 20.9±0.2; with CRF receptor and AC cDNA: 38.2 ±3.4; mean±SEM, n=4/group P<0.001, Student's t-test, representative of 4 separate experiments) whereas the increment produced by CRF (1-25nmol/l) was not consistently altered (Figure 11d and data not shown). Preincubation with cyclosporin A significantly and selectively enhanced the cAMP response to lnmol/l CRF in cells transfected with AC (Figure 11d).

These data confirm that the cDNA for AC encodes a cAMP generating enzyme. At this point the cause of the differences in unstimulated cAMP production between COS7 cells and HEK293 cells is unknown, however, such discrepancies are not unprecedented in eukaryotic expression systems (Premont (1994) Meth. Enzymol. 238:116-127; and Adie et al, (1994) Biochem. J. 303:803-808). With respect to the paradoxical effect of L-685,818 on unstimulated cAMP levels, it is likely that it has some calcineurin inhibiting activity at a high concentration (100µmol/l) which may be sufficient to enhance basal cAMP production. However, this small inhibitory effect is not enough to cause a functionally relevant reduction of activity when calcineurin is activated by the cAMP-induced increase of intracellular free Ca²⁺ that is known to occur upon a cAMP stimulation in both HEK293 and COS7 (Lin et al, (1995) Mol. Pharmacol. 47:131-139; and Widman et al, (1994) Mol. Pharmacol. 45:1029-1035 (1994)) cells. The consistent finding of the transient transfection experiments is that cAMP production in response to low CRF concentrations is enhanced by immunosuppressant blockers of calcineurin and, in the case of FK506, this is blocked by the antagonist L-685,818. In this respect the findings with cloned and transiently transfected AC are in good agreement with the findings in AtT20 cells and with pilot studies in HEK293 cells stably transfected with AC (Antoni et al, unpublished data).

### SEQUENCE COMPARISON

### METHOD:

Comparisons with other adenylyl cylases were made using the GCG package available from the Human Genome Mapping Programme, Cambridge U.K. Initial alignments with FKBP12 were made using GeneJockeyII (Biosoft, Cambridge U.K.), and adjusted visually by the aid of the programme.

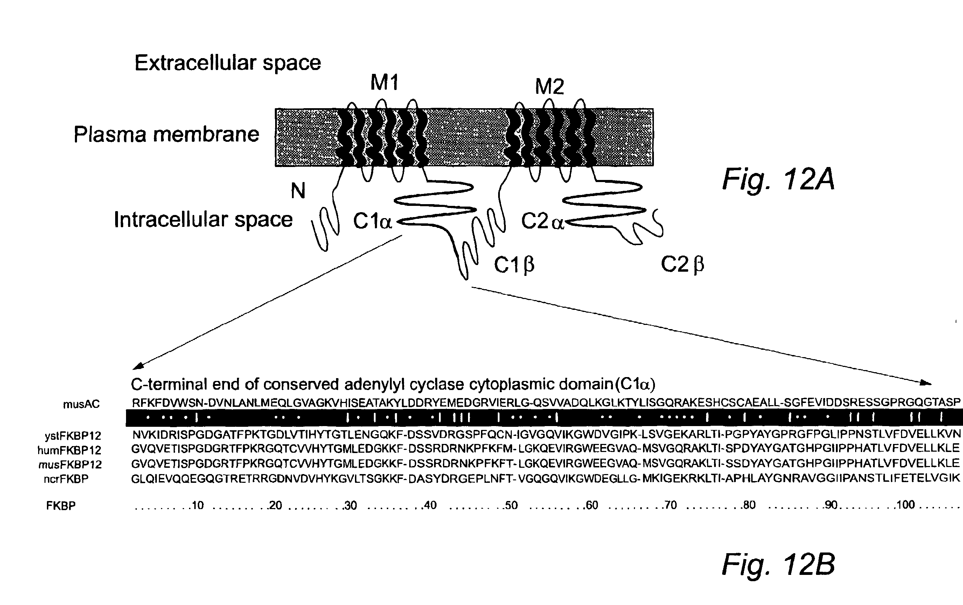

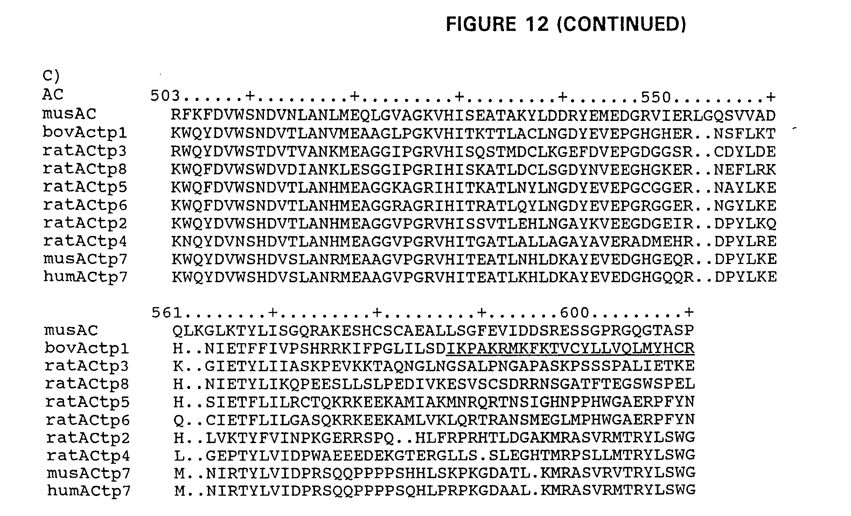

Closer examination of the primary sequence of AC revealed that residues 503 to 610 in the first cytoplasmic loop show approximately 40% similarity with FKBP12 (Figure 12a and b). The first part of this FKBP12-like segment consists of the C-terminal segment of the C1α domain which is highly conserved in all adenylyl cyclases (residues 503-570 in Figure 12c) (Taussig et al, (1995) J. Biol. Chem. 270:1-4). The second part is the beginning of the long non-conserved segment (C1β domain). Importantly, some of the sequence identities correspond to Asp37, Gly86, Phe87, Ile90 in FKBP12 (Figure 12b) and are amino acid residues of key importance for the high-affinity interaction of the FK506/FKBP12 complex with calcineurin (Aldape et al, (1992) J. Biol. Chem. 267:16029-16032; Yang et al, (1993) J. Am. Chem. Soc. 115:819-820; and Braun et al, (1995) FASEB J. 9:63-72). The best overall sequence similarity is observed with yeast FKBP12, which in fact has been shown to bind calcineurin in the absence of FK506 (Cardenas et al, (1994) EMBO J. 13:5944-5957). Given the prominent actions of calcineurin blocker immunosuppressants on cAMP formation in systems expressing AC it seems reasonable to suggest that AC ₍₅₀₃₋₆₁₀₎ may be a physiologically relevant docking site for calcineurin.

Adenylyl cyclase type 1 (ACtp1) is also thought to interact directly with a protein co-factor distinct from G-protein subunits as this cyclase is markedly stimulated by Ca²⁺/calmodulin (for review see Taussig et al, (1995) J. Biol. Chem. 270:1-4). Residues 495-522 of ACtpl (495-518 shown underlined in Figure 12c) bind calmodulin with nanomolar affinity (Vorherr et al, (1993) Biochemistry (USA) 32:6081-6088). Moreover, point mutations of Phe₅₀₃ and Lys₅₀₄ virtually abolish the stimulatory effect of Ca²⁺/calmodulin on this cyclase (Wu et al, (1993) J. Biol. Chem. 286:23766-23768). Intriguingly, the position of residues 495-522 along the cytoplasmic loop of AC corresponds to the C-terminal part of the FKBP12-like sequence in AC (Figure 12c). It seems therefore that the junction between the C1α and C1β domains in adenylyl cyclase 1 and in AC is a site of allosteric regulation by calcium binding proteins. In the case of AC a strong candidate for such a regulatory protein is calcineurin.

### AC is enriched in nerve cells

### METHOD

Northern analysis of total RNA (20µg per lane) was performed by transfer onto positively charged nylon membrane (Appligene, Ullkirch, France) then hybridized to radio-labelled probe in 50% formamide and washed according to standard procedures (Sambrook et al, Molecular Cloning: a Laboratory Manual Ed. 2 (Cold Spring Harbor Press, USA, 1989)). The cDNA probes used correspond to amino acid residues 1-195 (JP164) and 1105-1165 (JP114).

Ribonuclease protection assays were performed using an RPA II kit (Ambion, AMS Biotechnology, Witney, Oxon, U.K.) according to the manufacturer's instructions using radio-labelled anti-sense riboprobe derived from JP114 or JP142 (corresponding to amino acids 320-478). The AC probes showed no significant cross hybridisation in Southern analysis with adenylyl cyclase isotype cDNAs 1, 2, 3, 5 or 6. A 250bp probe hybridising with β-actin was used as the internal standard to correct for differences in RNA loading. Blots and gels were exposed to X-ray film as well as to Molecular Dynamics Phosphorimager casettes and quantified with the ImageQuant software using the 28S RNA band and the β-actin band as standards for RNA loading in Northern and RNase protection analysis, respectively. Division of the integrated volume of pixels of the selected radiolabelled band with the integrated volume of the internal standard band yields the relative hybridisation intensity, which was used to compare the intensity of labelled RNA bands within blots. The relative abundance of the protected RNA species in a given tissue is expressed as the percentage of the relative hybridisation intensity of the whole brain RNA band run in the same assay.

Importantly, we have discovered that AC mRNA is present in relatively high levels in the hypothalamic paraventricular nucleus that contains the major contingent of CRF41 and vasopressin producing neuroendocrine motoneurones and controls the secretion of ACTH by the anterior pituitary gland. In addition, the levels of mRNA are apparently controlled by adrenal corticosteroids, so that lowering of adrenocortical steroid levels causes an increased expression of AC mRNA. This reinforces the notion that AC is expressed in areas of the brain important for the negative feedback action of adrenal corticosteroids, in the hypothalamic-pituitary-adrenocortical axis. This system is a servomechanism where, when corticosteroid feedback is detected as inadequate by the relevant as yet unidentified components of the system, the levels of AC mRNA are increased.

### RESULTS

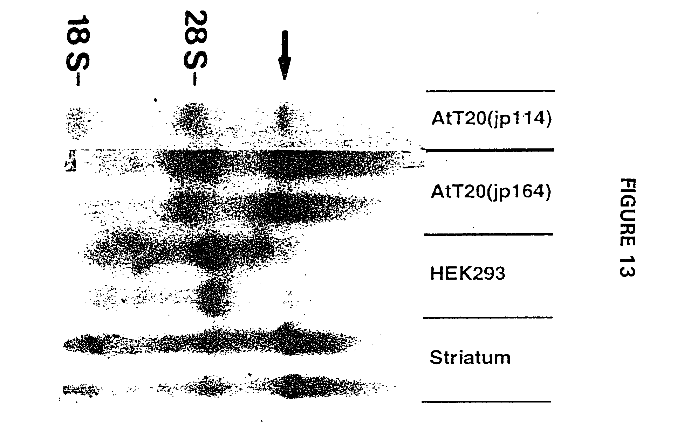

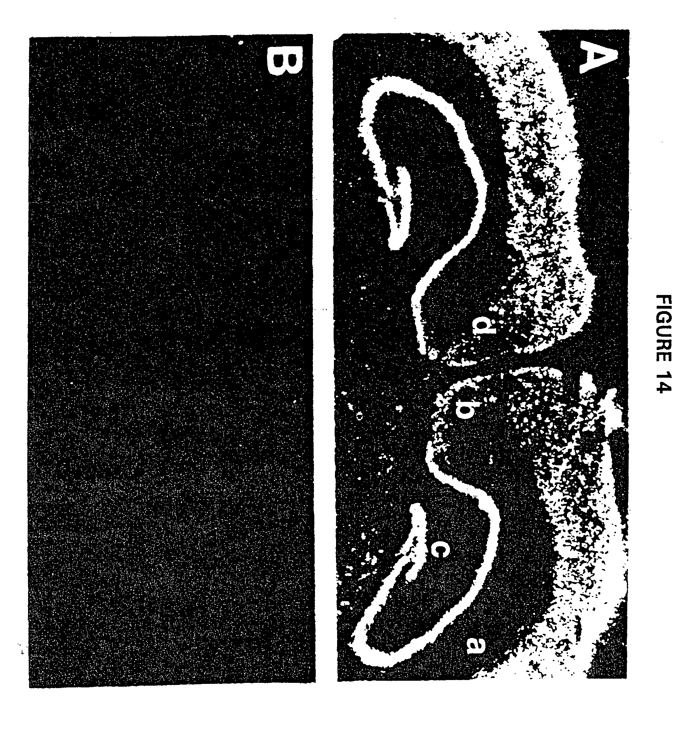

Existence of a calcineurin-regulated adenylyl cyclase is of great potential significance for brain function as calcineurin constitutes about 1% of total brain protein (Klee et al, (1988) Adv. Enzymol. 61:149-200). AC is in fact highly abundant in the brain: Northern blot analysis shows the presence of a 9kb mRNA in mouse striatum and AtT20 cells (Figure 13). RNAse protection anaylsis of dissected regions of the mouse brain suggest the relative abundance of AC mRNA to be hippocampus ≥ striatum ≥ cortex ≥ cerebellum ≥ olfactory bulb > brain stem > diencephalon ≥ anterior pituitary gland (range 3-fold, see Figure 13 for methods). These findings indicated a primarily neuronal localisation which was confirmed by *in situ* hybridisation histochemistry, showing robust AC mRNA bybridisation in hippocampal neurons (Figure 14).

RNAse protection assay also shows the presence of AC10 mRNA, amongst other tissues, in mouse kidney, thymus and spleen (relative abundance 50, 10 and 8%, respectively if brain = 100%, see Figure 13 for methods) all of which are important targets of immunosuppressants (Schreier et al, (1993) Transplant. Proc. 25:502-507; and Dumont et al, (1992) J. Exp. Med. 176:751-760).

### Functional implications of calcineurin regulated adenylyl cyclase

In summary, the present results show the existence of a new member of the adenylyl cyclase family of proteins that is abundant in the brain and is inhibited by calcineurin. The intricate control of calcineurin activity by its EF-hand protein β-subunit as well as additional regulation by calmodulin (Schreier et al, (1993) Transplant. Proc. 25:502-507) suggest a potentially fundamental role for AC in situations where fine interplay between intracellular Ca²⁺ and cAMP determines cellular function (Cooper et al, (1995) Nature 374:421-424). Furthermore, the abundance of AC mRNA in the striatum and hippocampus, where calcineurin is particularly enriched relative to other regions of the brain (Kuno et al, (1992) J. Neurochem. 58:1643-1651) is of major interest with respect to synaptic function. Finally, it remains to be explored whether or not the currently known Ca²⁺ regulated cyclases are also subject to control by calcineurin. A recent study has in fact reported the facilitation of cAMP formation by Ca²⁺/calcineurin suggestive of calcineurin control of a Ca²⁺-stimulated adenylyl cyclase (Baukal et al, (1994) J. Biol. Chem. 269:24546-24549).

### EXAMPLE 4 : HUMAN AC9

### Isolation of a complete human AC9 cDNA clone

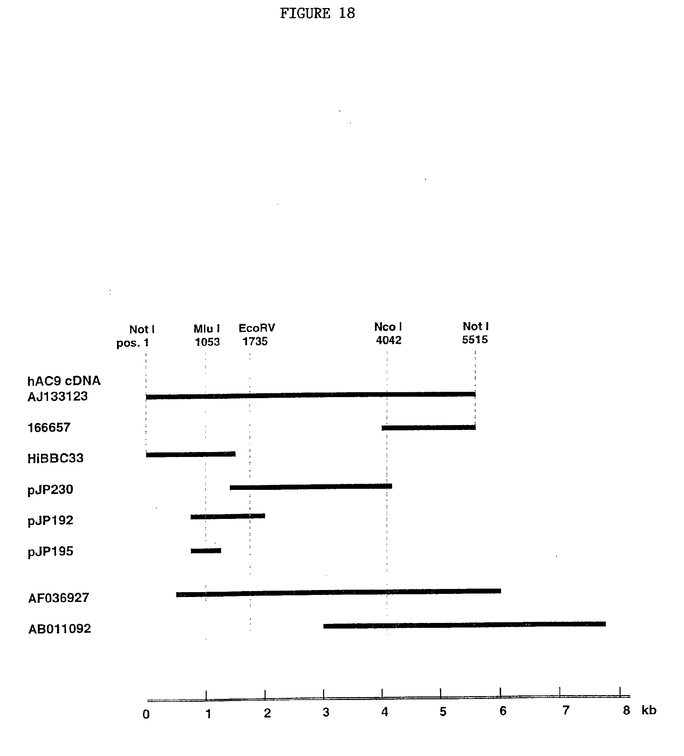

The assembly of a full length human AC9 cDNA clone is represented in Figure 18. The NCBI BLAST computer program (http://www.ncbi.nlm.nih.gov/BLAST/) was used to search the GenBank/EMBL and dbEST (expressed sequence tag) DNA sequence databases for human EST clones exhibiting similarity to mouse AC9 cDNA sequence Z50190. Two entries of interest were initially identified under GenBank/EMBL Accession Numbers R88632 and T08314. The EST cDNA clones, IMAGE 166657 (from adult human brain) and HIBBC33 (from infant human brain), from which these sequences were derived, were respectively obtained from the MRC Human Genome Mapping Project Resource Centre (MRC HGMP-RC, Cambridge, UK) and The Institute for Genome Research (TIGR, Gathersburg, Maryland, USA, distributed by ATCC, Rockville, Maryland, USA). Due to the directed nature of cDNA cloning (Adams et al, (1993) Nat. Genet. 4: 373-80) the database sequence entries for these clones was presumed to represent part of the sense strand of the cDNA. This holds true for sequence R88632 (258bp) which exhibits 77% sequence identity with nucleotides 3855 to 4113 of mouse AC9 sequence Z50190. However, sequence T08314 (228bp) exhibited 82% identity with residues 682-909 on the anti-sense strand of mouse AC9 sequence Z50190 suggesting that this cDNA was cloned randomly rather than directionally. This insert may have arisen due to fragmentation of cDNA prior to vector ligation or as a result of mis-priming of cDNA synthesis from a source other than the polyadenylated 3' end of mRNA although a complete sequence representing the primer of cDNA synthesis (Adams et al, (1993) supra) was not found. The inserts of both EST clones were completely sequenced by the dideoxynucleotide method using universal and sequence-specific primers using an ABI Prism dye terminator DNA cycle sequencing kit (Perkin Elmer), resolved on a 4.75% acrylamide denaturing gel and data collected on an Applied Biosystems/Perkin Elmer 373 Stretch automated sequencer. Sequences were analyzed independently using Gene Jockey II program (Biosoft, Cambridge, U.K.) for the assessment of errors and visual confirmation of base calling. IMAGE clone 166657 was found to contain the 3' part of an open reading frame (ORF) encoding human AC9 followed by an untranslated region (UTR) and polyadenylated tail. Clone HIBBC33 contained 5' UTR sequence and the 5' part of the ORF encoding human AC9. To complete cloning of the human AC9 cDNA, the gap between these EST cDNA clones containing the 5' and 3' parts of the ORF were isolated by polymerase chain reaction (PCR) amplification using the High Fidelity Expand PCR system (Boehringer Mannheim/Roche) with sequence-specific primers. A PCR product of 2860bp (1309-4169; pJP230) was amplified from a denatured human skeletal muscle cDNA library (Clontech) using primers 5'-GAC CTT TGG CTA CCA TTT CCG GGA TG-3' (SEQ ID NO: 100) and 5'-CTT CGC TCA CCT GGA TGC GGC ACT C-3' (SEQ ID NO: 101) respectively. The ends of this PCR product were blunt ended with T4 DNA polymerase (Boehringer Mannheim/Roche) prior to ligation into the SmaI site of plasmid vector pUC9 and subsequently sequenced as above. Additional overlapping clones of PCR products derived from human AC9 cDNA (853-1196; pJP 192 and 853-1167; pJP195), amplified as above or from a pool of denatured human cDNA libraries (Clontech) using primers based on mouse AC9 cDNA (upstream primer 5'-GGA GCG CTG CTT TCC GCA G-3' (SEQ ID NO: 102) with downstream primers 5'-CAT GTT CAC CAT CTC TTT CTT CTC C-3' (SEQ ID NO: 103) and 5'-GTG GCC GTG AGA GTA TGA TTG GAG CTG TC-3' (SEQ ID NO: 104) respectively), were propagated in the TA cloning vector pCRII (Invitrogen) and sequenced as above. The complete human AC9 cDNA (5515bp, including 5' and 3' Not I cloning sites) was assembled from the above clones using recombinant DNA techniques. Internal PCR products pJP192 and pJP230 were joined at EcoRV site; 1735. The resulting clone was joined to the 5'UTR, initiation codon and 5' part of the ORF in clone HIBBC33 at MluI site; 1053. Finally the insert from clone IMAGE 166657 containing the 3' ORF and UTR was joined to the above clone at NcoI site; 4042 and the complete cDNA cloned as a NotI fragment into pBluescript (Stratagene) and the expression vector pcDNA3 (Invitrogen) for use in subsequent experiments.

### Functional expression of human AC9

The complete coding sequence of human AC9 was cloned into the expression vector pcDNA3. This constuct and pcDNA3 (10*µ*g) were each linearised by PvuI digestion prior to electroporation into HEK293 cells. Stably transfected clones were selected for resistance to G418 (Gibco) at 0.6*µ* g/ml in Dulbecco's modified Eagle's medium (DMEM) supplemented with 10% foetal calf serum. A total of 12 clones were expanded and over-expression of human AC9 confirmed in 9 cell lines by immunoblotting using antisera raised against a pentadecapeptide derived from the N-terminus of mouse AC9 (Paterson et al, (1995) Biochem. Biophys. Res. Commun. 214:1000-8). All but one of these 9 clones showed the inhibitory effects of Ca²⁺ on cAMP accumulation assayed in the presence of cyclic nucleotide phosphdiesterase (PDE) blockers (Antoni et al, (1998) J. Neurosci. 18:9650-61). Analysis of clone 7 is reported here.

HEK-293 cells display complex intracellular Ca2+ handling mechanisms that may be controlled by cAMP and calcineurin (Lin et al, (1995) Mol. Pharmacol. 47:131-9; Gromada et al, (1995) FEBS Letts. 373:182-6; Querfurth et al, (1998) Biochem J. 334:79-86), and thus complicate the interpretation of the data. Therefore, intracellular stores of Ca²⁺ were depleted to reduce the contribution of intracellular Ca²⁺ pools to the intracellular free Ca²⁺ signal. All procedures were carried out at 37°C, where required, immunosuppressant drugs FK506 (courtesy of Fujisawa GmbH, Munich, Germany) cyclosporin A (courtesy of Novartis Ltd, U.K.) and L685,818 (Courtesy of Merck & Co, Rahway, NJ) rapamycin (courtesy of Dr Hans Fliri, Sandoz Pharma, Basel, Switzerland) or ethanol vehicle (0.1% v/v) were added to the medium at the onset of the Ca²⁺ depletion period. Two different Ca²⁺ depletion protocols were used (Antoni et al, (1998) supra). In the first, cells were incubated in a balanced salt solution containing (mM) 133 NaCl, 5.4 KCl, 0.25 Na₂HPO₄, 0.44 KH₂PO₄, 1 MgSO4, 2 EGTA, 5.6 D-glucose, 25 Hepes pH7.40 and 0.1% (w/v) bovine serum albumin (HBSS/EGTA) with 10*µ*M ryanodine and 1*µ*M thapsigargin (both from LC Labs, Boston, MA). These conditions deplete rapidly exchangeable Ca²⁺ pools and activate capacitative Ca²⁺ entry mechanisms (Cooper et al, (1994) Cellular Signalling 6:823-40). In the second protocol 5*µ*M 4-Br-A23187 was used instead of ryanodine and thapsigargin, and Ca/EGTA was added to obtain similar initial levels of [Ca²⁺]ᵢ as in the studies with ryanodine and thapsigargin. Under these conditions intracellular Ca²⁺ pools are depleted and, in addition to capacitative Ca²⁺ entry, Ca²⁺ influx may take place through the pores formed by the ionophore. The cells were incubated in Ca²⁺-depleting medium for 20min. Subsequently HBSS/EGTA containing various amounts of CaCl₂ (Ca/EGTA) was added to achieve extracellular concentrations of Ca²⁺ ranging between 0.125-2 mM. The pH of this solution was 7.65 in order to minimize the change of pH due to the displacement of protons from EGTA - a pH shift from 7.40 to 7.29 occurred upon achieving a free Ca²⁺ concentration of 2mM. After Ca/EGTA was added, the cells were incubated for 5min after which the cyclic nucleotide PDE inhibitors isobutylmethylxanthine (IBMX, Aldrich, Gillingham, Dorset, U.K.) and rolipram (courtesy of Dr H Wachtel, Schering AG, Berlin Germany), lmM and 0.1 mM respectively, were introduced. Unless indicated otherwise, the cells were incubated for a further 10 min and the incubation was then terminated by the addition of 0.2M HCl. The total cAMP content of the cells and the medium was determined after freeze-thawing and acetylation by radioimmunoassay (Antoni et al, (1995) J. Biol. Chem. 270:28055-61).

### Analysis of human AC9 mRNA expression Northern blotting

A multiple tissue northern blot (MTN; Clontech) of poly A⁺ RNA from human heart, brain, placenta, lung, liver, skeletal muscle, kidney and pancreas (2*µ*g per lane) was probed with a 528bp SmaI-MluI fragment from the 5' end of the human AC9 cDNA and subsequently with a human β-actin cDNA control probe supplied with the blot (each labelled with 32P-dCTP by random priming; Pharmacia "Ready-to-Go" system). Hybridization was carried out in Express Hyb solution (Clontech) according to the manufacturers instructions at 68°C for 1 hour, washed in 2xSSC/0.1 % SDS at room temperature followed by 0.1xSSC/0.1% SDS at 50°C then wrapped in plastic and exposed to Fuji X-ray film for autoradiography. Following removal of previous probes the filter was hybridised as above with a cDNA fragment derived by PCR amplification from a human skeletal muscle cDNA library (Clontech) corresponding to nucleotides 3816-4487 of clone KIAA0520 (GenBank/EMBL Accession Number AB011092) (26).

**Ribonuclease protection assays** were performed using a ³²P-CTP labelled riboprobe synthesised by *in vitro* transcription (T7 RNA polymerase Riboprobe System, Promega) from a *Hin*d*III*-linearised plasmid (pJP195; Figure 1) partial human AC9 cDNA clone representing nucleotides 853-1167 according to the kit manufacturer (Ambion). Probe fragments protected from nuclease digestion following incubation with 5*µ*g total RNA samples from normal adult human brain obtained 4h after death (JEB; Custodian, MRC Brain Bank, Western General Hospital, Edinburgh) or HEK293 cells (control) were resolved on a 6% denaturing acrylamide gel (8M Urea) and visualised by autoradiography.

**In situ hybridisation** was performed on 15*µ*m frozen sections from normal post mortem adult human brain obtained from the MRC Alzheimer Disease Brain Bank, Western General Hospital, Edinburgh - Professor Jeane E Bell, Custodian. Prepared sections were courtesy of Dr Celia Yates and Professor George Fink. ³⁵S-labelled riboprobes were prepared by *in vitro* transcription from the human AC9 partial cDNA clone pJP195 (Figure 18) using T7 (anti-sense) or SP6 (sense) RNA polymerase and used as previously described for mouse AC9 (Paterson et al, (1995) Biochem. Biophys. Res. Commun. 214:1000-8; Rosie et al, (1990) J. Endocrinol. 124:285-9). Following hybridisation, brain sections were exposed to β-max autoradiogaphy film (Amersham-Pharmacia) for 2 weeks. The autoradiogram was developed and photographed under white light illumination.

### Immunocytochemistry

HEK293 cells were plated on poly-D-lysine-coated coverslips and cultured as described above. Two to four days after plating, cells were washed in phosphate buffered saline (PBS) and fixed in 3% paraformaldehyde in PBS, pH 7.4, for 20 min at 4°C, treated with chilled (-20°C) absolute methanol for 10 min (4°C) and washed in 0.3 M glycine (3 times for 5 min) and 0.2 % Triton X-100 (15 min) both in PBS at room temperature. Fixed cells were incubated for 18-24 h at 4°C with affinity purified chick antibodies (1:400-1:500) raised against the C-terminus of mouse AC9 (Antoni et al, (1998) J. Neurosci. 18:9650-61). FITC-conjugated anti-chicken IgG (Jackson ImmunoRes.Lab.) was applied at 1:400 dilution for 1 hr at RT. After several washes in PBS specimens were mounted in CitiFluor (Sigma) and examined using a Leica TCS-NT confocal microscope. Specificity controls involved preincubation of the antibody with 10*µ*M antigen overnight at 4°C, after which immunocytochemistry was carried out as above.

### Detection of human AC9 by immunoblotting

Membrane proteins were prepared from HEK293-derived cell lines as described previously (Antoni et al, (1998) supra) . Approximately 10*µ*g membrane protein per lane were separated by SDS-PAGE and transferred by electroblotting on to PVDF membrane (Bio-Rad) at 220V in tank buffer (25mMTris, 192mM glycine, 0.036% SDS, 20% methanol) for 3hrs at room temperature. PVDF membranes were blocked at room temperature by shaking in PBS containing 5% (w/v) skimmed milk powder and 0.1% Tween 20 for 2hrs at room temperature, then washed 5x 5mins in PBS/0.1% Tween 20 before shaking incubation in PBS/0.1% Tween 20 containing primary antibody (rabbit anti-mouse AC9 N-terminus at 1:4,000 dilution or chicken anti-mouse AC9 C-terminus at 1:10,000 dilution) for lhr at room temperature. PVDF membranes were then washed as before and incubated in PBS/0.1% Tween 20 containing sheep anti-rabbit IgG-Horseradish peroxidase (HRP) conjugate (kindly provided by the Scottish Antibody Production Unit) at 1:8,000 dilution or goat anti-chicken IgY-HRP conjugate (Promega) at 1:10,000 dilution respectively) for 1 hour at room temperature then finally washed as above, exposed to reagents for development of chemiluminescence and visualised by exposure to Hyperfilm (ECL system, Amersham-Pharmacia, Aylesbury, UK). To confirm specificity, primary antisera were pre-incubated with mouse AC9-derived pentadeca-peptide antigens at final concentrations 1*µ*g/ml (C-terminal) and 0.1*µ*g/ml (N-terminal) for 20 & 45 mins respectively prior to probing of the PVDF membranes.

### Results

### Cloning and Sequencing of Human AC9 cDNA

Searching of human EST databases for similarity to mouse AC9 identifed two cDNA clones: IMAGE clone 166657 and HIBBC33, which were obtained as described in materials and methods. Oligonucleotide primers based on the DNA sequence of the inserts of these were used to construct a complete cDNA by PCR amplification from human cDNA libraries as described in materials and methods and outlined in Figure 18. In the cDNA we describe (GenBank/EMBL Accession Number AJ133123) a short open reading frame (ORF) of 72bp exists near the 5' end (263-334). However, the initiation codon of this ORF does not form part of a Kozak consensus sequence suggesting that translation may not be initiated although this remains to be confirmed. Downstream, a large ORF of 4059bp is apparent, from 539-4597. This encodes a putative protein of 1353 amino acids, identical in length to the mouse AC9 protein. The deduced amino acid sequence of hAC9 reported here exhibits 92% sequence identity with the mouse AC9 protein and 86.7% identity at the nucleotide level. Structural features predicted from the amino acid sequence using the computer program TopPred 2 (von Heijne (1992) J. Mol. Biol. 225:487-494) are typical of membrane-associated mammalian adenylyl cyclases cloned to date, namely, two sets of six transmembrane regions (M1 and M2), each followed by a large cytoplasmic region (C1 and C2 respectively). These domains are conserved between the human and mouse AC9 proteins along with many motifs for potential post-translational modifications. With N and C termini localised intracellularly, the predicted topology of membrane-bound AC9 indicates conserved sites which may be suitable for modification include those for N-linked glycosylation, located extracellularly (residues 206, 955, 964), as well as intracellular sites suitable for tyrosine phosphorylation (residues 543, 1004, 1225) and serine/threonine phosphorylation by cAMP-dependent protein kinase (residues 373, 374), calmodulin-dependent protein kinase II (residues 1217), casein kinase II (residues 433, 582, 589, 691, 766, 1200) and protein kinase C (residues 27, 87, 112, 357, 365, 535, 620, 661, 762, 838, 1003, 1023, 1179, 1212, 1307).

### Expression of Human AC9 Protein Detected by Immunocytochemical Analysis

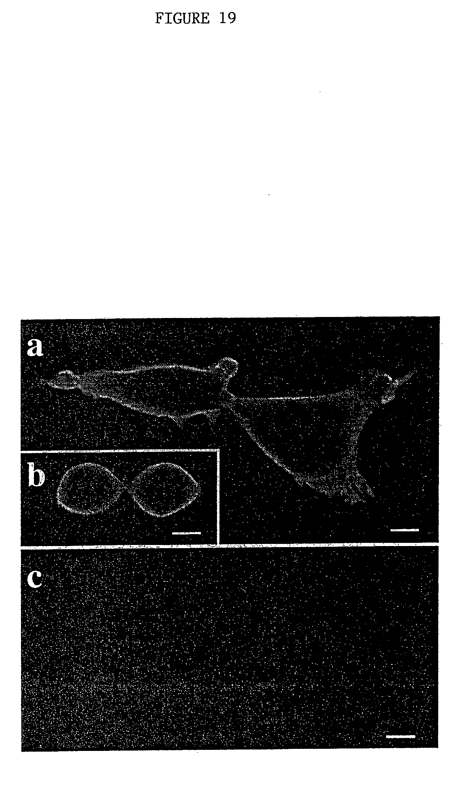

Immunocytochemical analysis using antisera raised against the C-terminus of the mouse AC9 protein revealed fluorescent labelling in HEK 293 cells stably transfected with cDNA encoding human AC9 (Figure 19a & b). No labelling was observed when the antiserum was pre-incubated with peptide antigen (Figure 19c) indicating specific detection of human AC9 protein. A large majority of human AC9 expressed in HEK 293 cells appeared to be localised to the plasma membrane.

### Detection of Multiple Human AC9 Polypeptides by Immunoblotting

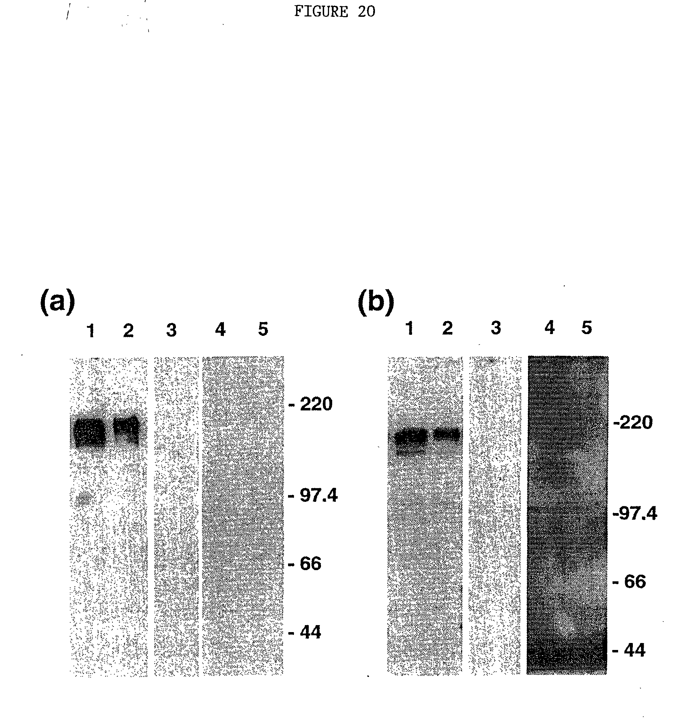

Immunoblotting of extracts from pcDNA3 and hAC9 stably transfected HEK293 cells resolved by SDS-polyacrylamide gel electrophoresis (PAGE) showed that hAC9 protein was expressed and specifically recognised by polyclonal antisera raised against peptides derived from the N- or the C-termini of mouse AC9 (Figure 20). In each case, a broad immunoreactive band migrating between 160-180kDa was detected, most probably attributable to post-translational modification by phosphorylation and/or N-glycosylation of the human AC9 protein in HEK 293. These observations confirm the predicted conservation of the sequences of the human and mouse AC9 at the N- and C-termini.

### Ca²⁺/Calcineurin Inhibition of cAMP Production by Human AC9

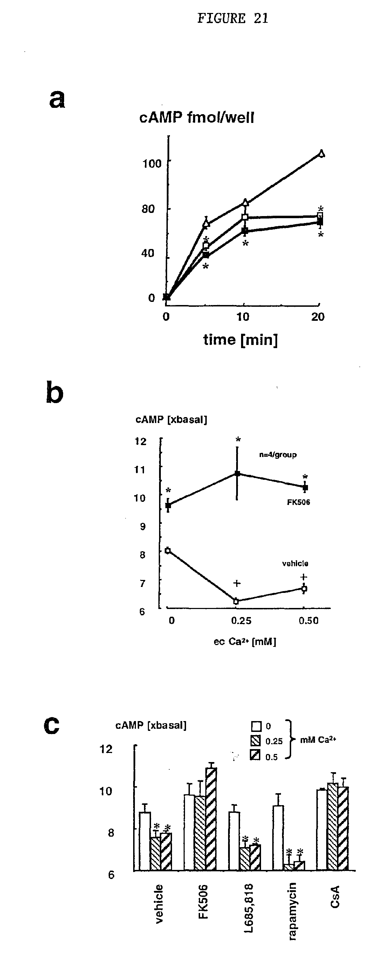

In the presence of phosphodiesterase inhibitors, the cAMP content of stably transfected HEK293 cell lines expressing human AC9 increased with time and this was reduced by the presence of Ca²⁺ in the medium (Figure 21a). No statistically significant increase of cAMP was observed in wild type HEK293 cells or HEK293 cell lines transfected with pcDNA3 vector alone. Similar data were obtained in cells treated in 5*µ*M 4-Br-A23187 containing medium (Figure 21b) moreover, the inhibitory effect of Ca²⁺ was blocked by FK506. L-685,818 and rapamycin, which both interact with FK506-binding-protein-12, but do not inhibit calcineurin (29), failed to block the inhibitory action of Ca²⁺ on cAMP production (Figure 21c). By contrast, cyclosporin A, which blocks calcineurin through a complex formed with cyclophilin A (Schreiber (1992) Cell 70:365-8) also abolished the effect of Ca²⁺. These data indicate that the human AC9 expressed in HEK293 cells is able to synthesize cAMP and its activity is inhibited by Ca²⁺ *via* calcineurin.

### Tissue distribution of two forms of Human AC9 mRNA

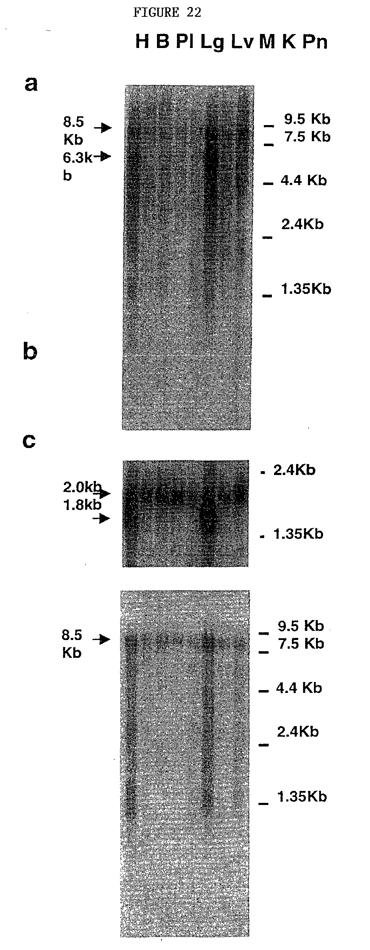

Analysis of hAC9 mRNA by Northern blotting (Figure 22a) using a radio-labelled probe derived from the 5' end of the hAC9 cDNA (SmaI-MluI fragment; 528bp) revealed hybridisation to a transcript of approximately 8.5kb in all tissues represented, including brain, heart, skeletal muscle and pancreas. In addition, an approximately 6.3kb mRNA was detected which appeared to be restricted to heart and skeletal muscle and gave a more intense signal than the 8.5kb species. A human cDNA sequence (GenBank/EMBL Accession Number AB011092), described as part of a study reporting the predicted coding sequences of 100 cDNA clones isolated from human brain (Nagase et al, (1998) DNA Research 5:31-9), was retrieved by database searching and found to be identical to nucleotides 2900-5472 of the human AC9 cDNA sequence reported here but continues with a 3' untranslated region extended by 2234bp. A cDNA fragment derived from this region used in hybridisation with the same MTN Northern blot detected the 8.5kb human AC9 mRNA exclusively (Figure 22c). These data are consistent with tissue-specific differential use of alternative polyadenylation signals on completion of mRNA transcription from the human AC9 gene. Subsequent searching of the GenBank/EMBL DNA sequence database for comparison of human AC9 cDNA 3'UTR sequences with the human AC9 gene locus (GenBank/EMBL Accession Number AC005736) confirmed the positions of alternative polyadenylation sites.

### Expression of AC9 mRNA in human brain

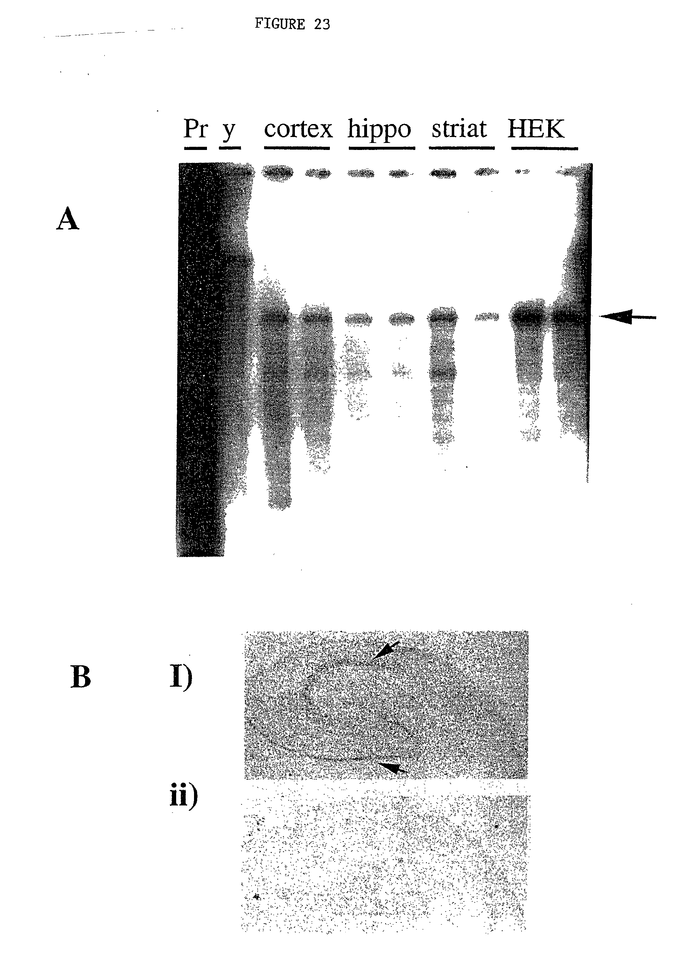

To address the potential role of AC9 within the human brain, expression of AC9 mRNA was investigated by ribonuclease protection assay of total RNA samples isolated from post-mortem hippocampus, cortex and striatum (Figure 23a). Protection of the anti-sense AC9 riboprobe was observed in each of these regions of human brain, indicating expression of AC9 mRNA in areas important for learning and memory functions. AC9 mRNA distribution in human forebrain was also investigated by *in situ* hybridisation (Figure 23b) where enriched expression was detected specifically in neurones of the dentate gyrus and hippocampal CA1-CA3 regions.

### Discussion

We have isolated and sequenced a human cDNA encoding an adenylyl cyclase which, distinct from a recently reported predicted human AC9 polypeptide (Hacker et al, (1998) Genomics 50:97-104), encodes a homologue of murine AC9. Cyclic AMP production by this new human AC9 cDNA clone is inhibited by Ca²⁺ through the protein phosphatase calcineurin. Analysis of the pattern of the expression of the novel AC9 mRNA indicates differential tissue specificity in the polyadenylation of transcripts. This has not been observed in the mouse and may provide an additional level of control in the regulation of AC9 expression in humans.

### Confirmation of the Predicted Primary Sequence of the Human AC9 Protein

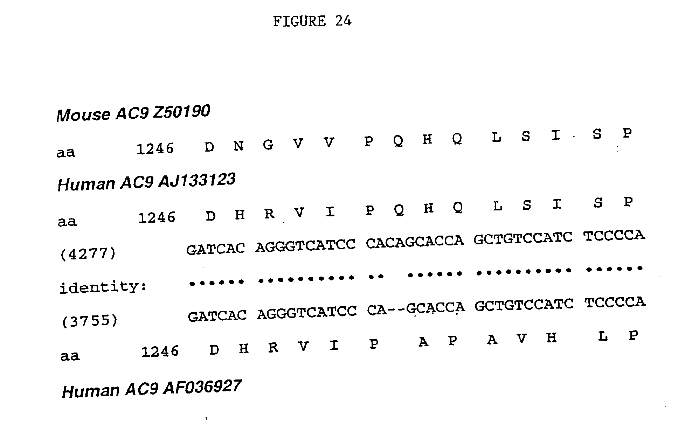

The human AC9 cDNA we have cloned predicts a polypeptide of the same length (1353 amino acid residues) as mouse AC9 and is 92% identical with the mouse AC9 polypeptide sequence. The novel human AC9 could be detected with anti-sera raised against the Nor the C-terminal regions of the mouse AC9 polypeptide sequence, confirming the predicted conservation of these regions between the two proteins. The 3' end of the cDNA reported here is identical to part of a 5'-truncated human AC9 cDNA clone KIAA0520 (Nagase et al, (1998) DNA Research 5:31-9). These sequences differ from that of another human AC9 cDNA clone reported recently by Hacker et al. who predicted an alternative C-terminus for the encoded polypeptide (Hacker et al, (1998) Genomics 50:97-104). By comparison of sequences (Figure 24), the prediction by Hacker et al. results from a 2bp deletion causing a frame shift in the deduced amino acid sequence. Our data, therefore, predict and confirm an amino acid sequence for human AC9 which is distinct from that reported for a previously isolated clone of the human enzyme but which is conserved with mouse AC9.

### Inhibition of human AC9 activity by Ca²⁺/calcineurin

Stably transfected HEK293-derived cell lines which over-express human AC9 showed a marked increase in cAMP production in comparison to wild type or pcDNA3-transfected control cells. The synthesis of cAMP was reduced by intracellular Ca²⁺ concentrations in the range of 100-150nM which is similar to previous data with HEK293 cells overexpressing mouse AC9 (Paterson et al, Biochem. Biophys. Res. Commun. 214:1000-8; Antoni et al, (1998) J. Neurosci. 18:9650-61). The inhibitory effect of Ca²⁺ was blocked by FK506 or cyclosporin A, which are two mechanistically distinct blockers of the Ca²⁺/calmodulin activated protein phosphatase calcineurin (Sabatini et al, (1992) Cell 70:365-8). In the presence of FK506 or cyclosporin A, cAMP production in hAC9 expressing cells was enhanced. Furthermore, application of L685,818 or rapamycin had no effect on cAMP production. The results indicate that the activity of human AC9, like mouse AC9 (Paterson et al, Biochem. Biophys. Res. Commun. 214:1000-8; Antoni et al, (1995) J. Biol. Chem. 270:28055-61; Antoni et al, (1998) J. Neurosci. 18:9650-61), is under inhibitory control by Ca²⁺ which is mediated by calcineurin. The lack of Ca²⁺ inhibition of AC9 observed by others may be due to analysis in insect cells (Premont et al, (1996) J. Biol. Chem. 271:13900-7) which may lack the necessary co-factors or may be attributable to a feature of the cDNA and/or transfected cell clone analysed (Hacker et al, (1998) Genomics 50:97-104). Immunological detection of multiple human AC9 polypeptides may represent differential post-translational modifications in HEK293 cells. Correlation of these observations with multiple phosphorylation or other post-translational modifications of human AC9 protein remains to be addressed. Immunocytochemical analysis shown in Figure 19 suggests that the great majority of human AC9 polypeptides expressed in HEK 293 cells are localised to the plasma membrane.

### Differential Human AC9 mRNA Expression

In similarity with the mouse (Premont et al, (1996) J. Biol. Chem. 271:13900-7) and in agreement with a recent study of another human AC9 cDNA clone (Hacker et al, (1998) Genomics 50:97-104), a broad pattern of expression was observed for an 8.5kb AC9 mRNA in human brain and peripheral tissues when assayed by Northern blotting using a probe derived from the 5' end of the cDNA we have isolated. In the present study, a 6.3kb human AC9 mRNA also detected by this probe appeared to be restricted to human heart and skeletal muscle. Previously, however, a probe representing a 5'-truncated human AC9 cDNA detected a 6.3kb transcript at high levels in skeletal muscle, at low levels in kidney, liver, lung and placenta but which was apparently absent from heart (Hacker et al, (1998) supra). Although the probes used in these two studies overlap by approximately 100bp near the 5' end of the ORF, the hybridisation conditions used may mean that these probes would be capable of detecting differentially spliced human AC9 mRNA transcripts if an intron/exon junction were located within this region. Lack of detection of the 6.3kb mRNA in heart may potentially reflect mRNA splicing of upstream exons, detectable by the 5' probe, to alternative downstream exons. Similarly, it is possible that hybridisation to the 6.3kb transcript by the 5'-truncated 4.65kb partial cDNA probe reflects the presence of alternative 5'-end sequences not detectable by the 5' probe used in this study. However, comparison of our cDNA sequence with human AC9 genomic sequence available in the GenBank/EMBL database (Accession Number AC005736) indicates no evidence of mRNA splicing in this region.
The β-actin control in our Northern blotting analysis suggests the presence of proportionally greater amounts of heart and skeletal muscle mRNAs. Therefore, it is possible that the 6.3kb AC9 mRNA was present in other tissues but not at a level detectable under the conditions we have used and that expression of this transcript is not necessarily restricted to heart and skeletal muscle as our results suggest.
A probe derived from the extended 3' untranslated region of the 8.5kb human AC9 transcript which is present in the cDNA clone KIAA0520 exclusively detected the 8.5kb mRNA in northern blotting. This indicates the use of alternative polyadenylation signals within the human AC9 gene to be the mechanism accounting for the observation of two mRNAs. Polyadenylation of many eukaryotic mRNAs is understood to contribute to inhibition of mRNA degradation and/or potentiate mRNA translation, reviewed in (Wickers et al, (1997) Curr. Opin. Genet. Dev. 7:220-32). In addition, 5' and particularly 3' untranslated regions (UTRs) have been found to be important for the regulation of mRNA translation, stability and localisation, reviewed in (Wickers et al, (1997) supra; Jackson et al, (1997) Curr. Opin. Genet. Dev. 7:233-41), most significantly during development, reviewed in (Curtis et al, (1995) Cell 81:171-8; Seydoux (1996) Curr. Opin. Genet. Dev. 6:555-61; Macdonald et al, (1996) Curr. Opin. Genet. Dev. 6:403-7). The spatial and/or temporal distribution of trans-acting factors interacting with cis elements within 3'UTRs and form a complex with the 5'UTR and associated translation initiation factors has been proposed a molecular mechanism for the control of gene expression by regulation of translation efficiency (Wickers et al, (1997) supra). Although sequences within the 3'UTRs of human and mouse AC9 mRNAs are highly conserved, the 3'UTRs of human AC9 transcripts extend beyond that of the mouse with the 8.5kb mRNA possessing considerable more 3' untranslated sequence than the 6.3kb message. Although the functional significance of 3'UTRs in AC9 mRNAs remains to be addressed but may be subject to differential post-transcriptional regulation providing an additional mechanism of control at the level of mRNA stability and/or translational efficiency facilitating differential regulation of AC9 expression which may be relevant for the appropriate function of the enzyme in humans.

In cardiac myocytes, adenylyl cyclase VI (AC6) expression has been shown to be limiting for transmembrane β-adrenergic signal transduction. Furthermore, all currently known cardiac adenylyl cyclases are under inhibitory control by Ca²⁺. Thus precise control of cAMP levels mediated through regulation of AC expression and control of AC activity appears essential for optimal cardiac function (Gao et al, (1998) PNAS USA 95:1038-43). Alterations to the levels of AC expression in heart, including AC9, may therefore contribute to an impairment of cardiac function related to reduction or loss of cAMP production. A role for post-transcriptional regulation in the control of adenylyl cyclase expression has recently been suggested from studies of ACVIII (AC8) (Muglia et al, (1999) J. Neurosci. 19:2051-8) which have revealed conservation of large 5'UTRs of AC8 mRNAs from mouse, rat and human.

Detection of AC9 mRNA by ribonuclease protection assay revealed expression in hippocampus, neocortex and striatum of post-mortem human brain. *In situ* hybridisation analysis revealed enriched expression of AC9 mRNA in hippocampus and indicated a neuronal localisation in projection neurones of the CA1-CA3 regions. The Ca²⁺/calmodulin activated protein phosphatase calcineurin, which regulates AC9 activity, is also highly abundant in these regions (Winder et al, (1998) Cell 92:25-37). These findings are likely to be relevant to the function of AC9 in brain, particularly in hippocampus, where precise control of Ca²⁺ and cAMP levels is pivotal for synaptic plasticity and suggests potential involvement in functions important for cognitive behaviour (Mons et al, (1995) Trends in Neurosci. 18:536:42; Xia et al, (1997) Curr. Opin. Neurobiol. 7:391-6; Antoni et al, (1998) J. Neurosci. 18:9650-61; Winder et al, (1998) Cell 92:25-27; Mons et al, (1998) Life Sciences 62:1647-52).

Isolation of a human AC9 has enabled our characterisation of the enzyme, allowing comparison with that of the mouse. Differential usage of polyadenylation signals within the gene providing the potential for alternative mRNA post-transcriptional regulation has been identified as a distinct feature of human AC9 likely to be important for precise control of expression. In similarity with mouse AC9, cAMP production by human AC9 is under inhibitory control by Ca²⁺/calcineurin. Post-translational modifications of the human AC9 protein, including possible phosphorylation, now require characterisation and their potential functional consequences investigated. The physiological function of AC9 and its potential involvement in human disease, particularly in view of its broad expression pattern, are complex issues to address. Although important differences have been described, many features are common to the human and murine enzymes. The production of mouse models may, therefore, be useful in the investigation of specific functions of AC9 *in vivo.*

**TABLE I**

| | | | |
|---|---|---|---|
| Effect of pretreatment with FK506 (1µmol/l) and BAPTA/AM (20µmol/l) on cAMP accumulation induced by β-adrenergic stimulation in AtT20 cells. Data are means ±SEM, n=4/group. IBMX 0.5mmol/l and rokpram 0.1mmol/l present throughout. nd - not determined, previous studies showed that there is no effect of either preincubation protocol on basal cAMP levels. | | | |

| | | **Pretreatment** | |
|---|---|---|---|
| Isoproterenol (nmol/l) | None | FK506 | BAPTA/AM |
| None | 0.43±04 | nd | nd |
| 0.4 | 0.7±0.04 | 0.94±0.09 | 0.96±0.06 |
| 2 | 2.0±0.08 | 3.3±0.7 | 3.6±0.6 |
| 10 | 9.5±0.33 | 15.3±2.5 | 11.4±0.9 |
| 100 | 19.5±.5 | 31.4±1.9 | 35.5±3.4 |
| 1000 | 32.1±0.5 | 45.3±9 | 46.9±9 |

**TABLE II**

| | | |
|---|---|---|
| PCR protocols for 5' RACE base cDNA cloning of AC from AtT20 cell total RNA | | |

| **Reaction using primers 2 or 3 with UAP** | | |
|---|---|---|
| 1 cycle | 95°C | 5min |
| | 80° | 5min (Taq polymerase added) |
| | | |
| 5 cycles | 94°C | 45sec |
| | 55°C | 30sec |
| | 72°C | 90sec |
| | | |
| 30 cycles | 94°C | 45sec |
| | 57°C | 30sec |
| | 72°C | 90sec |
| | | |
| 1 cycle | 72°C | 10min |
| | | |

| **Reaction using primer 5 with UAP**: | | |
|---|---|---|
| 1 cycle | 95°C | 5min |
| | 80°C | 5min (Taq polymerase added) |
| | | |
| 35 cycles | 94°C | 45sec |
| | 55°C | 30sec |
| | 72°C | 90sec |
| | | |
| 1 cycle | 72°C | 10min |
| | | |

| **Reaction using primer 6 with UAP** | | |
|---|---|---|
| 1 cycle | 95°C | 5min |
| | 80°C | 5min (Taq polymerase added) |
| | | |
| 35 cycles | 94°C | 60sec |
| | 58°C | 60sec |
| | 72°C | 90sec |
| | | |
| 1 cycle | 72°C | 10min |

**TABLE III**

| **Potential sites of phosphorylation by common S/T protein kinases in AC** | |
|---|---|
| **A KINASE:** | |
| Motif: RXS | Position |
| RAS | 85 |
| RES | 597 |
| RTS | 763 |
| RSS | 919 |
| | |

| Motif: RXXS | |
|---|---|
| RASS | 85 (SEQ ID NO: 15) |
| RVDS | 201 (SEQ ID NO: 16) |
| RSRS | 304 (SEQ ID NO: 17) |
| RESS | 597 (SEQ ID NO: 18) |
| RPAS | 972 (SEQ ID NO: 19) |
| RVLS | 1213 (SEQ ID NO: 20) |
| | |

| **C KINASE** | |
|---|---|
| Motif: (S/T)X(K/R) S preferred with hydrophobic AA on COOH side of S (23/37 of known sites are like this) | |
| SIR | 837 |
| SMR | 1022 |
| SWR | 1310 |
| SVK | 356 |
| SVR | 27 |
| SYR | 761 |
| SYR | 1002 |
| SYR | 1211 |
| TAK | 534 |
| TLR | 187 |
| TVK | 619 |
| | |

| Motif (K/R)XX(S/T) (S preferred with hydrophobic AA) | |
|---|---|
| KIKTI | 1105 (SEQ ID NO: 21) |
| KTATL | 266 (SEQ ID NO: 22) |
| KISTL | 436 (SEQ ID NO: 23) |
| KKSSI | 370 (SEQ ID NO: 24) |
| KEDSL | 732 (SEQ ID NO: 25) |
| KFDSM | 94 (SEQ ID NO: 26) |
| KIQSM | 1019 (SEQ ID NO: 27) |
| RPASL | 972 (SEQ ID NO: 28) |
| | |
| | |

| **CASEIN KINASE II:** | |
|---|---|
| Motif: 3 acidic aa-s after phosphorylation site are a strong indication (*), highly S preferring | |
| | |
| SCAEA | 581 (SEQ ID NO: 29) |
| SGFEV | 588^{*} (SEQ ID NO: 30) |
| SLCEI | 690 (SEQ ID NO: 31) |
| SYQEE | 765 (SEQ ID NO: 32) |
| SEFET | 885^{*} (SEQ ID NO: 33) |
| SWREP | 1310 (SEQ ID NO: 34) |
| TTSET | 261 (SEQ ID NO: 35) |
| TKCEK | 432 (SEQ ID NO: 36) |
| TKCEK | 432 |
| TGVEC | 1199 (SEQ ID NO: 37) |
| | |
| | |

| **CAM KINASE II:** | |
|---|---|
| Motif: XRXX(S/T) Position | |
| | |
| ERASS | 84 (SEQ ID NO: 38) |
| GRVDS | 200 (SEQ ID NO: 39) |
| VRSRS | 303 (SEQ ID NO: 40) |
| SRESS | 596 (SEQ ID NO: 41) |
| RRPAS | 971 (SEQ ID NO: 42) |
| YRVLS | 1212 (SEQ ID NO: 43) |
| KRHAT | 358 (SEQ ID NO: 44) |
| IREKT | 719 (SEQ ID NO: 45) |
| SRMDT | 1194 (SEQ ID NO: 46) |
| GRSPT | 1270 (SEQ ID NO: 47) |

**TABLE IV**

| | | |
|---|---|---|
| Inhibition by 1µmol/l FK506 of the effect of extracellular calcium ions on 10nmol/1 CRF-induced cAMP formation in calcium-depleted AtT20 cells in the presence of 0.5 mmol/1 IBMX (see Legend to Fig 1 and Example 1 for methods). Data are means ± SEM, pmol/well, n=6/group. 2-way ANOVA gave a significant (P<0.05) interaction between extracellular calcium and FK506. Unstimulated cAMP levels were 0.5±0.06pmol/l. | | |

| **Extracellular free [Ca**^{**2+**}**] Vehicle** | **FK506** | |
|---|---|---|
| **[mmol/l]** | | |
| 0 | 12.1±0.16 | 10.3±0.10 |
| 0.5 | 10.4±0.96 | 10.0±0.11 |
| 1.0 | 9.7±0.10 | 10.4±0.10 |
| 1.5 | 6.2±0.04 | 11.3±0.13 |
| 2.0 | 5.2±0.54 | 9.5±0.11 |

## Claims

1. A protein or polypeptide having adenylate cyclase activity, said activity being regulatable by calcineurin.

2. A protein or polypeptide as claimed in Claim 1 which is encoded by a polynucleotide comprising a nucleotide sequence derived from the sequence set out in SEQ ID NO: 1 or in SEQ ID NO: 98, or a part thereof.

3. A protein or polypeptide as claimed in Claim 1 comprising the amino acid sequence as set out in SEQ ID NO: 2 or in SEQ ID NO: 99, a functional equivalent or part thereof.

4. A protein or polypeptide as claimed in Claim 1 which includes amino acid Nos 503-570 of SEQ ID NO: 2 or which includes amino acid Nos 503-570 of SEQ ID NO: 99.

5. An isolated polynucleotide comprising a nucleotide sequence as set out in SEQ ID NO: 98, or a part thereof.

6. A recombinant polynucleotide construct comprising a polynucleotide as claimed in Claim 5.

7. A vector comprising a polynucleotide as claimed in Claim 5.

8. A host cell transformed with a vector as claimed in Claim 7.

9. A host cell as claimed in Claim 8 which is derived from the Human Embryonic Kidney Cell line 293.

10. A method of influencing cellular metabolism by using the protein or polypeptide as claimed in Claim 1 to affect the adenylate cyclase activity or the amount of said protein or polypeptide.

11. The method as claimed in Claim 10 wherein said regulator is calcineurin, its activators, inhibitors and competitors; antibodies to amino acids 503-570; or β-adrenergic agonists and β-adrenergic antagonists.

12. The method as claimed in Claim 10 wherein said regulator is an antibody to amino acids 503-570 of SEQ ID NOS: 2 or 99.

13. A method of treating a human or non-human animal body, said method comprising administrating an agent to affect the adenylate cyclase activity of, or the amount of a protein or polypeptide as claimed in Claim 1, said protein or polypeptide being present within said body.

14. A method as claimed in Claim 13 wherein said protein or polypeptide is naturally present within said body.

15. A method as claimed in Claim 13 to affect or combat neurological-based disorders; psychiatrically-based disorders; endocrine-based disorders; cardiovascular-based disorders; pregnancy-based disorders; respiratory-based disorders; bone-based disorders; kidney-based disorders; gut-based disorders; or tumours.

16. A method of designing or testing for a therapeutic agent which influences cellular metabolism comprising administering the protein or polypeptide as claimed in Claim 1 and detecting its effect on cellular metabolism.


SEQUENCE LISTING

<110> Medical Research Council

<120> Adenylate cyclase and uses therefor

<130> P24360-

<140>
<141>

<160> 104

<170> PatentIn Ver. 2.1

<210> 1
<211> 4473
<212> DNA
<213> Mouse

<220>
<221> CDS
<222> (43)..(4101)
<223> PRODUCT = "Adenylate cyclase coding region"

<220>
<223> ANTI-SENSE : NO

<220>
<223> CELL TYPE :  Corticotroph tumour cell line

<220>
<223> CELL LINE : AtT20

<220>
<223> STRANDEDNESS : Single

<220>
<223> TOPOLOGY : Linear

<220>
<223> MOLECULE TYPE : cDNA

<220>
<223> HYPOTHETICAL : NO

<400> 1
tgcccctgac ctggtcggga aaggttccaa gagctcggca ac atg gct tcc tca     54

Met Ala Ser Ser
1

ccc cac cag cag ctg ctg cat cac cat agc acc gag gtg agc tgc gac 102
Pro His Gln Gln Leu Leu His His His Ser Thr Glu Val Ser Cys Asp
5                   10                  15                  20

tca agc gga gac agc aac agc gtg agg gtc aag atc aac cct aag cag 150
Ser Ser Gly Asp Ser Asn Ser Val Arg Val Lys Ile Asn Pro Lys Gln
                25                  30                  35

ctg tcc tcc aac acc cac ccg aag cac tgc aag tac agc atc tcc tcc 198
Leu Ser Ser Asn Thr His Pro Lys His Cys Lys Tyr Ser Ile Ser Ser
            40                  45                  50

agc tgt agc agc tcg gga gac tca ggg ggc ctt ccc cgg agg gtt ggc 246
Ser Cys Ser Ser Ser Gly Asp Ser Gly Gly Leu Pro Arg Arg Val Gly
        55                  60                  65

ggc ggg ggt cgc ctg cgc aga cag aag aag ctg ccc cag ctt ttt gag 294
Gly Gly Gly Arg Leu Arg Arg Gln Lys Lys Leu Pro Gln Leu Phe Glu
    70                  75                  80

agg gcc tcc agc cgg tgg tgg gac ccc aaa ttc gac tcc atg aac ctg 342
Arg Ala Ser Ser Arg Trp Trp Asp Pro Lys Phe Asp Ser Met Asn Leu
85                  90                  95                  100

gag gag gcc tgc ctg gag cgc tgc ttt ccg cag acc cag cgc cgc ttc 390
Glu Glu Ala Cys Leu Glu Arg Cys Phe Pro Gln Thr Gln Arg Arg Phe
                105                 110                 115

cgg tac gca ctc ttt tat gtg ggc ttc gcc tgc ctt ctc tgg agc atc 438
Arg Tyr Ala Leu Phe Tyr Val Gly Phe Ala Cys Leu Leu Trp Ser Ile
            120                 125                 130

tat ttc gct gtc cac atg aaa tcc aaa gtg att gtc atg gtg gtc cca 486
Tyr Phe Ala Val His Met Lys Ser Lys Val Ile Val Met Val Val Pro
        135                 140                 145

gct ctg tgc ttc ctg gtg gtg tgt gtg ggc ttt ttc ctg ttt act ttc 534
Ala Leu Cys Phe Leu Val Val Cys Val Gly Phe Phe Leu Phe Thr Phe
    150                 155                 160

acc aag ctg tac gcc cgg cat tat gcg tgg acc tcg ctg gct ctc acc 582
Thr Lys Leu Tyr Ala Arg His Tyr Ala Trp Thr Ser Leu Ala Leu Thr
165                 170                 175                 180

ctg ctg gtg ttc gcc ctg acc ctg gct gcg cag ttt cag gtt tgg aca 630

Leu Leu Val Phe Ala Leu Thr Leu Ala Ala Gln Phe Gln Val Trp Thr
                185                 190                 195

cct ctg tca gga cgt gtt gac agc tcc aat cat act ctc acg gcc act 678
Pro Leu Ser Gly Arg Val Asp Ser Ser Asn His Thr Leu Thr Ala Thr
            200                 205                 210

ccg gcg gac act tgc tta tct caa gta gga agc ttc tcc ata tgc atc 726
Pro Ala Asp Thr Cys Leu Ser Gln Val Gly Ser Phe Ser Ile Cys Ile
        215                 220                 225

gaa gtg ctc ctt ttg ctc tac aca gtc atg cag tta cct ctg tac ctg 774
Glu Val Leu Leu Leu Leu Tyr Thr Val Met Gln Leu Pro Leu Tyr Leu
    230                 235                 240

agc ttg ttt ttg ggg gtg gtc tat tct gtc ctt ttt gag acc ttc ggc 822
Ser Leu Phe Leu Gly Val Val Tyr Ser Val Leu Phe Glu Thr Phe Gly
245                 250                 255                 260

tac cac ttc cga aac gaa gac tgc tac cct tct ccg ggc cct ggg gcc 870
Tyr His Phe Arg Asn Glu Asp Cys Tyr Pro Ser Pro Gly Pro Gly Ala
                265                 270                 275

ctg cac tgg gag ctg ctg agc aga gcc ctg ctt cac gtg tgc att cac 918
Leu His Trp Glu Leu Leu Ser Arg Ala Leu Leu His Val Cys Ile His
            280                 285                 290

gct atc ggg atc cat ctg ttt gtc atg tct cag gtg agg tcc agg agc 966
Ala Ile Gly Ile His Leu Phe Val Met Ser Gln Val Arg Ser Arg Ser
        295                 300                 305

acc ttt ctc aag gtg gga caa tcc att atg cac ggc aaa gat ctg gaa 1014
Thr Phe Leu Lys Val Gly Gln Ser Ile Met His Gly Lys Asp Leu Glu
    310                 315                 320

gta gag aaa gcc ctg aaa gag agg atg att cat tca gtg atg cca aga 1062
Val Glu Lys Ala Leu Lys Glu Arg Met Ile His Ser Val Met Pro Arg
325                 330                 335                 340

atc ata gcc gac gac tta atg aaa caa ggg gac gag gag agt gag aat 1110
Ile Ile Ala Asp Asp Leu Met Lys Gln Gly Asp Glu Glu Ser Glu Asn
                345                 350                 355

tct gtc aag agg cat gcc acc tcc agt ccc aag aac agg aag aag aag 1158
Ser Val Lys Arg His Ala Thr Ser Ser Pro Lys Asn Arg Lys Lys Lys
            360                 365                 370

tcc tcc ata cag aag gca ccg ata gca ttc cgc ccc ttt aag atg cag 1206

Ser Ser Ile Gln Lys Ala Pro Ile Ala Phe Arg Pro Phe Lys Met Gln
        375                 380                 385

cag att gaa gaa gtc agt att tta ttt gca gac att gtg ggt ttc acc    125[?]
Gln Ile Glu Glu Val Ser Ile Leu Phe Ala Asp Ile Val Gly Phe Thr
    390                 395                 400

aag atg agc gcc aac aaa tct gcg cat gcc ttg gta ggc cta ctc aat    130[?]
Lys Met Ser Ala Asn Lys Ser Ala His Ala Leu Val Gly Leu Leu Asn
405                 410                 415                 420

gac ctg ttc ggt cgc ttt gac cgc ctg tgt gag cag acc aag tgt gag    135[?]
Asp Leu Phe Gly Arg Phe Asp Arg Leu Cys Glu Gln Thr Lys Cys Glu
                425                 430                 435

aag atc agc act ctg ggg gac tgt tat tac tgt gtg gca ggg tgt ccg    1398
Lys Ile Ser Thr Leu Gly Asp Cys Tyr Tyr Cys Val Ala Gly Cys Pro
            440                 445                 450

gag ccc cgg gca gac cat gcc tac tgc tgc att gaa atg ggc tta ggc    1446
Glu Pro Arg Ala Asp His Ala Tyr Cys Cys Ile Glu Met Gly Leu Gly
        455                 460                 465

atg ata aaa gcc atc gag cag ttc tgc cag gag aag aaa gag atg gtg    1494
Met Ile Lys Ala Ile Glu Gln Phe Cys Gln Glu Lys Lys Glu Met Val
    470                 475                 480

aac atg cgt gtt ggg gtt cac acg ggg act gtc ctg tgt ggc atc ctg    1542
Asn Met Arg Val Gly Val His Thr Gly Thr Val Leu Cys Gly Ile Leu
485                 490                 495                 500

ggc atg agg agg ttt aaa ttt gat gtg tgg tcc aac gat gtg aac ttg    1590
Gly Met Arg Arg Phe Lys Phe Asp Val Trp Ser Asn Asp Val Asn Leu
                505                 510                 515

gct aat ctc atg gag cag ctg gga gtg gct ggc aaa gtt cac ata tct    1638
Ala Asn Leu Met Glu Gln Leu Gly Val Ala Gly Lys Val His Ile Ser
            520                 525                 530

gag gcc act gca aaa tac tta gac gac agg tat gaa atg gaa gat ggg    1686
Glu Ala Thr Ala Lys Tyr Leu Asp Asp Arg Tyr Glu Met Glu Asp Gly
        535                 540                 545

aga gtt att gag cgc ctt ggg cag agt gtg gtg gct gac cag ttg aaa    1734
Arg Val Ile Glu Arg Leu Gly Gln Ser Val Val Ala Asp Gln Leu Lys
    550                 555                 560

ggt ttg aag aca tac ctg ata tcg ggt cag aga gcc aag gag tcc cac    1782

Gly Leu Lys Thr Tyr Leu Ile Ser Gly Gln Arg Ala Lys Glu Ser His
565                 570                 575                 580

tgc agc tgt gca gag gcc ctg ctt tct ggc ttt gag gtc att gac gac    1830
Cys Ser Cys Ala Glu Ala Leu Leu Ser Gly Phe Glu Val Ile Asp Asp
                585                 590                 595

tca cgg gag tcc tca ggc cct agg gga cag ggg aca gca tcg cca ggg    1878
Ser Arg Glu Ser Ser Gly Pro Arg Gly Gln Gly Thr Ala Ser Pro Gly
            600                 605                 610

agt gtc agt gat ttg gcg cag act gtc aaa acc ttt gat aac ctt aag    1926
Ser Val Ser Asp Leu Ala Gln Thr Val Lys Thr Phe Asp Asn Leu Lys
        615                 620                 625

act tgc cct tct tgt gga atc aca ttt gct ccc aaa tct gaa gct ggt    1974
Thr Cys Pro Ser Cys Gly Ile Thr Phe Ala Pro Lys Ser Glu Ala Gly
    630                 635                 640

gca gaa gga gga act gtg caa aat ggc tgt caa gac gag cct aag acc    2022
Ala Glu Gly Gly Thr Val Gln Asn Gly Cys Gln Asp Glu Pro Lys Thr
645                 650                 655                 660

agc acc aag gct tct gga gga ccc aac tcc aaa acc cag aat gga ctt    2070
Ser Thr Lys Ala Ser Gly Gly Pro Asn Ser Lys Thr Gln Asn Gly Leu
                665                 670                 675

ctg agc cct cct gca gag gag aag ctc act aac agc cag acc tcc ctc    2118
Leu Ser Pro Pro Ala Glu Glu Lys Leu Thr Asn Ser Gln Thr Ser Leu
            680                 685                 690

tgt gag atc ctg caa gag aag gga cgg tgg gca ggc gtg agc ttg gac    2166
Cys Glu Ile Leu Gln Glu Lys Gly Arg Trp Ala Gly Val Ser Leu Asp
        695                 700                 705

cag tca gcc ctc ctc ccg ctc agg ttc aag aac atc cgt gag aaa act    2214
Gln Ser Ala Leu Leu Pro Leu Arg Phe Lys Asn Ile Arg Glu Lys Thr
    710                 715                 720

gat gcc cac ttt gtt gat gtc atc aaa gaa gac agc ctg atg aaa gat    2262
Asp Ala His Phe Val Asp Val Ile Lys Glu Asp Ser Leu Met Lys Asp
725                 730                 735                 740

tat ttc ttc aag ccg ccc atc aat cag ttc agc ctg aac ttc ctg gac    2310
Tyr Phe Phe Lys Pro Pro Ile Asn Gln Phe Ser Leu Asn Phe Leu Asp
                745                 750                 755

cag gag ctg gag cga tca tat aga acc agc tac cag gaa gag gtc ata    2358

Gln Glu Leu Glu Arg Ser Tyr Arg Thr Ser Tyr Gln Glu Glu Val Ile
            760                 765                 770

aag aat tct cct gtg aag acg ttc gcc agt gcc acc ttc agc tcc ctt 2406
Lys Asn Ser Pro Val Lys Thr Phe Ala Ser Ala Thr Phe Ser Ser Leu
        775                 780                 785

ctg gat gtg ttt ctg tca acc acc gtg ttc ctg att ctc tcc atc acc 2454
Leu Asp Val Phe Leu Ser Thr Thr Val Phe Leu Ile Leu Ser Ile Thr
    790                 795                 800

tgc ttc cta aag tat gga gcc acc gcc acc cct ccc cca ccg gct gcc 2502
Cys Phe Leu Lys Tyr Gly Ala Thr Ala Thr Pro Pro Pro Pro Ala Ala
805                 810                 815                 820

ctg gcc gtc ttt ggt gca gac ctg ctg ctg gag gtg ctt tcc ctc ata 2550
Leu Ala Val Phe Gly Ala Asp Leu Leu Leu Glu Val Leu Ser Leu Ile
                825                 830                 835

gtg tcc atc aga atg gtg ttt ttc cta gag gat gtc atg aca tgc aca 2598
Val Ser Ile Arg Met Val Phe Phe Leu Glu Asp Val Met Thr Cys Thr
            840                 845                 850

aag tgg ttg ctg gaa tgg atc gct ggc tgg ctc cct cgc cac tgc att 2646
Lys Trp Leu Leu Glu Trp Ile Ala Gly Trp Leu Pro Arg His Cys Ile
        855                 860                 865

ggg gca atc ttg gtg tct ctt cct gcc ctg gct gtc tat tca cac atc 2694
Gly Ala Ile Leu Val Ser Leu Pro Ala Leu Ala Val Tyr Ser His Ile
    870                 875                 880

acc tct gag ttt gag acc aac ata cat gtc acc atg ttc act ggc tct 2742
Thr Ser Glu Phe Glu Thr Asn Ile His Val Thr Met Phe Thr Gly Ser
885                 890                 895                 900

gcg gtg ctg gtg gcc gtt gtg cac tac tgt aac ttc tgc cag ctc agc 2790
Ala Val Leu Val Ala Val Val His Tyr Cys Asn Phe Cys Gln Leu Ser
                905                 910                 915

tcc tgg atg agg tcc tcc ctt gcc acc atc gtg ggg gct ggg ctg ctg 2838
Ser Trp Met Arg Ser Ser Leu Ala Thr Ile Val Gly Ala Gly Leu Leu
            920                 925                 930

ctt ctg ctc cac atc tcc ctg tgt cag gac agt tcc att gtg atg tcc 2886
Leu Leu Leu His Ile Ser Leu Cys Gln Asp Ser Ser Ile Val Met Ser
        935                 940                 945

ccc ttg gac tca gca cag aat ttc agt gcc cag agg aac cca tgc aac 2934

Pro Leu Asp Ser Ala Gln Asn Phe Ser Ala Gln Arg Asn Pro Cys Asn
    950                 955                 960

agc tca gtg ctg cag gac ggc agg agg ccg gcc agc ctc ata ggc aag 2982
Ser Ser Val Leu Gln Asp Gly Arg Arg Pro Ala Ser Leu Ile Gly Lys
965                 970                 975                 980

gag ctt atc ctc acc ttc ttc ctc ctg ctc ctc ttg gtc tgg ttc ctg 3030
Glu Leu Ile Leu Thr Phe Phe Leu Leu Leu Leu Leu Val Trp Phe Leu
                985                 990                 995

aac cgg gag ttc gag gtc agc tac cgg ctg cac tac cat ggg gat gtg 3078
Asn Arg Glu Phe Glu Val Ser Tyr Arg Leu His Tyr His Gly Asp Val
            1000                1005                1010

gag gcc gac cta cac cgc acc aag atc cag agc atg aga gac cag gct 3126
Glu Ala Asp Leu His Arg Thr Lys Ile Gln Ser Met Arg Asp Gln Ala
        1015                1020                1025

gac tgg cta ctg cgg aac atc atc ccc tac cat gtg gct gag cag ctc 3174
Asp Trp Leu Leu Arg Asn Ile Ile Pro Tyr His Val Ala Glu Gln Leu
    1030                1035                1040

aag gtc tct cag acc tac tcc aag aac cat gac agc ggg gga gtc atc 3222
Lys Val Ser Gln Thr Tyr Ser Lys Asn His Asp Ser Gly Gly Val Ile
1045                1050                1055                1060

ttt gcc agc att gtc aac ttc agt gaa ttc tat gag gag aac tat gag 3270
Phe Ala Ser Ile Val Asn Phe Ser Glu Phe Tyr Glu Glu Asn Tyr Glu
                1065                1070                1075

ggg ggc aag gag tgc tac cgt gtc ctc aac gag ctg atc ggt gac ttc 3318
Gly Gly Lys Glu Cys Tyr Arg Val Leu Asn Glu Leu Ile Gly Asp Phe
            1080                1085                1090

gat gag ctc ttg agc aag ccg gac tat aat agc atc gag aag atc aag 3366
Asp Glu Leu Leu Ser Lys Pro Asp Tyr Asn Ser Ile Glu Lys Ile Lys
        1095                1100                1105

acc atc ggg gcc aca tac atg gca gcc tca ggg ctg aac acg gcc cag 3414
Thr Ile Gly Ala Thr Tyr Met Ala Ala Ser Gly Leu Asn Thr Ala Gln
    1110                1115                1120

tgt cag gag ggt ggc cac cca cag gag cat ctg cgt atc ctc ttc gag 3462
Cys Gln Glu Gly Gly His Pro Gln Glu His Leu Arg Ile Leu Phe Glu
1125                1130                1135                1140

ttc gcc aag gag atg atg cgc gtg gtg gat gac ttc aac aac aat atg 3510

Phe Ala Lys Glu Met Met Arg Val Val Asp Asp Phe Asn Asn Asn Met
                1145                1150                1155

tta tgg ttc aac ttc aag ctc agg gtc ggc ttt aac cac gga ccc ctc 3558
Leu Trp Phe Asn Phe Lys Leu Arg Val Gly Phe Asn His Gly Pro Leu
            1160                1165                1170

aca gca ggt gtc ata ggt acc acc aag ctg ctg tat gac atc tgg ggg 3606
Thr Ala Gly Val Ile Gly Thr Thr Lys Leu Leu Tyr Asp Ile Trp Gly
        1175                1180                1185

gac acc gtc aac atc gcc agc agg atg gac acc act ggt gtg gag tgc 3654
Asp Thr Val Asn Ile Ala Ser Arg Met Asp Thr Thr Gly Val Glu Cys
    1190                1195                1200

cgt atc cag gtg agc gaa gag agc tac cgt gtg ctg agc aag atg ggt 3702
Arg Ile Gln Val Ser Glu Glu Ser Tyr Arg Val Leu Ser Lys Met Gly
1205                1210                1215                1220

tat gac ttt gac tac cga ggg acc gtg aat gtc aag ggg aaa ggg cag 3750
Tyr Asp Phe Asp Tyr Arg Gly Thr Val Asn Val Lys Gly Lys Gly Gln
                1225                1230                1235

atg aag acc tac ctt tac cca aag tgc acg gac aat gga gtg gtt ccc 3798
Met Lys Thr Tyr Leu Tyr Pro Lys Cys Thr Asp Asn Gly Val Val Pro
            1240                1245                1250

cag cac cag ctg tcc atc tcc cca gac atc cga gtc cag gtg gac ggc 3846
Gln His Gln Leu Ser Ile Ser Pro Asp Ile Arg Val Gln Val Asp Gly
        1255                1260                1265

agc att ggg cgg tct ccc aca gat gag att gcc aac ttg gtg cct tcc 3894
Ser Ile Gly Arg Ser Pro Thr Asp Glu Ile Ala Asn Leu Val Pro Ser
    1270                1275                1280

gtt cag tat tcg gac aag gct tcc ctg gga tct gat gat agc aca cag 3942
Val Gln Tyr Ser Asp Lys Ala Ser Leu Gly Ser Asp Asp Ser Thr Gln
1285                1290                1295                1300

gct aag gaa gct cac ctg tcc tct aag agg tcc tgg aga gag cca gtc 3990
Ala Lys Glu Ala His Leu Ser Ser Lys Arg Ser Trp Arg Glu Pro Val
                1305                1310                1315

aaa gca gag gaa agg ttt cca ttt ggc aaa gcc ata gaa aag gac agc 4038
Lys Ala Glu Glu Arg Phe Pro Phe Gly Lys Ala Ile Glu Lys Asp Ser
            1320                1325                1330

tgt gaa gac ata gga gta gaa gag gcc agt gaa ctc agc aag ctc aat 4086

Cys Glu Asp Ile Gly Val Glu Glu Ala Ser Glu Leu Ser Lys Leu Asn
        1335                1340                1345

gtc tca aag agt gtg tgaggcagcg ccgagagctg ccaaggtgct ctgcgtgtcc    4141
Val Ser Lys Ser Val
    1350

aaacacagta acatctgtgt cgataggctg ttgtgctatc tagcacctca gtttctgtcc 4201

ccagatgtgg tgtcacgtgg tcatttcagc ccgaatctct gtgtggagca cagttattca 4261

gggttcattt ccacccattt cggttttcct ttacttgcgt tcctggaagc cttttcctgg 4321

aagcctgccc ccagcccagc caggggatcc agtcagcagc gtggagggat tcaagtgcct 4381

tcagggtctg gccttgcgtc tggggctgag gccactggtg gaatcatggc cctggggatt 4441

atttgacttc tttaagtttt tttttttttt tt                                4473

<210> 2
<211> 1353
<212> PRT
<213> Mouse
<223> HYPOTHETICAL : NO

<400> 2
Met Ala Ser Ser Pro His Gln Gln Leu Leu His His His Ser Thr Glu
  1               5                  10                  15

Val Ser Cys Asp Ser Ser Gly Asp Ser Asn Ser Val Arg Val Lys Ile
             20                  25                  30

Asn Pro Lys Gln Leu Ser Ser Asn Thr His Pro Lys His Cys Lys Tyr
         35                  40                  45

Ser Ile Ser Ser Ser Cys Ser Ser Ser Gly Asp Ser Gly Gly Leu Pro
     50                  55                  60

Arg Arg Val Gly Gly Gly Gly Arg Leu Arg Arg Gln Lys Lys Leu Pro
 65                  70                  75                  80

Gln Leu Phe Glu Arg Ala Ser Ser Arg Trp Trp Asp Pro Lys Phe Asp
                 85                  90                  95

Ser Met Asn Leu Glu Glu Ala Cys Leu Glu Arg Cys Phe Pro Gln Thr
            100                 105                 110

Gln Arg Arg Phe Arg Tyr Ala Leu Phe Tyr Val Gly Phe Ala Cys Leu
        115                 120                 125

Leu Trp Ser Ile Tyr Phe Ala Val His Met Lys Ser Lys Val Ile Val
    130                 135                 140

Met Val Val Pro Ala Leu Cys Phe Leu Val Val Cys Val Gly Phe Phe
145                 150                 155                 160

Leu Phe Thr Phe Thr Lys Leu Tyr Ala Arg His Tyr Ala Trp Thr Ser
                165                 170                 175

Leu Ala Leu Thr Leu Leu Val Phe Ala Leu Thr Leu Ala Ala Gln Phe
            180                 185                 190

Gln Val Trp Thr Pro Leu Ser Gly Arg Val Asp Ser Ser Asn His Thr
        195                 200                 205

Leu Thr Ala Thr Pro Ala Asp Thr Cys Leu Ser Gln Val Gly Ser Phe
    210                 215                 220

Ser Ile Cys Ile Glu Val Leu Leu Leu Leu Tyr Thr Val Met Gln Leu
225                 230                 235                 240

Pro Leu Tyr Leu Ser Leu Phe Leu Gly Val Val Tyr Ser Val Leu Phe
                245                 250                 255

Glu Thr Phe Gly Tyr His Phe Arg Asn Glu Asp Cys Tyr Pro Ser Pro
            260                 265                 270

Gly Pro Gly Ala Leu His Trp Glu Leu Leu Ser Arg Ala Leu Leu His
        275                 280                 285

Val Cys Ile His Ala Ile Gly Ile His Leu Phe Val Met Ser Gln Val
    290                 295                 300

Arg Ser Arg Ser Thr Phe Leu Lys Val Gly Gln Ser Ile Met His Gly
305                 310                 315                 320

Lys Asp Leu Glu Val Glu Lys Ala Leu Lys Glu Arg Met Ile His Ser
                325                 330                 335

Val Met Pro Arg Ile Ile Ala Asp Asp Leu Met Lys Gln Gly Asp Glu
            340                 345                 350

Glu Ser Glu Asn Ser Val Lys Arg His Ala Thr Ser Ser Pro Lys Asn
        355                 360                 365

Arg Lys Lys Lys Ser Ser Ile Gln Lys Ala Pro Ile Ala Phe Arg Pro
    370                 375                 380

Phe Lys Met Gln Gln Ile Glu Glu Val Ser Ile Leu Phe Ala Asp Ile
385                 390                 395                 400

Val Gly Phe Thr Lys Met Ser Ala Asn Lys Ser Ala His Ala Leu Val
                405                 410                 415

Gly Leu Leu Asn Asp Leu Phe Gly Arg Phe Asp Arg Leu Cys Glu Gln
            420                 425                 430

Thr Lys Cys Glu Lys Ile Ser Thr Leu Gly Asp Cys Tyr Tyr Cys Val
        435                 440                 445

Ala Gly Cys Pro Glu Pro Arg Ala Asp His Ala Tyr Cys Cys Ile Glu
    450                 455                 460

Met Gly Leu Gly Met Ile Lys Ala Ile Glu Gln Phe Cys Gln Glu Lys
465                 470                 475                 480

Lys Glu Met Val Asn Met Arg Val Gly Val His Thr Gly Thr Val Leu
                485                 490                 495

Cys Gly Ile Leu Gly Met Arg Arg Phe Lys Phe Asp Val Trp Ser Asn
            500                 505                 510

Asp Val Asn Leu Ala Asn Leu Met Glu Gln Leu Gly Val Ala Gly Lys
        515                 520                 525

Val His Ile Ser Glu Ala Thr Ala Lys Tyr Leu Asp Asp Arg Tyr Glu
    530                 535                 540

Met Glu Asp Gly Arg Val Ile Glu Arg Leu Gly Gln Ser Val Val Ala
545                 550                 555                 560

Asp Gln Leu Lys Gly Leu Lys Thr Tyr Leu Ile Ser Gly Gln Arg Ala
                565                 570                 575

Lys Glu Ser His Cys Ser Cys Ala Glu Ala Leu Leu Ser Gly Phe Glu
            580                 585                 590

Val Ile Asp Asp Ser Arg Glu Ser Ser Gly Pro Arg Gly Gln Gly Thr
        595                 600                 605

Ala Ser Pro Gly Ser Val Ser Asp Leu Ala Gln Thr Val Lys Thr Phe
    610                 615                 620

Asp Asn Leu Lys Thr Cys Pro Ser Cys Gly Ile Thr Phe Ala Pro Lys
625                 630                 635                 640

Ser Glu Ala Gly Ala Glu Gly Gly Thr Val Gln Asn Gly Cys Gln Asp
                645                 650                 655

Glu Pro Lys Thr Ser Thr Lys Ala Ser Gly Gly Pro Asn Ser Lys Thr
            660                 665                 670

Gln Asn Gly Leu Leu Ser Pro Pro Ala Glu Glu Lys Leu Thr Asn Ser
        675                 680                 685

Gln Thr Ser Leu Cys Glu Ile Leu Gln Glu Lys Gly Arg Trp Ala Gly
    690                 695                 700

Val Ser Leu Asp Gln Ser Ala Leu Leu Pro Leu Arg Phe Lys Asn Ile
705                 710                 715                 720

Arg Glu Lys Thr Asp Ala His Phe Val Asp Val Ile Lys Glu Asp Ser
                725                 730                 735

Leu Met Lys Asp Tyr Phe Phe Lys Pro Pro Ile Asn Gln Phe Ser Leu
            740                 745                 750

Asn Phe Leu Asp Gln Glu Leu Glu Arg Ser Tyr Arg Thr Ser Tyr Gln
        755                 760                 765

Glu Glu Val Ile Lys Asn Ser Pro Val Lys Thr Phe Ala Ser Ala Thr
    770                 775                 780

Phe Ser Ser Leu Leu Asp Val Phe Leu Ser Thr Thr Val Phe Leu Ile
785                 790                 795                 800

Leu Ser Ile Thr Cys Phe Leu Lys Tyr Gly Ala Thr Ala Thr Pro Pro
                805                 810                 815

Pro Pro Ala Ala Leu Ala Val Phe Gly Ala Asp Leu Leu Leu Glu Val
            820                 825                 830

Leu Ser Leu Ile Val Ser Ile Arg Met Val Phe Phe Leu Glu Asp Val
        835                 840                 845

Met Thr Cys Thr Lys Trp Leu Leu Glu Trp Ile Ala Gly Trp Leu Pro
    850                 855                 860

Arg His Cys Ile Gly Ala Ile Leu Val Ser Leu Pro Ala Leu Ala Val
865                 870                 875                 880

Tyr Ser His Ile Thr Ser Glu Phe Glu Thr Asn Ile His Val Thr Met
                885                 890                 895

Phe Thr Gly Ser Ala Val Leu Val Ala Val Val His Tyr Cys Asn Phe
            900                 905                 910

Cys Gln Leu Ser Ser Trp Met Arg Ser Ser Leu Ala Thr Ile Val Gly
        915                 920                 925

Ala Gly Leu Leu Leu Leu Leu His Ile Ser Leu Cys Gln Asp Ser Ser
    930                 935                 940

Ile Val Met Ser Pro Leu Asp Ser Ala Gln Asn Phe Ser Ala Gln Arg
945                 950                 955                 960

Asn Pro Cys Asn Ser Ser Val Leu Gln Asp Gly Arg Arg Pro Ala Ser
                965                 970                 975

Leu Ile Gly Lys Glu Leu Ile Leu Thr Phe Phe Leu Leu Leu Leu Leu
            980                 985                 990

Val Trp Phe Leu Asn Arg Glu Phe Glu Val Ser Tyr Arg Leu His Tyr
        995                 1000                1005

His Gly Asp Val Glu Ala Asp Leu His Arg Thr Lys Ile Gln Ser Met
    1010                1015                1020

Arg Asp Gln Ala Asp Trp Leu Leu Arg Asn Ile Ile Pro Tyr His Val
025 1030 1035 1040

Ala Glu Gln Leu Lys Val Ser Gln Thr Tyr Ser Lys Asn His Asp Ser
                1045                1050                1055

Gly Gly Val Ile Phe Ala Ser Ile Val Asn Phe Ser Glu Phe Tyr Glu
            1060                1065                1070

Glu Asn Tyr Glu Gly Gly Lys Glu Cys Tyr Arg Val Leu Asn Glu Leu
        1075                1080                1085

Ile Gly Asp Phe Asp Glu Leu Leu Ser Lys Pro Asp Tyr Asn Ser Ile
    1090                1095                1100

Glu Lys Ile Lys Thr Ile Gly Ala Thr Tyr Met Ala Ala Ser Gly Leu
105 1110 1115 1120

Asn Thr Ala Gln Cys Gln Glu Gly Gly His Pro Gln Glu His Leu Arg
                1125                1130                1135

Ile Leu Phe Glu Phe Ala Lys Glu Met Met Arg Val Val Asp Asp Phe
            1140                1145                1150

Asn Asn Asn Met Leu Trp Phe Asn Phe Lys Leu Arg Val Gly Phe Asn
        1155                1160                1165

His Gly Pro Leu Thr Ala Gly Val Ile Gly Thr Thr Lys Leu Leu Tyr
    1170                1175                1180

Asp Ile Trp Gly Asp Thr Val Asn Ile Ala Ser Arg Met Asp Thr Thr
185                 1190                1195                1200

Gly Val Glu Cys Arg Ile Gln Val Ser Glu Glu Ser Tyr Arg Val Leu
                1205                1210                1215

Ser Lys Met Gly Tyr Asp Phe Asp Tyr Arg Gly Thr Val Asn Val Lys
            1220                1225                1230

Gly Lys Gly Gln Met Lys Thr Tyr Leu Tyr Pro Lys Cys Thr Asp Asn
        1235                1240                1245

Gly Val Val Pro Gln His Gln Leu Ser Ile Ser Pro Asp Ile Arg Val
    1250                1255                1260

Gln Val Asp Gly Ser Ile Gly Arg Ser Pro Thr Asp Glu Ile Ala Asn
265                 1270                1275                1280

Leu Val Pro Ser Val Gln Tyr Ser Asp Lys Ala Ser Leu Gly Ser Asp
                1285                1290                1295

Asp Ser Thr Gln Ala Lys Glu Ala His Leu Ser Ser Lys Arg Ser Trp
            1300                1305                1310

Arg Glu Pro Val Lys Ala Glu Glu Arg Phe Pro Phe Gly Lys Ala Ile
        1315                1320                1325

Glu Lys Asp Ser Cys Glu Asp Ile Gly Val Glu Glu Ala Ser Glu Leu
    1330                1335                1340

Ser Lys Leu Asn Val Ser Lys Ser Val
345                 1350

<210> 3
<211> 10
<212> PRT
<213> Unknown Organism

<220>
<223> TOPOLOGY : Linear

<220>
<223> MOLECULE TYPE : Peptide

<220>
<223> CELL TYPE : Skeletal muscle

<220>
<223> Description of Unknown Organism:

<400> 3
Leu Arg Gln Ser Arg Leu Ser Ser Ser Lys
1               5                   10

<210> 4
<211> 11
<212> PRT
<213> mouse

<220>
<223> TOPOLOGY : Linear

<220>
<223> MOLECULE  TYPE : Peptide

<220>
<223> CELL LINE : AtT20

<400> 4
Ile Asp Asp Ser Arg Glu Ser Ser Gly Pro Arg
1               5                   10

<210> 5
<211> 26
<212> DNA
<213> Artificial Sequence

<220>
<223> HYPOTHETICAL : NO

<220>

<223> ANTI-SENSE : NO

<220>
<223> CELL LINE : mammalian adenylate cyclases

<220>
<223> TOPOLOGY : Linear

<220>
<221> misc_feature
<222> (12)
<223> Modified base; mod_base= i

<220>
<223> STRANDEDNESS : Single

<220>
<223> Description of Artificial Sequence:  PCR Primer
      corresponding to highly conserved first
      cytoplasmic domain of mammalian adenyl cyclases

<400> 5
ctcatcgatg gagaytgyta ytaytg                                          26


<210> 6
<211> 26
<212> DNA
<213> Artificial Sequence

<220>
<223> STRANDEDNESS : Single

<220>
<223> TOPOLOGY : Linear

<220>
<223> MOLECULE TYPE : cDNA

<220>
<223> HYPOTHETICAL : NO

<220>
<223> ANTI-SENSE : NO

<220>
<223> CELL LINE : Mammalian adenylate cyclases

<220>
<221> misc_feature
<222> (12)
<223> modified base; mod_base = i

<220>
<223> Description of Artificial Sequence:PCR Primer
      corresponding to highly conserved first
      cytoplasmic domain of mammalian adenyl cyclases

<400> 6
ggctcgagcc aaacrtcrta ytgcca                                          26

<210> 7
<211> 39
<212> DNA
<213> Artificial Sequence

<220>
<223> STRANDEDNESS : Single

<220>
<223> TOPOLOGY : Linear

<220>
<223> MOLECULE TYPE : cDNA

<220>
<223> HYPOTHETICAL : NO

<220>
<223> ANTI-SENSE : NO

<220>
<223> CELL LINE : Mammalian adenylate cyclases

<220>
<221> misc_feature
<222> (13)
<223> modified base; mod_base= i

<220>
<221> misc_feature
<222> (19)
<223> modified base; mod_base= i

<220>

<221> misc_feature
<222> (22)
<223> Modified base; mod_base= i

<220>
<221> misc_feature
<222> (25)
<223> Modified base; mod_base =i

<220>
<221> misc_feature
<222> (28)
<223> Modified base; Mod_base= i

<220>
<221> misc_feature
<222> (31)
<223> Modified base; mod_base= i

<220>
<223> Description of Artificial Sequence: PCR Primer
      corresponsing to highly conserved second
      cytoplasmic domain of mammalian adenyl cyclases

<400> 7
gaagcttaar ataaaracaa taggawsaac atayatggc                    39


<210> 8
<211> 36
<212> DNA
<213> Artificial Sequence

<220>
<223> STRANDEDNESS : Single

<220>
<223> TOPOLOGY : Linear

<220>
<223> MOLECULE  TYPE : cDNA

<220>
<223> HYPOTHETICAL : NO

<220>
<223> ANTI-SENSE : NO

<220>
<223> CELL LINE : Mammalian adenylate cyclases

<220>
<221> misc_feature
<222> (13)
<223> Modified base; mod_base= i

<220>
<221> misc_feature
<222> (16)
<223> Modified base; mod_base= i

<220>
<221> misc_feature
<222> (22)
<223> Modified base; mod_base= i

<220>
<221> misc_feature
<222> (28)
<223> Modified base; Mod_base= i

<220>
<223> Description of Artificial Sequence: PCR Primer
      corresponsing to highly conserved second
      cytoplasmic domain of mammalian adenyl cyclases

<400> 8
gggatccacr ttaacagtrt taccccaaat rtcrta                                36


<210> 9
<211> 19
<212> DNA
<213> Artificial Sequence

<220>
<223> STRANDEDNESS : Single

<220>
<223> TOPOLOGY : Linear

<220>
<223> MOLECULE TYPE : cDNA

<220>
<223> HYPOTHETICAL : NO

<220>
<223> ANTI-SENSE : YES

<220>
<223> CELL LINE : AtT20

<220>
<223> Description of Artificial Sequence:PCR Primer to
      cDNA  clone jp134 of AtT20

<400> 9
cgtcaatgac ctcaaagcc                                                    19


<210> 10
<211> 27
<212> DNA
<213> Artificial Sequence

<220>
<223> STRANDEDNESS : Single

<220>
<223> TOPOLOGY : Linear

<220>
<223> MOLECULE TYPE : cDNA

<220>
<223> HYPOTHETICAL : NO

<220>
<223> ANTI-SENSE : YES

<220>
<223> CELL LINE : AtT20

<220>
<223> Description of Artificial Sequence:PCR Primer to
      cDNA  clone jp134 of AtT20

<400> 10
gcctctgcac agctgcagtg ggactcc                                           27


<210> 11
<211> 33

<212> DNA
<213> Artificial Sequence

<220>
<223> STRANDEDNESS : Single

<220>
<223> TOPOLOGY : Linear

<220>
<223> MOLECULE TYPE : cDNA

<220>
<223> HYPOTHETICAL : NO

<220>
<223> ANTI-SENSE : YES

<220>
<223> CELL LINE : AtT20

<220>
<223> Description of Artificial Sequence:PCR Primer to
      cDNA  clone jp134 of AtT20

<400> 11
cctggcagaa ctgctcgatg gcttttatca tgc                                    33


<210> 12
<211> 18
<212> DNA
<213> Artificial Sequence

<220>
<223> STRANDEDNESS : Single

<220>
<223> TOPOLOGY : Linear

<220>
<223> MOLECULE TYPE : cDNA

<220>
<223> HYPOTHETICAL : NO

<220>
<223> ANTI-SENSE : YES

<220>
<223> CELL LINE : AtT20

<220>
<223> Description of Artificial Sequence: PCR Primer to
      1kb extension of cDNA clone jp134 of AtT20

<400> 12
ggagaagctt cctacttg                                                    18


<210> 13
<211> 29
<212> DNA
<213> Artificial Sequence

<220>
<223> STRANDEDNESS : Single

<220>
<223> TOPOLOGY : Linear

<220>
<223> MOLECULE  TYPE :  cDNA

<220>
<223> HYPOTHETICAL : NO

<220>
<223> ANTI-SENSE : YES

<220>
<223> CELL LINE : AtT20

<220>
<223> Description of Artificial Sequence: PCR Primer to
      1kb extension of cDNA clone jp134 of AtT20

<400> 13
gtggccgtga gagtatgatt ggagctgtc                                        29


<210> 14
<211> 26
<212> DNA
<213> Artificial Sequence

<220>
<223> STRANDEDNESS : Single

<220>
<223> TOPOLOGY : Linear

<220>
<223> MOLECULE TYPE: cDNA

<220>
<223> HYPOTHETICAL : NO

<220>
<223> ANTI-SENSE : YES

<220>
<223> CELL LINE : AtT20

<220>
<223> Description of Artificial Sequence: PCR Primer to
      1kb extension of cDNA clone jp134 of AtT20

<400> 14
gtccaaacct gaaactgcgc acgcag                                          26


<210> 15
<211> 4
<212> PRT
<213> Mouse

<220>
<223> STRANDEDNESS : Single

<220>
<223> TOPOLOGY : Linear

<220>
<223> MOLECULE  TYPE : Peptide

<220>
<223> HYPOTHETICAL : NO

<220>
<223> ANTI-SENSE : NO

<400> 15
Arg Ala Ser Ser

1

<210> 16
<211> 4
<212> PRT
<213> Mouse

<220>
<223> STRANDEDNESS : Single

<220>
<223> TOPOLOGY : Linear

<220>
<223> MOLECULE  TYPE : Peptide

<220>
<223> HYPOTHETICAL : NO

<220>
<223> ANTI-SENSE : NO

<400> 16
Arg Val Asp Ser
1

<210> 17
<211> 4
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>

<223> ANTI-SENSE  :  NO

<400> 17
Arg Ser Arg Ser
  1

<210> 18
<211> 4
<212> PRT
<213> Mouse

<220>
<223> STRANDEDNESS: Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 18
Arg Glu Ser Ser
  1

<210> 19
<211> 4
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 19
Arg Pro Ala Ser
  1

<210> 20
<211> 4
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 20
Arg Val Leu Ser
  1

<210> 21
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 21
Lys Ile Lys Thr Ile
 1               5

<210> 22
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 22
Lys Thr Ala Thr Leu
 1               5

<210> 23
<211> 5
<212> PRT
<213> Mouse

<220>

<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 23
Lys Ile Ser Thr Leu
 1               5

<210> 24
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 24
Lys Lys Ser Ser Ile
 1               5

<210> 25
<211> 5

<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 25
Lys Glu Asp Ser Leu
1               5

<210> 26
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 26
Lys Phe Asp Ser Met
1               5

<210> 27
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 27
Lys Ile Gln Ser Met
1               5

<210> 28
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 28
Arg Pro Ala Ser Leu
1               5

<210> 29
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 29
Ser Cys Ala Glu Ala
1               5

<210> 30
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 30
Ser Gly Phe Glu Val
 1               5

<210> 31
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 31
Ser Leu Cys Glu Ile
 1               5

<210> 32
<211> 5
<212> PRT
<213> Mouse

<220>
<223> STRANDEDNESS : Single

<220>
<223> TOPOLOGY : Linear

<220>

<223> MOLECULE TYPE : Peptide

<220>
<223> HYPOTHETICAL : NO

<220>
<223> ANTI-SENSE : NO

<400> 32
Ser Tyr Gln Glu Glu
1               5

<210> 33
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 33
Ser Glu Phe Glu Thr
1               5

<210> 34
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 34
Ser Trp Arg Glu Pro
1               5

<210> 35
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 35
Thr Thr Ser Glu Thr
1               5

<210> 36
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 36
Thr Lys Cys Glu Lys
1               5

<210> 37
<211> 5
<212> PRT
<213> Mouse

<220>
<223> STRANDENESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 37
Thr Gly Val Glu Cys
1               5

<223> ANTI-SENSE : NO

<400> 41
Ser Arg Glu Ser Ser
1               5

<210> 42
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 42
Arg Arg Pro Ala Ser
1               5

<210> 43
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 43
Tyr Arg Val Leu Ser
1               5

<210> 44
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 44
Lys Arg His Ala Thr
1               5

<210> 45
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 45
Ile Arg Glu Lys Thr
1               5

<210> 46
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 46
Ser Arg Met Asp Thr
1               5

<210> 47
<211> 5
<212> PRT
<213> Mouse

<220>

<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 47
Gly Arg Ser Pro Thr
1               5

<210> 48
<211> 90
<212> PRT
<213> Human

<400> 48
Lys Ile Lys Thr Ile Gly Ser Thr Tyr Met Ala Ala Ala Gly Leu Ser
1               5                   10                  15

Val Ala Ser Gly His Glu Asn Gln Glu Leu Glu Arg Gln His Ala His
            20                  25                  30

Ile Gly Val Met Val Glu Phe Ser Ile Ala Leu Met Ser Lys Leu Asp
        35                  40                  45

Gly Ile Asn Arg His Ser Phe Asn Ser Phe Arg Leu Arg Val Gly Ile
    50                  55                  60

Asn His Gly Pro Val Ile Ala Gly Val Ile Gly Ala Arg Lys Pro Gln
65                  70                  75                  80

Tyr Asp Ile Trp Gly Asn Thr Val Asn Val
                85                  90

<210> 49
<211> 90

<212> PRT
<213> Mouse

<400> 49
Lys Ile Lys Thr Ile Gly Ser Thr Tyr Met Ala Ala Ala Gly Leu Ser
1               5                   10                  15

Ala Pro Ser Gly His Glu Asn Gln Asp Leu Glu Arg Lys His Val His
            20                  25                  30

Ile Gly Val Leu Val Glu Phe Ser Met Ala Leu Met Ser Lys Leu Asp
        35                  40                  45

Gly Ile Asn Arg His Ser Phe Asn Ser Phe Arg Leu Arg Val Gly Ile
    50                  55                  60

Asn His Gly Pro Val Ile Ala Gly Val Ile Gly Ala Arg Lys Pro Gln
65                  70                  75                  80

Tyr Asp Ile Trp Gly Asn Thr Val Asn Val
                85                  90

<210> 50
<211> 91
<212> PRT
<213> Rat

<400> 50
Lys Ile Lys Thr Ile Gly Ser Thr Tyr Met Ala Ala Thr Gly Leu Ser
1               5                   10                  15

Ala Ile Pro Ser Gln Glu His Ala Gln Glu Pro Glu Arg Gln Tyr Met
            20                  25                  30

His Ile Gly Thr Met Val Glu Phe Ala Tyr Ala Leu Val Gly Lys Leu
        35                  40                  45

Asp Ala Ile Asn Lys His Ser Phe Asn Asp Phe Lys Leu Arg Val Gly
    50                  55                  60

Ile Asn His Gly Pro Val Ile Ala Gly Val Ile Gly Ala Gln Lys Pro
65                  70                  75                  80

Gln Tyr Asp Ile Trp Gly Asn Thr Val Asn Val
                85                  90

<210> 51
<211> 91
<212> PRT
<213> Rat

<400> 51
Lys Ile Lys Thr Ile Gly Ser Thr Tyr Met Ala Ala Thr Gly Leu Asn
1               5                   10                  15

Ala Thr Pro Gly Gln Asp Thr Gln Gln Asp Ala Glu Arg Ser Cys Ser
            20                  25                  30

His Leu Gly Thr Met Val Glu Phe Ala Val Ala Leu Gly Ser Lys Leu
        35                  40                  45

Gly Val Ile Asn Lys His Ser Phe Asn Asn Phe Arg Leu Arg Val Gly
    50                  55                  60

Leu Asn His Gly Pro Val Val Ala Gly Val Ile Gly Ala Gln Lys Pro
65                  70                  75                  80

Gln Tyr Asp Ile Trp Gly Asn Thr Val Asn Val
                85                  90

<210> 52
<211> 86
<212> PRT
<213> Human

<400> 52
Lys Ile Lys Thr Ile Gly Ser Thr Tyr Met Ala Val Ser Gly Leu Ser
1               5                   10                  15

Pro Glu Lys Gln Gln Cys Glu Asp Lys Trp Gly His Leu Cys Ala Leu
            20                  25                  30

Ala Asp Phe Ser Leu Ala Leu Thr Glu Ser Ile Gln Glu Ile Asn Lys
        35                  40                  45

His Ser Phe Asn Asn Phe Glu Leu Arg Ile Gly Ile Ser His Gly Ser
    50                  55                  60

Val Val Ala Gly Val Ile Gly Ala Lys Lys Pro Gln Tyr Asp Ile Trp
65                  70                  75                  80

Asp Tyr Ala Met Arg Leu Met Glu Gln Met Lys His Ile Asn Glu His
35 40 45

Ser Phe Asn Asn Phe Gln Met Lys Ile Gly Leu Asn Met Gly Pro Val
50 55 60

Val Ala Gly Val Ile Gly Ala Arg Lys Pro Gln Tyr Asp Ile Trp Gly
65 70 75 80

Asn Thr Val Asn Val
85

<210> 57
<211> 85
<212> PRT
<213> Rat

<400> 57
Lys Ile Lys Thr Ile Gly Ser Thr Tyr Met Ala Ala Ser Gly Leu Asn
1               5                   10                  15

Ala Ser Thr Tyr Asp Gln Val Gly Arg Ser His Ile Thr Ala Leu Ala
            20                  25                  30

Asp Tyr Ala Met Arg Leu Met Glu Gln Met Lys His Ile Asn Glu His
        35                  40                  45

Ser Phe Asn Asn Phe Gln Met Lys Ile Gly Leu Asn Met Gly Pro Val
    50                  55                  60

Val Ala Gly Val Ile Gly Ala Arg Lys Pro Gln Tyr Asp Ile Trp Gly
65                  70                  75                  80

Asn Thr Val Asn Val
                85

<210> 58
<211> 85
<212> PRT
<213> Dog

<400> 58
Lys Ile Lys Thr Ile Gly Ser Thr Tyr Met Ala Ala Ser Gly Leu Asn
1               5                   10                  15

Ala Ser Thr Tyr Asp Gln Ala Gly Arg Ser His Ile Thr Ala Leu Ala
            20                  25                  30

Asp Tyr Ala Met Arg Leu Met Glu Gln Met Lys His Ile Asn Glu His
        35                  40                  45

Ser Phe Asn Asn Phe Gln Met Lys Ile Gly Leu Asn Met Gly Pro Val
    50                  55                  60

Val Ala Gly Val Ile Gly Ala Arg Lys Pro Gln Tyr Asp Ile Trp Gly
65                  70                  75                  80

Asn Thr Val Asn Val
                85

<210> 59
<211> 85
<212> PRT
<213> Rat

<400> 59
Lys Ile Lys Thr Ile Gly Ser Thr Tyr Met Ala Ala Ser Gly Leu Asn
1               5                   10                  15

Asp Ser Thr Tyr Asp Lys Ala Gly Lys Thr His Ile Lys Ala Leu Ala
            20                  25                  30

Asp Phe Ala Met Lys Leu Met Asp Gln Met Lys Tyr Ile Asn Glu His
        35                  40                  45

Ser Phe Asn Asn Phe Gln Met Lys Ile Gly Leu Asn Ile Gly Pro Val
    50                  55                  60

Val Ala Gly Val Ile Gly Ala Arg Lys Pro Gln Tyr Asp Ile Trp Gly
65                  70                  75                  80

Asn Thr Val Asn Val
                85

<210> 60
<211> 85
<212> PRT
<213> Rabbit

<400> 60
Lys Ile Lys Thr Ile Gly Ser Thr Tyr Met Ala Ala Ser Gly Leu Asn
1               5                   10                  15

Asp Ser Thr Tyr Asp Lys Val Gly Lys Thr His Ile Lys Ala Leu Ala
            20                  25                  30

Asp Phe Ala Met Lys Leu Met Asp Gln Met Lys Tyr Ile Asn Glu His
        35                  40                  45

Ser Phe Asn Asn Phe Gln Met Lys Ile Gly Leu Asn Ile Gly Pro Val
    50                  55                  60

Val Ala Gly Val Ile Gly Ala Arg Lys Pro Gln Tyr Asp Ile Trp Gly
65                  70                  75                  80

Asn Thr Val Asn Val
                85

<210> 61
<211> 85
<212> PRT
<213> Dog

<400> 61
Lys Ile Lys Thr Ile Gly Ser Thr Tyr Met Ala Ala Ser Gly Leu Asn
1               5                   10                  15

Asp Ser Thr Tyr Asp Lys Val Gly Lys Thr His Ile Lys Ala Leu Ala
            20                  25                  30

Asp Phe Ala Met Lys Leu Met Asp Gln Met Lys Tyr Ile Asn Glu His
        35                  40                  45

Ser Phe Asn Asn Phe Gln Met Lys Ile Gly Leu Asn Ile Gly Pro Val
    50                  55                  60

Val Ala Gly Val Ile Gly Ala Arg Lys Pro Gln Tyr Asp Ile Trp Gly
65                  70                  75                  80

Asn Thr Val Asn Val
                85

Ala Lys Tyr Leu Asp Asp Arg Tyr Glu Met Glu Asp Gly Arg Val Ile
35 40 45

Glu Arg Leu Gly Gln Ser Val Val Ala Asp Gln Leu Lys Gly Leu Lys
50 55 60

Thr Tyr Leu Ile Ser Gly Gln Arg Ala Lys Glu Ser His Cys Ser Cys
65 70 75 80

Ala Glu Ala Leu Leu Ser Gly Phe Glu Val Ile Asp Asp Ser Arg Glu
85 90 95

Ser Ser Gly Pro Arg Gly Gln Gly Thr Ala Ser Pro
100 105

<210> 66
<211> 107
<212> PRT
<213> Saccharomyces cerevisiae

<400> 66
Asn Val Lys Ile Asp Arg Ile Ser Pro Gly Asp Gly Ala Thr Phe Pro
1               5                   10                  15

Lys Thr Gly Asp Leu Val Thr Ile His Tyr Thr Gly Thr Leu Glu Asn
            20                  25                  30

Gly Gln Lys Phe Asp Ser Ser Val Asp Arg Gly Ser Pro Phe Gln Cys
        35                  40                  45

Asn Ile Gly Val Gly Gln Val Ile Lys Gly Trp Asp Val Gly Ile Pro
    50                  55                  60

Lys Leu Ser Val Gly Glu Lys Ala Arg Leu Thr Ile Pro Gly Pro Tyr
65                  70                  75                  80

Ala Tyr Gly Pro Arg Gly Phe Pro Gly Leu Ile Pro Pro Asn Ser Thr
                85                  90                  95

Leu Val Phe Asp Val Glu Leu Leu Lys Val Asn
            100                 105

<210> 67

<211> 107
<212> PRT
<213> Human

<400> 67
Gly Val Gln Val Glu Thr Ile Ser Pro Gly Asp Gly Arg Thr Phe Pro
1               5                   10                  15

Lys Arg Gly Gln Thr Cys Val Val His Tyr Thr Gly Met Leu Glu Asp
            20                  25                  30

Gly Lys Lys Phe Asp Ser Ser Arg Asp Arg Asn Lys Pro Phe Lys Phe
        35                  40                  45

Met Leu Gly Lys Gln Glu Val Ile Arg Gly Trp Glu Glu Gly Val Ala
    50                  55                  60

Gln Met Ser Val Gly Gln Arg Ala Lys Leu Thr Ile Ser Pro Asp Tyr
65                  70                  75                  80

Ala Tyr Gly Ala Thr Gly His Pro Gly Ile Ile Pro Pro His Ala Thr
                85                  90                  95

Leu Val Phe Asp Val Glu Leu Leu Lys Leu Glu
            100                 105

<210> 68
<211> 107
<212> PRT
<213> Mouse

<400> 68
Gly Val Gln Val Glu Thr Ile Ser Pro Gly Asp Gly Arg Thr Phe Pro
1               5                   10                  15

Lys Arg Gly Gln Thr Cys Val Val His Tyr Thr Gly Met Leu Glu Asp
            20                  25                  30

Gly Lys Lys Phe Asp Ser Ser Arg Asp Arg Asn Lys Pro Phe Lys Phe
        35                  40                  45

Thr Leu Gly Lys Gln Glu Val Ile Arg Gly Trp Glu Glu Gly Val Ala
    50                  55                  60

Gln Met Ser Val Gly Gln Arg Ala Lys Leu Ile Ile Ser Ser Asp Tyr
65                  70                  75                  80

Met Glu Ala Gly Gly Val Pro Gly Arg Val His Ile Ser Ser Val Thr
                20                  25                  30

Leu Glu His Leu Asn Gly Ala Tyr Lys Val Glu Glu Gly Asp Gly Glu
            35                  40                  45

Ile Arg Asp Pro Tyr Leu Lys Gln His Leu Val Lys Thr Tyr Phe Val
    50                  55                  60

Ile Asn Pro Lys Gly Glu Arg Arg Ser Pro Gln His Leu Phe Arg Pro
65                  70                  75                  80

Arg His Thr Leu Asp Gly Ala Lys Met Arg Ala Ser Val Arg Met Thr
                85                  90                  95

Arg Tyr Leu Ser Trp Gly
            100

<210> 76
<211> 103
<212> PRT
<213> Rat

<400> 76
Lys Asn Gln Tyr Asp Val Asn Ser His Asp Val Thr Leu Ala Asn His
 1               5                  10                  15

Met Glu Ala Gly Gly Val Pro Gly Arg Val His Ile Thr Gly Ala Thr
                20                  25                  30

Leu Ala Leu Leu Ala Gly Ala Tyr Ala Val Glu Arg Ala Asp Met Glu
            35                  40                  45

His Arg Asp Pro Tyr Leu Arg Glu Leu Gly Glu Pro Thr Tyr Leu Val
    50                  55                  60

Ile Asp Pro Trp Ala Glu Glu Glu Asp Glu Lys Gly Thr Glu Arg Gly
65                  70                  75                  80

Leu Leu Ser Ser Leu Glu Gly His Thr Met Arg Pro Ser Leu Leu Met
                85                  90                  95

Thr Arg Tyr Leu Ser Trp Gly
            100

<210> 77
<211> 103
<212> PRT
<213> Mouse

<400> 77
Lys Trp Gln Tyr Asp Val Trp Ser His Asp Val Ser Leu Ala Asn Arg
1               5                   10                  15

Met Glu Ala Ala Gly Val Pro Gly Arg Val His Ile Thr Glu Ala Thr
            20                  25                  30

Leu Asn His Leu Asp Lys Ala Tyr Glu Val Glu Asp Gly His Gly Glu
        35                  40                  45

Gln Arg Asp Pro Tyr Leu Lys Glu Met Asn Ile Arg Thr Tyr Leu Val
    50                  55                  60

Ile Asp Pro Arg Ser Gln Gln Pro Pro Pro Pro Ser His His Leu Ser
65                  70                  75                  80

Lys Pro Lys Gly Asp Ala Thr Leu Lys Met Arg Ala Ser Val Arg Val
                85                  90                  95

Thr Arg Tyr Leu Ser Trp Gly
            100

<210> 78
<211> 103
<212> PRT
<213> Human

<400> 78
   Lys Trp Gln Tyr Asp Val Trp Ser His Asp Val Ser Leu Ala Asn Arg
   1               5                   10                  15
   Met Glu Ala Ala Gly Val Pro Gly Arg Val His Ile Thr Glu Ala Thr
               20                  25                  30
   Leu Lys His Leu Asp Lys Ala Tyr Glu Val Glu Asp Gly His Gly Gln
           35                  40                  45
   Gln Arg Asp Pro Tyr Leu Lys Glu Met Asn Ile Arg Thr Tyr Leu Val
       50                  55                  60
   Ile Asp Pro Arg Ser Gln Gln Pro Pro Pro Pro Ser Gln His Leu Pro
   65                  70                  75                  80
   Arg Pro Lys Gly Asp Ala Ala Leu Lys Met Arg Ala Ser Val Arg Met
                   85                  90                  95
   Thr Arg Tyr Leu Ser Trp Gly
               100

<210> 79
<211> 107
<212> PRT
<213> Yeast

<400> 79
Asn Val Lys Ile Asp Arg Ile Ser Pro Gly Asp Gly Ala Thr Phe Pro
1               5                   10                  15

Lys Thr Gly Asp Leu Val Thr Ile His Tyr Thr Gly Thr Leu Glu Asn
            20                  25                  30

Gly Gln Lys Phe Asp Ser Ser Val Asp Arg Gly Ser Pro Phe Gln Cys
        35                  40                  45

Asn Ile Gly Val Gly Gln Val Ile Lys Gly Trp Asp Val Gly Ile Pro
    50                  55                  60

Lys Leu Ser Val Gly Glu Lys Ala Arg Leu Thr Ile Pro Gly Pro Tyr
65                  70                  75                  80

Ala Tyr Gly Pro Arg Gly Phe Pro Gly Leu Ile Pro Pro Asn Ser Thr
                85                  90                  95

Leu Val Phe Asp Val Glu Leu Leu Lys Val Asn
            100                 105

<210> 80
<211> 107
<212> PRT
<213> Human

<400> 80
Gly Val Gln Val Glu Thr Ile Ser Pro Gly Asp Gly Arg Thr Phe Pro
1               5                   10                  15

Lys Arg Gly Gln Thr Cys Val Val His Tyr Thr Gly Met Leu Glu Asp
            20                  25                  30

Gly Lys Lys Phe Asp Ser Ser Arg Asp Arg Asn Lys Pro Phe Lys Phe
        35                  40                  45

Met Leu Gly Lys Gln Glu Val Ile Arg Gly Trp Glu Glu Gly Val Ala
    50                  55                  60

Gln Met Ser Val Gly Gln Arg Ala Lys Leu Thr Ile Ser Pro Asp Tyr
65                  70                  75                  80

Ala Tyr Gly Ala Thr Gly His Pro Gly Ile Ile Pro Pro His Ala Thr
                85                  90                  95

Leu Val Phe Asp Val Glu Leu Leu Lys Leu Glu
            100                 105

<210> 81
<211> 107
<212> PRT
<213> Mouse

<400> 81
Gly Val Gln Val Glu Thr Ile Ser Pro Gly Asp Gly Arg Thr Phe Pro
1               5                   10                  15

Lys Arg Gly Gln Thr Cys Val Val His Tyr Thr Gly Met Leu Glu Asp
            20                  25                  30

Gly Lys Lys Phe Asp Ser Ser Arg Asp Arg Asn Lys Pro Phe Lys Phe
        35                  40                  45

Thr Leu Gly Lys Gln Glu Val Ile Arg Gly Trp Glu Glu Gly Val Ala
    50                  55                  60

Gln Met Ser Val Gly Gln Arg Ala Lys Leu Ile Ile Ser Ser Asp Tyr
65                  70                  75                  80

Ala Tyr Gly Ala Thr Gly His Pro Gly Ile Ile Pro Pro His Ala Thr
                85                  90                  95

Leu Val Phe Asp Val Glu Leu Leu Lys Leu Glu
            100                 105

<210> 82
<211> 107
<212> PRT
<213> Bovine

<400> 82
Gly Val Glu Ile Glu Thr Ile Ser Pro Gly Asp Gly Arg Thr Phe Pro
1               5                   10                  15

Lys Lys Gly Gln Thr Cys Val Val His Tyr Thr Gly Met Leu Gln Asn
            20                  25                  30

Gly Lys Lys Phe Asp Ser Ser Arg Asp Arg Asn Lys Pro Phe Lys Phe
        35                  40                  45

Arg Ile Gly Lys Gln Glu Val Ile Lys Gly Phe Glu Glu Gly Ala Ala
    50                  55                  60

Gln Met Ser Leu Gly Gln Arg Ala Lys Leu Thr Cys Thr Pro Asp Val
65                  70                  75                  80

Ala Tyr Gly Ala Thr Gly His Pro Gly Val Ile Pro Pro Asn Ala Thr
                85                  90                  95

Leu Ile Phe Asp Val Glu Leu Leu Asn Leu Glu
            100                 105

<210> 83
<211> 107
<212> PRT
<213> Neurospora crassa

<400> 83
Gly Leu Gln Ile Glu Val Gln Gln Glu Gly Gln Gly Thr Arg Glu Thr
1               5                   10                  15

Arg Arg Gly Asp Asn Val Asp Val His Tyr Lys Gly Val Leu Thr Ser
            20                  25                  30

Gly Lys Lys Phe Asp Ala Ser Tyr Asp Arg Gly Glu Pro Leu Asn Phe
        35                  40                  45

Thr Val Gly Gln Gly Gln Val Ile Lys Gly Trp Asp Glu Gly Leu Leu
    50                  55                  60

Gln Tyr Leu Asn Gly Asp Tyr Glu Val Glu Pro Gly Arg Gly Gly Glu
35 40 45

Arg Asn Ala Tyr Leu Lys Glu Gln His Ile Glu Thr Phe Leu Ile Leu
50 55 60

Gly Ala Ser Gln Lys Arg Lys Glu Glu Lys Ala Met Leu Ala Lys Leu
65 70 75 80

Gln Arg Thr Arg Ala Asn Ser Met Glu Gly Leu Met Pro Arg Trp Val
85 90 95

Pro Asp Arg Ala Phe Ser Arg Thr Lys Asp Ser Lys Ala Phe Arg Gln
100 105 110

Met

<210> 94
<211> 106
<212> PRT
<213> Mouse

<400> 94
Arg Phe Lys Phe Asp Val Trp Ser Asn Asp Val Asn Leu Ala Asn Leu
1               5                   10                  15

Met Glu Gln Leu Gly Val Ala Gly Lys Val His Ile Ser Glu Ala Thr
            20                  25                  30

Ala Lys Tyr Leu Asp Asp Arg Tyr Glu Met Glu Asp Gly Arg Val Ile
        35                  40                  45

Glu Arg Leu Gly Gln Ser Val Val Ala Asp Gln Leu Lys Gly Leu Lys
    50                  55                  60

Thr Tyr Leu Ile Ser Gly Gln Arg Ala Lys Glu Ser His Cys Ser Cys
65                  70                  75                  80

Ala Glu Ala Leu Leu Gly Phe Glu Val Ile Asp Asp Ser Arg Glu Ser
                85                  90                  95

Ser Gly Pro Arg Gly Gln Gly Thr Ala Ser
            100                 105

<210> 95
<211> 109
<212> PRT
<213> Rat

<400> 95
Lys Trp Gln Tyr Asp Val Trp Ser His Asp Val Thr Leu Ala Asn His
1               5                   10                  15

Met Glu Ala Gly Gly Val Pro Gly Arg Val His Ile Ser Ser Val Thr
            20                  25                  30

Leu Glu His Leu Asn Gly Ala Tyr Lys Val Glu Glu Gly Asp Gly Glu
        35                  40                  45

Ile Arg Asp Pro Tyr Leu Lys Gln His Leu Val Lys Thr Tyr Phe Val
    50                  55                  60

Ile Asn Pro Lys Gly Glu Arg Arg Ser Pro Gln His Leu Phe Arg Pro
65                  70                  75                  80

Arg His Thr Leu Asp Gly Ala Lys Met Arg Ala Ser Val Arg Met Thr
                85                  90                  95

Arg Tyr Leu Glu Ser Trp Gly Ala Ala Lys Pro Phe Ala
            100                 105

<210> 96
<211> 109
<212> PRT
<213> Rat

<400> 96
Lys Trp Gln Tyr Asp Val Trp Ser His Asp Val Thr Leu Ala Asn His
1               5                   10                  15

Met Glu Ala Gly Gly Val Pro Gly Arg Val His Ile Thr Gly Ala Thr
            20                  25                  30

Leu Ala Leu Leu Ala Gly Ala Tyr Ala Val Glu Arg Ala Asp Met Glu
        35                  40                  45

His Arg Asp Pro Tyr Leu Arg Glu Leu Gly Glu Pro Thr Tyr Leu Val
    50                  55                  60

Ile Asp Pro Trp Ala Glu Glu Glu Asp Glu Lys Gly Thr Glu Arg Gly
65                  70                  75                  80

Leu Leu Ser Ser Leu Glu Gly His Thr Met Arg Pro Ser Leu Leu Met
                85                  90                  95

Thr Arg Tyr Leu Glu Ser Trp Gly Ala Ala Lys Pro Phe
            100                 105

<210> 97
<211> 114
<212> PRT
<213> Rat

<400> 97
Lys Trp Gln Tyr Asp Val Trp Ser His Asp Val Ser Leu Ala Asn Arg
1               5                   10                  15

Met Glu Ala Ala Gly Val Pro Gly Arg Val His Ile Thr Glu Ala Thr
            20                  25                  30

Leu Asn His Leu Asp Lys Ala Tyr Glu Val Glu Asp Gly His Gly Glu
        35                  40                  45

Gln Arg Asp Pro Tyr Leu Lys Glu Met Asn Ile Arg Thr Tyr Leu Val
    50                  55                  60

Ile Asp Pro Arg Ser Gln Gln Pro Pro Pro Pro Ser His His Leu Ser
65                  70                  75                  80

Lys Pro Lys Gly Asp Lys Ala Thr Leu Lys Met Arg Ala Ser Val Arg
                85                  90                  95

Val Thr Arg Tyr Leu Glu Ser Trp Gly Ala Ala Arg Pro Phe Ala His
            100                 105                 110

Leu Asn

<210> 98
<211> 5515
<212> DNA
<213> Human

<220>
<221> CDS
<222> (539)..(4600)

<400> 98
   gcggccgcgg cgggagcggc ggcgcactcg gggggaagca ggccccacta gacgggccgc 60
   gccgacgggc tccgcgcact cgcagcccgg ggcgccccca cccccagcgc ccgccgccgc 120
   gggcagggcc ggcccggagc gcgggggcgg gccggggagc gcgggccggg ggcgcccgcc 180
   gaagctcgct gctccgggtc ggggtcgggg ccggggccgg ccgcgcgccg cctttgacgc 240
   atcggagcgc ggctcctgca ggatggaggg ctccgcgccg ccagcggagt tgtttgtgcg 300
   caggcggctc gcggggctgg gagcgctcaa ggtctgaact tccctccgga gccgcagctg 360
   gaggaggcga gcgcgcgagg aggagaagcc gcgcggcgcg gaggccaccc tcggggcgag 420
   aggcgcggaa ggcgagcgag caaagcggtc ccggagccac ggcggccacg cggcggggac 480
   ccccgggcgt tctaggtact ggtgaccccg gccggggcag gccccgggac tcgacaac 538
   atg gct tcc ccg ccc cac cag cag ctg ctg cat cac cac agc acc gag 586
   gtg agc tgc gac tcc agc ggg gac agc aac agc gtg cgc gtc aag atc 634
   aac ccc aag cag ctg tcc tcc aac agc cac ccc aag cac tgc aaa tac 682
   agc atc tcc tct agc tgc agc agc tct ggg gac tcc ggg ggc gtc ccc 730
   cgg cga gtg ggc ggc gga ggc cgg ctg cgc agg cag aag aag ctg ccc 778
   cag ctg ttc gag agg gcc tcc agc cgc tgg tgg gac ccc aag ttc gac 826
   tcg gtg aac ctg gag gag gcc tgc ctg gag cgc tgc ttc ccg cag acc 874
   cag cgc cgg ttc cgg tat gcg ctc ttc tac atc ggc ttc gcc tgc ctt 922
   ctg tgg agc atc tat ttt gcg gtc cac atg aga tcc aga ctg atc gtc 970
   atg gtc gcc ccc gcg ctg tgc ttc ctc ctg gtg tgt gtg ggc ttc ttt 1018
   ctg ttt acc ttc acc aag ctg tac gcc cgg cat tac gcg tgg acc tcg 1066
   ctg gct ctc acc ctg ctg gtg ttc gcc ctg acc ctg gct gcg cag ttc 1114
   cag gtc ttg acg cct gtc tca gga cgc ggc gac agc tcc aac ctt acg 1162
   gcc aca gcc cgg ccc aca gat act tgc tta tct caa gtg ggg agc ttc 1210
   tcc atg tgc atc gaa gtg ctc ttt ttg ctc tat acc gtc atg cac tta 1258
   cct ttg tac ctg agt ttg tgt ctg ggg gtg gcc tac tct gtc ctt ttc 1306
   gag acc ttt ggc tac cat ttc cgg gat gaa gcc tgc ttc ccc tcg ccc 1354
   gga gcc ggg gcc ctg cac tgg gag ctg ctg agc agg ggg ctg ctc cac 1402
   ggc tgc atc cac gcc atc ggg gtc cac ctg ttc gtc atg tcc cag gtg 1450
   agg tcc agg agc acc ttc ctc aag gtg ggg caa tcc att atg cac ggg 1498
   aag gac ctg gaa gtg gaa aaa gcc ctc aaa gag agg atg att cat tcc 1546
   gtg atg cca aga atc ata gcc gat gac tta atg aag cag gga gat gag 1594
   gag agt gag aat tct gtc aag aga cat gcc acc tcg agc ccc aag aac 1642
   agg aag aaa aag tct tcc atc caa aaa gct cct ata gcc ttc cgc cct 1690
   ttt aag atg cag cag atc gaa gaa gtc agt att tta ttt gca gat atc 1738
   gtg ggc ttc acc aag atg agt gcc aac aag tct gcc cac gcc ctg gtg 1786
   ggt ctc ctg aac gat ctg ttc ggt cgc ttc gac cgc ctg tgt gag gag 1834
   acc aag tgt gag aaa atc agc acc ctg gga gac tgt tac tac tgc gtg 1882
   gcg ggc tgt ccc gag ccc cgg gcc gac cat gcc tac tgc tgc atc gag 1930
   atg ggc ctg ggc atg atc aag gcc atc gag cag ttc tgc cag gag aag 1978
   aag gag atg gtg aac atg aga gtc ggg gtg cac acg cgc acc gtc ctt 2026
   tgc ggc atc ctg ggc atg agg agg ttt aaa ttt gac gtg tgg tcc aac 2074
   gat gtg aac ctg gcc aat ctc atg gag cag ctg gga gtg gcc ggc aaa 2122
   gtt cac att tct gag gcc acc gca aaa tac tta gat gac cgg tac gaa 2170
   atg gaa gat ggg aaa gtt att gaa cgg ctg ggc cag agc gtg gtt gct 2218
   gac cag ttg aaa ggt ttg aag aca tac ctg ata tcg ggt cag aga gcc 2266
   aag gag tct cgc tgc agc tgt gca gag gcc ttg ctt tct ggc ttt gag 2314
   gtc att gac ggc tca cag gtg tcc tca ggc cct agg gga cag ggg aca 2362
   gcg tca tca ggg aat gtc agt gac ttg gcg cag act gtc aaa acc ttt 2410
   gat aac ctt aag acc tgc cct tcg tgc gga atc aca ttt gct ccc aaa 2458
   tct gaa gcc ggc gcc gag gga gga gca cct caa aac ggc tgc caa gac 2506
   gag cat aaa aac agc acc aag gct tct gga gga cct aat ccc aaa act 2554
   cag aac ggg ctc ctc agc cct ccc caa gag gag aag ctc acc aac agt 2602
   cag act tct ctg tgt gag atc ttg cag gag aag gga agg tgg gca ggg 2650
   gtg agc ctg gac cag tcg gct ctc ctt ccg ctg agg ttc aag aac atc 2698
   cgg gag aaa acg gac gcc cac ttt gtg gac gtt atc aaa gaa gac agc 2746
   ctg atg aaa gat tac ttt ttt aag ccg ccc att aat cag ttc agc ctg 2794
   aac ttc ctg gat cag gag ctg gag cga tcc tac agg acc agc tat cag 2842
   gaa gag gtc ata aag aac tcc ccc gtg aag acg ttt gct agt ccc acc 2890
   ttc agc tcc ctc ctg gat gtg ttt ctg tcg acc aca gtg ttt ctg acg 2938
   ctg tcc acc acc tgc ttc ctg aag tac gag gcg gcc acc gtg cct ccc 2986
   ccg ccc gcc gcc ctg gcg gtc ttc agt gca gcc ctg ctg ctg gag gtg 3034
   ctg tcc ctc gcg gtg tcc atc agg atg gtg ttc ttc ctg gag gac gtc 3082
   atg gcc tgc acc aag cgc ctg ctg gag tgg atc gcc ggc tgg cta cca 3130
   cgt cac tgc atc ggg gcc atc ctg gtg tcg ctt ccc gca ctg gcc gtc 3178
   tac tcc cat gca acc tcc gaa tat gag acc aac ata cac ttc cca gtg    3226
   ttc aca ggc tcg gcc gcg ctg att gcc gtc gtg cac tac tgt aac ttc    3274
   tgc cag ctc agc tcc tgg atg agg tcc tcc ctc gcc acc gtc gtg ggg    3322
   gcc ggg ccg ctg ctc ctg ctc tac gtc tcc ctg tgc cca gac agt tct    3370
   gta tta act tcg ccc ctt gac gca gta cag aat ttc agt tcc gag agg    3418
   aac ccg tgc aat agt tcg gtg ccg cgt gac ctc cgg cgg ccc gcc agc    3466
   ctc atc ggc cag gag gtg gtt ctc gtc ttc ttt ctc ctg ctc ttg ttg    3514
   gtc tgg ttc ctg aat cgc gaa ttt gaa gtc agc tac cgc ctc cac tac    3562
   cac gga gac gtg gaa gcg gat ctt cac cgc acc aag atc cag agc atg    3610
   cgg gac cag gca gac tgg ctg ctg agg aac atc atc ccc tac cac gtg    3658
   gct gag cag ctg aag gtg tcc cag acc tac tcc aag aac cac gac agc    3706
   gga ggg gtg atc ttc gcc agc atc gtc aac ttc agc gag ttc tac gag    3754
   gag aac tac gag ggc ggc aag gag tgc tac cgg gtc ctc aac gag ctc 3802
   atc ggg gac ttt gac gag ctc cta agc aag ccg gac tac agc agc atc 3850
   gag aag atc aag acc atc gga gcc acg tac atg gcg gcg tca ggg ctg 3898
   aac acc gcg cag gcc cag gac ggc agc cac ccg cag gag cac ctg cag 3946
   atc ctg ttc gag ttc gcc aag gag atg atg cgc gtg gtg gac gac ttc 3994
   aac agc aac atg ctg tgg ttc aac ttc aag ctc cgc gtc ggc ttc aac 4042
   cat ggg ccc ctc acg gcc ggg gtc atc ggc acc acc aag ctg ctg tac 4090
   gac atc tgg gga gac acc gtc aac atc gcc agc agg atg gac acc acc 4138
   ggc gtg gag tgc cgc atc cag gtg agc gaa gag agc tac cgc gtc ttg 4186
   agc aag atg ggc tat gac ttc gac tac aga ggg acc gtg aat gtc aag 4234
   ggg aaa ggc cag atg aag acc tac ctg tac cca aag tgc acg gat cac 4282
   agg gtc atc cca cag cac cag ctg tcc atc tcc cca gac atc cgc gtc 4330
   cag gtg gat ggc agc atc gga cgg tct ccc aca gac gag att gcc aac 4378
   ctg gtg cct tct gtc cag tat gtg gac aag aca tct ctg ggt tct gac 4426
   agc agc acg cag gcc aag gat gcc cac ctg tcc cgc aag aga ccg tgg 4474
   aag gag ccc gtc aaa gcc gaa gaa agg ggt cga ttt ggc aaa gcc ata 4522
   gag aaa gac gac tgt gac gaa aca gga ata gaa gaa gcc aac gaa ctc 4570
   acc aag ctc aac gtt tca aag agt gtg tga ggcggcgccc acccgctgcc 4620
   cgaggtgctc tgtttgtcga aacacagtaa tatttgtatt tggctgttgt gctttccaag 4680
   cgccacagtt gccctccccg gacgtggtgt tatgtggtca tttcagccct aacttctgtg 4740
   tggatcacag ttattcaggg ttcattttca tccattcttc cctttcgctc ccttccctgg 4800
   aaaccccgct gcctctgggt catccgttca gcacgtggtg gagaacaagt gccttcaggg 4860
   ctggcctcgg cctcgagtct cgggacagag gccgccagtg gagatcatgg ctttgggtat 4920
   tatttgactt ttagaacaaa agctgtggtt aagatctcat ttttattgct ttttcccacg 4980
   tcccacgaga cactattttc ggttctctgg ctaataccct gtttttgagt ttattttgtt 5040
   tctgtctatg tcacagtgtt cctctacgac ccgacctctc tatgtaagca cacatgcgca 5100
   cacacacttg cattcatgaa tctgatataa agtgccagta atccgccaag agggggtgcg 5160
   aagggggcat gtcacgacag ctccgccacc ccccattgcc cacccgcact ttcccgagca 5220
   ccgcgccccg tgggctgtgg gtgagccgcg ctccctgcac tgagcgggtt taggggctcg 5280
   cccacatgca tgcaggccaa gacagcaaat gccagccggg cacgacgccc gtgtgcccag 5340
   gcctcggggg tctcagagcc gcctctcacc cccgaccctc cacccagggg tcttcccgtc 5400
   gggagtggag gcgttggtcc tggaagctga ctcatcggag agggaaatac caaataaaca 5460
   tccgaggttg caaaaaaaaa aaaaaaaaaa aaaaaaaaaa aaaaagcgcg gccgc 5515

<210> 99
<211> 1353
<212> PRT
<213> Human

<400> 99

<210> 100
<211> 26
<212> DNA
<213> Artificial Sequence

<220>
<223> Description of Artificial Sequence: Primer

<400> 100
gacctttggc taccatttcc gggatg 26

<210> 101
<211> 25
<212> DNA

<213> Artificial Sequence

<220>
<223> Description of Artificial Sequence: Primer

<400> 101
cttcgctcac ctggatgcgg cactc                                          25


<210> 102
<211> 19
<212> DNA
<213> Artificial Sequence

<220>
<223> Description of Artificial Sequence: Primer based
      on mouse adenyl cyclase 9

<400> 102
ggagcgctgc tttccgcag                                                 19


<210> 103
<211> 25
<212> DNA
<213> Artificial Sequence

<220>
<223> Description of Artificial Sequence: Primer based
      on mouse adenyl cyclase 9

<400> 103
catgttcacc atctctttct tctcc                                          25


<210> 104
<211> 29
<212> DNA
<213> Artificial Sequence

<220>
<223> Description of Artificial Sequence: Primer based
      on mouse adenyl cyclase 9

<400> 104
gtggccgtga gagtatgatt ggagctgtc                                      29

<210> 38
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 38
Glu Arg Ala Ser Ser
1               5

<210> 39
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 39
Gly Arg Val Asp Ser
1               5

<210> 40
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>
<223> ANTI-SENSE : NO

<400> 40
Val Arg Ser Arg Ser
1               5

<210> 41
<211> 5
<212> PRT
<213> Mouse

<220>
<223> STRANDEDNESS : Single

<220>
<223> TOPOLOGY : Linear

<220>
<223> MOLECULE TYPE : Peptide

<220>
<223> HYPOTHETICAL : NO

<220>

Gly Lys Thr Val Asn Leu
                    85

<210> 53
<211> 86
<212> PRT
<213> Rat

<400> 53
Lys Ile Lys Thr Ile Gly Ser Thr Tyr Met Ala Val Ser Gly Leu Ser
1               5                   10                  15

Pro Glu Lys Gln Gln Cys Glu Asp Lys Trp Gly His Leu Cys Ala Leu
            20                  25                  30

Ala Asp Phe Ser Leu Ala Leu Thr Glu Ser Ile Gln Glu Ile Asn Lys
        35                  40                  45

His Ser Phe Asn Asn Phe Glu Leu Arg Ile Gly Ile Ser His Gly Ser
    50                  55                  60

Val Val Ala Gly Val Ile Gly Ala Lys Lys Pro Gln Tyr Asp Ile Trp
65                  70                  75                  80

Gly Lys Thr Val Asn Leu
                    85

<210> 54
<211> 85
<212> PRT
<213> Human

<400> 54
Lys Ile Lys Thr Ile Gly Ser Thr Tyr Met Ala Ala Ser Gly Leu Asn
1               5                   10                  15

Ala Ser Thr Tyr Asp Gln Val Gly Arg Ser His Ile Thr Ala Leu Ala
            20                  25                  30

Asp Tyr Ala Met Arg Leu Met Glu Gln Met Lys His Ile Asn Glu His
        35                  40                  45

Ser Phe Asn Asn Phe Gln Met Lys Ile Gly Leu Asn Met Gly Pro Val

        50                  55                  60

Val Ala Gly Val Ile Gly Ala Arg Lys Pro Gln Tyr Asp Ile Trp Gly
65                  70                  75                  80

Asn Thr Val Asn Val
                85

<210> 55
<211> 85
<212> PRT
<213> Mouse

<400> 55
Lys Ile Lys Thr Ile Gly Ser Thr Tyr Met Ala Ala Ser Gly Leu Asn
1               5                   10                  15

Ala Ser Thr Tyr Asp Gln Val Gly Arg Ser His Ile Thr Ala Leu Ala
            20                  25                  30

Asp Tyr Ala Met Arg Leu Met Glu Gln Met Lys His Ile Asn Glu His
        35                  40                  45

Ser Phe Asn Asn Phe Gln Met Lys Ile Gly Leu Asn Met Gly Pro Val
    50                  55                  60

Val Ala Gly Val Ile Gly Ala Arg Lys Pro Gln Tyr Asp Ile Trp Gly
65                  70                  75                  80

Asn Thr Val Asn Val
                85

<210> 56
<211> 85
<212> PRT
<213> Mouse

<400> 56
Lys Ile Lys Thr Ile Gly Ser Thr Tyr Met Ala Ala Ser Gly Leu Asn
1               5                   10                  15

Ala Ser Thr Tyr Asp Gln Val Gly Arg Ser His Ile Thr Ala Leu Ala
            20                  25                  30

<210> 62
<211> 88
<212> PRT
<213> Bovine

<400> 62
Lys Ile Lys Thr Ile Gly Ser Thr Tyr Met Ala Ala Val Gly Leu Ala
1               5                   10                  15

Pro Thr Ala Gly Thr Lys Ala Lys Lys Cys Ile Ser Ser His Leu Ser
            20                  25                  30

Thr Leu Ala Asp Phe Ala Ile Glu Met Phe Asp Val Leu Asp Glu Ile
        35                  40                  45

Asn Tyr Gln Ser Tyr Asn Asp Phe Val Leu Arg Val Gly Ile Asn Val
    50                  55                  60

Gly Pro Val Val Ala Gly Val Ile Gly Ala Arg Arg Pro Gln Tyr Asp
65                  70                  75                  80

Ile Trp Gly Asn Thr Val Asn Val
                85

<210> 63
<211> 98
<212> PRT
<213> Rat

<400> 63
Lys Ile Lys Thr Ile Gly Ser Thr Tyr Met Ala Ala Ser Gly Val Thr
1               5                   10                  15

Pro Asp Val Asn Thr Asn Gly Phe Thr Ser Ser Ser Lys Glu Glu Lys
            20                  25                  30

Ser Asp Lys Glu Arg Trp Gln His Leu Ala Asp Leu Ala Asp Phe Ala
        35                  40                  45

Leu Ala Met Lys Asp Thr Leu Thr Asn Ile Asn Asn Gln Ser Phe Asn
    50                  55                  60

Asn Phe Met Leu Arg Ile Gly Met Asn Lys Gly Gly Val Leu Ala Gly
65                  70                  75                  80

Val Ile Gly Ala Arg Lys Pro His Tyr Asp Ile Trp Gly Asn Thr Val
                85                  90                  95

Asn Val

<210> 64
<211> 88
<212> PRT
<213> Mouse

<220>
<223> Description of Artificial Sequence: Primer based
      on mouse adenyl cyclase 9

<400> 64
Lys Ile Lys Thr Ile Gly Ala Thr Tyr Met Ala Ala Ser Gly Leu Asn
 1               5                  10                  15

Thr Ala Gln Cys Gln Glu Gly Gly His Pro Gln Glu His Leu Arg Ile
            20                  25                  30

Leu Phe Glu Phe Ala Lys Glu Met Met Arg Val Val Asp Asp Phe Asn
        35                  40                  45

Asn Asn Met Leu Trp Phe Asn Phe Lys Leu Arg Val Gly Phe Asn His
    50                  55                  60

Gly Pro Leu Thr Ala Gly Val Ile Gly Thr Thr Lys Leu Leu Tyr Asp
65                  70                  75                  80

Ile Trp Gly Asp Thr Val Asn Ile
                85

<210> 65
<211> 108
<212> PRT
<213> Mouse

<400> 65
Arg Phe Lys Phe Asp Val Trp Ser Asn Asp Val Asn Leu Ala Asn Leu
 1               5                  10                  15

Met Glu Gln Leu Gly Val Ala Gly Lys Val His Ile Ser Glu Ala Thr
            20                  25                  30

Ala Tyr Gly Ala Thr Gly His Pro Gly Ile Ile Pro Pro His Ala Thr
85 90 95

Leu Val Phe Asp Val Glu Leu Leu Lys Leu Glu
100 105

<210> 69
<211> 107
<212> PRT
<213> Neurospora crassa

<400> 69
Gly Leu Gln Ile Glu Val Gln Gln Glu Gly Gln Gly Thr Arg Glu Thr
1               5                   10                  15

Arg Arg Gly Asp Asn Val Asp Val His Tyr Lys Gly Val Leu Thr Ser
            20                  25                  30

Gly Lys Lys Phe Asp Ala Ser Tyr Asp Arg Gly Glu Pro Leu Asn Phe
        35                  40                  45

Thr Val Gly Gln Gly Gln Val Ile Lys Gly Trp Asp Glu Gly Leu Leu
    50                  55                  60

Gly Met Lys Ile Gly Glu Lys Arg Lys Leu Thr Ile Ala Pro His Leu
65                  70                  75                  80

Ala Tyr Gly Asn Arg Ala Val Gly Gly Ile Ile Pro Ala Asn Ser Thr
                85                  90                  95

Leu Ile Phe Glu Thr Glu Leu Val Gly Ile Lys
            100                 105

<210> 70
<211> 104
<212> PRT
<213> Bovine

<400> 70
Lys Trp Gln Tyr Asp Val Trp Ser Asn Asp Val Thr Leu Ala Asn Val
1               5                   10                  15

Met Glu Ala Ala Gly Leu Pro Gly Lys Val His Ile Thr Lys Thr Thr
            20                  25                  30

Leu Ala Cys Leu Asn Gly Asp Tyr Glu Val Glu Pro Gly His Gly His
        35                  40                  45

Glu Arg Asn Ser Phe Leu Lys Thr His Asn Ile Glu Thr Phe Phe Ile
    50                  55                  60

Val Pro Ser His Arg Arg Lys Ile Phe Pro Gly Leu Ile Leu Ser Asp
65                  70                  75                  80

Ile Lys Pro Ala Lys Arg Met Lys Phe Lys Thr Val Cys Tyr Leu Leu
                85                  90                  95

Val Gln Leu Met Tyr His Cys Arg
            100

<210> 71
<211> 104
<212> PRT
<213> Rat

<400> 71
Arg Trp Gln Tyr Asp Val Trp Ser Thr Asp Val Thr Val Ala Asn Lys
1               5                   10                  15

Met Glu Ala Gly Gly Ile Pro Gly Arg Val His Ile Ser Gln Ser Thr
            20                  25                  30

Met Asp Cys Leu Lys Gly Glu Phe Asp Val Glu Pro Gly Asp Gly Gly
        35                  40                  45

Ser Arg Cys Asp Tyr Leu Asp Glu Lys Gly Ile Glu Thr Tyr Leu Ile
    50                  55                  60

Ile Ala Ser Lys Pro Glu Val Lys Lys Thr Ala Gln Asn Gly Leu Asn
65                  70                  75                  80

Gly Ser Ala Leu Pro Asn Gly Ala Pro Ala Ser Lys Pro Ser Ser Ser
                85                  90                  95

Pro Ala Leu Ile Glu Thr Lys Glu
            100

<210> 72
<211> 104
<212> PRT
<213> Rat

<400> 72
Lys Trp Gln Phe Asp Val Trp Ser Trp Asp Val Asp Ile Ala Asn Lys
1               5                   10                  15

Leu Glu Ser Gly Gly Ile Pro Gly Arg Ile His Ile Ser Lys Ala Thr
            20                  25                  30

Leu Asp Cys Leu Ser Gly Asp Tyr Asn Val Glu Glu Gly His Gly Lys
        35                  40                  45

Glu Arg Asn Glu Phe Leu Arg Lys His Asn Ile Glu Thr Tyr Leu Ile
    50                  55                  60

Lys Gln Pro Glu Glu Ser Leu Leu Ser Leu Pro Glu Asp Ile Val Lys
65                  70                  75                  80

Glu Ser Val Ser Cys Ser Asp Arg Arg Asn Ser Gly Ala Thr Phe Thr
                85                  90                  95

Glu Gly Ser Trp Ser Pro Glu Leu
            100

<210> 73
<211> 104
<212> PRT
<213> Rat

<400> 73
Lys Trp Gln Phe Asp Val Trp Ser Asn Asp Val Thr Leu Ala Asn His
1               5                   10                  15

Met Glu Ala Gly Gly Lys Ala Gly Arg Ile His Ile Thr Lys Ala Thr
            20                  25                  30

Leu Asn Tyr Leu Asn Gly Asp Tyr Glu Val Glu Pro Gly Cys Gly Gly
        35                  40                  45

Glu Arg Asn Ala Tyr Leu Lys Glu His Ser Ile Glu Thr Phe Leu Ile
    50                  55                  60

Leu Arg Cys Thr Gln Lys Arg Lys Glu Glu Lys Ala Met Ile Ala Lys

65                  70                  75                  80

Met Asn Arg Gln Arg Thr Asn Ser Ile Gly His Asn Pro Pro His Trp
                85                  90                  95

Gly Ala Glu Arg Pro Phe Tyr Asn
            100

<210> 74
<211> 104
<212> PRT
<213> Rat

<400> 74
Lys Trp Gln Phe Asp Val Trp Ser Asn Asp Val Thr Leu Ala Asn His
 1               5                  10                  15

Met Glu Ala Gly Gly Arg Ala Gly Arg Ile His Ile Thr Arg Ala Thr
            20                  25                  30

Leu Gln Tyr Leu Asn Gly Asp Tyr Glu Val Glu Pro Gly Arg Gly Gly
        35                  40                  45

Glu Arg Asn Gly Tyr Leu Lys Glu Gln Cys Ile Glu Thr Phe Leu Ile
    50                  55                  60

Leu Gly Ala Ser Gln Lys Arg Lys Glu Glu Lys Ala Met Leu Val Lys
65                  70                  75                  80

Leu Gln Arg Thr Arg Ala Asn Ser Met Glu Gly Leu Met Pro His Trp
                85                  90                  95

Gly Ala Glu Arg Pro Phe Tyr Asn
            100

<210> 75
<211> 102
<212> PRT
<213> Rat

<400> 75
Lys Trp Gln Tyr Asp Val Trp Ser His Asp Val Thr Leu Ala Asn His
 1               5                  10                  15

Gly Met Lys Ile Gly Glu Lys Arg Lys Leu Thr Ile Ala Pro His Leu
65                  70                  75                  80

Ala Tyr Gly Asn Arg Ala Val Gly Gly Ile Ile Pro Ala Asn Ser Thr
                85                  90                  95

Leu Ile Phe Glu Thr Glu Leu Val Gly Ile Lys
            100                 105

<210> 84
<211> 105
<212> PRT
<213> Human

<400> 84
Arg Val Asp His Cys Pro Ile Lys Ser Arg Lys Gly Asp Val Leu His
1               5                   10                  15

Met His Tyr Thr Gly Lys Leu Glu Asp Gly Thr Glu Phe Asp Ser Ser
            20                  25                  30

Leu Pro Gln Asn Gln Pro Phe Val Phe Ser Leu Gly Thr Gly Gln Val
        35                  40                  45

Ile Lys Gly Trp Asp Gln Gly Leu Leu Gly Met Tyr Glu Gly Glu Lys
    50                  55                  60

Arg Lys Leu Val Ile Pro Ser Glu Leu Gly Tyr Gly Glu Arg Gly Ala
65                  70                  75                  80

Pro Pro Lys Ile Pro Gly Gly Ala Thr Leu Val Phe Glu Val Glu Leu
                85                  90                  95

Leu Lys Ile Glu Arg Arg Thr Glu Leu
            100                 105

<210> 85
<211> 103
<212> PRT
<213> Human

<400> 85
Ser Val Leu Lys Lys Gly Asp Lys Thr Asn Phe Pro Lys Lys Gly Asp

1               5                   10                  15

Val Val His Cys Trp Tyr Thr Gly Thr Leu Gln Asp Gly Thr Val Phe
            20                  25                  30

Asp Thr Asn Ile Gln Thr Ser Lys Pro Leu Ser Phe Lys Val Gly Val
        35                  40                  45

Gly Lys Val Ile Arg Gly Trp Asp Glu Ala Leu Leu Thr Met Ser Lys
    50                  55                  60

Gly Glu Lys Ala Arg Leu Glu Ile Glu Pro Glu Trp Ala Tyr Gly Lys
65                  70                  75                  80

Lys Gly Gln Pro Ala Lys Ile Pro Pro Asn Ala Lys Leu Thr Phe Glu
                85                  90                  95

Val Glu Leu Val Asp Ile Asp
            100

<210> 86
<211> 104
<212> PRT
<213> Yeast

<400> 86
Ile Lys Arg Ile Pro Val Glu Asp Cys Leu Ile Lys Ala Met Pro Gly
1               5                   10                  15

Asp Lys Val Lys Val His Tyr Thr Gly Ser Leu Glu Ser Gly Thr Val
            20                  25                  30

Phe Asp Ser Ser Tyr Ser Arg Gly Ser Pro Ile Ala Phe Glu Leu Gly
        35                  40                  45

Val Gly Arg Val Ile Lys Gly Trp Asp Gln Gly Val Ala Gly Met Cys
    50                  55                  60

Val Gly Glu Lys Arg Lys Leu Gln Ile Pro Ser Ser Leu Ala Tyr Gly
65                  70                  75                  80

Glu Arg Gly Val Pro Gly Val Ile Pro Pro Ser Ala Asp Leu Val Phe
                85                  90                  95

Asp Val Glu Leu Val Asp Val Lys
            100

<210> 87
<211> 110
<212> PRT
<213> Bovine

<400> 87
Thr Gly Arg Val Leu Cys Gly Val Leu Gly Leu Arg Lys Trp Gln Tyr
1               5                   10                  15

Asp Val Trp Ser Asn Asp Val Thr Leu Ala Asn Val Met Glu Ala Ala
            20                  25                  30

Gly Leu Pro Gly Lys Val His Ile Thr Lys Thr Thr Leu Ala Cys Leu
        35                  40                  45

Asn Gly Asp Tyr Glu Val Glu Pro Gly His Gly His Glu Arg Asn Ser
    50                  55                  60

Phe Leu Lys Thr His Asn Ile Glu Thr Phe Phe Ile Val Pro Ser His
65                  70                  75                  80

Arg Arg Lys Ile Phe Pro Gly Leu Ile Ile Ser Asp Ile Lys Pro Ala
                85                  90                  95

Lys Arg Met Lys Phe Lys Thr Val Cys Tyr Leu Ile Val Gln
            100                 105                 110

<210> 88
<211> 109
<212> PRT
<213> Rat

<400> 88
Ala Asn Lys Met Glu Ala Gly Gly Ile Pro Gly Arg Val His Ile Ser
1               5                   10                  15

Gln Ser Thr Met Asp Cys Leu Lys Gly Glu Phe Asp Val Glu Pro Gly
            20                  25                  30

Asp Gly Gly Ser Arg Cys Asp Tyr Leu Asp Glu Lys Gly Ile Glu Thr
        35                  40                  45

Tyr Leu Ile Ile Ala Ser Lys Pro Glu Val Lys Lys Thr Ala Gln Asn

    50                  55                  60

Gly Leu Asn Gly Ser Ala Leu Pro Asn Gly Ala Pro Ala Ser Lys Pro
65                  70                  75                  80

Ser Ser Pro Ala Leu Ile Glu Thr Lys Glu Pro Asn Gly Ser Ala His
                85                  90                  95

Ala Ser Gly Ser Thr Ser Glu Glu Ala Glu Glu Gln Glu
            100                 105

<210> 89
<211> 101
<212> PRT
<213> Rat

<400> 89
Ala Asn Lys Leu Glu Ser Gly Gly Ile Pro Gly Arg Ile His Ile Ser
1               5                   10                  15

Lys Ala Thr Leu Asp Cys Leu Ser Gly Asp Tyr Asn Val Glu Glu Gly
            20                  25                  30

His Gly Lys Glu Arg Asn Glu Phe Leu Arg Lys His Asn Ile Glu Thr
        35                  40                  45

Tyr Leu Ile Lys Gln Pro Glu Glu Ser Leu Leu Ser Leu Pro Glu Asp
    50                  55                  60

Ile Val Lys Glu Ser Val Ser Cys Ser Asp Arg Arg Asn Ser Gly Ala
65                  70                  75                  80

Thr Phe Thr Glu Gly Ser Trp Ser Pro Glu Leu Pro Phe Asp Asn Ile
                85                  90                  95

Val Gly Lys Gln Asn
            100

<210> 90
<211> 101
<212> PRT
<213> Drosophilia calmodulin

<400> 90

Ala Asn His Met Glu Ser Gly Gly Glu Pro Gly Arg Val His Val Thr
1               5                   10                  15

Arg Ala Thr Asp Ser Leu Ser Gly Glu Tyr Glu Val Glu Ala Gly His
            20                  25                  30

Gly Asp Glu Arg Ser Ser Tyr Leu Arg Asp His Gly Val Asp Thr Phe
        35                  40                  45

Phe Ile Val Pro Pro Pro His Arg Arg Lys Pro Leu Met Leu Asn Thr
    50                  55                  60

Leu Gly Val Arg Ser Ala Ile Gly Ser Arg Arg Lys Leu Ser Phe Arg
65                  70                  75                  80

Asn Val Ser Asn Val Val Met Gln Leu Leu His Thr Ile Lys Phe Ser
                85                  90                  95

Glu Pro Val Pro Phe
            100

<210> 91
<211> 109
<212> PRT
<213> Rat

<400> 91
Lys Trp Gln Phe Asp Val Trp Ser Asn Asp Val Thr Leu Ala Asn His
1               5                   10                  15

Met Glu Ala Gly Gly Lys Ala Gly Arg Ile His Ile Thr Lys Ala Thr
            20                  25                  30

Leu Asn Tyr Leu Asn Gly Asp Tyr Glu Val Glu Pro Gly Cys Gly Gly
        35                  40                  45

Glu Arg Asn Ala Tyr Leu Lys Glu His Ser Ile Glu Thr Phe Leu Ile
    50                  55                  60

Leu Arg Cys Thr Gln Lys Arg Lys Glu Glu Lys Ala Met Ile Ala Lys
65                  70                  75                  80

Met Asn Arg Gln Arg Thr Asn Ser Ile Gly His Asn Pro Pro His Trp
                85                  90                  95

Gly Ala Glu Arg Pro Phe Tyr Asn His Leu Gly Gly Asn

                100                 105

<210> 92
<211> 119
<212> PRT
<213> Dog

<400> 92
Gly Met Asp Met Ile Glu Ala Ile Ser Leu Val Arg Glu Val Thr Gly
1               5                   10                  15

Val Asn Val Asn Met Arg Val Gly Ile His Ser Gly Arg Val His Cys
            20                  25                  30

Gly Val Leu Gly Leu Arg Lys Trp Gln Phe Asp Val Trp Ser Asn Asp
        35                  40                  45

Val Thr Leu Ala Asn His Met Glu Ala Gly Gly Lys Ala Gly Arg Ile
    50                  55                  60

His Ile Thr Lys Ala Thr Leu Ser Tyr Leu Asn Gly Asp Tyr Glu Val
65                  70                  75                  80

Glu Pro Gly Cys Gly Gly Glu Arg Asn Ala Tyr Leu Lys Glu His Ser
                85                  90                  95

Ile Glu Thr Phe Leu Ile Leu Arg Cys Thr Gln Lys Arg Val Arg Gly
            100                 105                 110

Gly Gly Gly Pro Arg Pro Gly
        115

<210> 93
<211> 113
<212> PRT
<213> Rat

<400> 93
Lys Trp Gln Phe Asp Val Trp Ser Asn Asp Val Thr Leu Ala Asn His
1               5                   10                  15

Met Glu Ala Ala Arg Ala Gly Arg Ile His Ile Thr Arg Ala Thr Leu
            20                  25                  30